# EUROPEAN PATENT SPECIFICATION

(11) **EP 2 454 588 B1**
(45) Date of publication and mention of the grant of the patent: **15.07.2015**
(21) Application number: 10800649.5
(22) Date of filing: 16.07.2010
(51) Int. Cl.: C12Q 1/68, G01N 33/48

(54) **NUCLEIC ACID ANALYSIS**
NUKLEINSÄUREANALYSE
ANALYSE D'ACIDES NUCLÉIQUES

(30) Priority: 16.07.2009 US 226025 P; 16.07.2009 US 226106 P
(43) Date of publication of application: 23.05.2012
(73) Proprietor: The General Hospital Corporation, Boston, MA 02114 (US)
(72) Inventor: RUSSO, Leileata M., Cambridge, MA 02139 (US); MIRANDA, Kevin C., St. Louis MO 63108 (US); SKOG, Johan, New York, NY 10069 (US)
(74) Representative: Kehoe, Laura Ellen
(86) International application number: PCT/US2010/042365
(87) International publication number: WO 2011/009104

(56) References cited:
- WO-A1-2009/036236
- WO-A1-2009/036236
- WO-A1-2009/100029
- WO-A2-02/099064
- US-A1- 2006 223 072
- US-A1- 2008 287 669
- NILSSON J ET AL: "Prostate cancer-derived urine exosomes: a novel approach to biomarkers for prostate cancer", BRITISH JOURNAL OF CANCER, HARCOURT PUBLISHERS, vol. 100, no. 10, 1 May 2009 (2009-05-01), pages 1603-1607, XP008149487, ISSN: 0007-0920, DOI: 10.1038/SJ.BJC.6605058 [retrieved on 2009-04-28]
- SAAL SAMUEL ET AL: "MicroRNAs and the kidney: coming of age", CURRENT OPINION IN NEPHROLOGY AND HYPERTENSION, RAPID SCIENCE, LONDON, GB, vol. 18, no. 4, 3 July 2009 (2009-07-03), pages 317-323, XP008146126, ISSN: 1062-4813, DOI: 10.1097/MNH.0B013E32832C9DA2
- MELISSA PIPER HUNTER ET AL: "Detection of microRNA Expression in Human Peripheral Blood Microvesicles", PLOS ONE, PUBLIC LIBRARY OF SCIENCE, SAN FRANCISCO, CA; US, vol. 3, no. 11, 1 November 2008 (2008-11-01), pages E3694-1, XP008149486, ISSN: 1932-6203, DOI: 10.1371/JOURNAL.PONE.0003694 [retrieved on 2008-11-11]
- MIRANDA KEVIN C ET AL: "Nucleic acids within urinary exosomes/microvesicles are potential biomarkers for renal disease.", KIDNEY INTERNATIONAL JUL 2010 LNKD- PUBMED:20428099, vol. 78, no. 2, July 2010 (2010-07), pages 191-199, XP002686137, ISSN: 1523-1755
- HUNTER ET AL.: 'Detection of microRNA Expression in Human Peripheral Blood Microvesicles' PLOS ONE vol. 3, no. 11, November 2008, page E3694, XP008149486
- NILSSON ET AL.: 'Prostate cancer-derived urine exosomes: a novel approach to biomarkers for prostate cancer' BRITISH JOURNAL OF CANCER vol. 100, 28 April 2009, pages 1603 - 1607, XP008149487

### SEQUENCE LISTING

<110> THE GENERAL HOSPITAL CORPORATION
<120> A METHOD OF ANALYSING NUCLEIC ACIDS FROM MICROVESICLES
<130> 26504-008
<150> US 61/226,106
   <151> 2009-07-16
<150> US 61/226,025
   <151> 2009-07-16
<160> 29
<170> PatentIn version 3.5
<210> 1
   <211> 376
   <212> DNA
   <213> HOMO SAPIENS
<400> 1
   tcatttccgg ggacagtgcc aggtggggag tttgactggg gctgtacacc tgtcaaacgg        60
   taacgcaggt gtcctaaggc gagctcaggg aggacagaaa cctcccgtgg agcagaaggg       120
   caaaagctcg cttgatcttg attttcagta cgaatacaga ccgtgaaagc ggggcctcac       180
   gatccttctg accttttggg ttttaagcag gaggtgtcag aaaagttacc acagggataa       240
   ctggcttgtg gcggccaagc gttcatagcg acgtcgcttt ttgatccttc gatgtcggct       300
   cttcctatca ttgtgaagca gaattcacca agcgttggat tgttcaccca ctaataggga       360
   acgtgagctg gaggtt                                                       376
<210> 2
   <211> 378
   <212> DNA
   <213> HOMO SAPIENS
<400> 2
   gaacctccag ctcacgttcc ctattagtgg gtgaacaatc caacgcttgg tgaattctgc        60
   ttcacaatga taggaagagc cgacatcgaa ggatcaaaaa gcgacgtcgc tatgaacgct       120
   tggccgccac aagccagtta tccctgtggt aacttttctg acacctcctg cttaaaaccc       180
   aaaaggtcag aaggatcgtg aggccccgct ttcacggtct gtattcgtac tgaaaatcaa       240
   gatcaagcga gcttttgccc ttctgctcca cgggaggttt ctgtcctccc tgagctcgcc       300
   ttaggacacc tgcgttaccg tttgacaggt gtacagcccc agtcaaactc cccacctggc       360
   actgtccccg gaaatgat                                                     378
<210> 3
   <211> 226
   <212> DNA
   <213> HOMO SAPIENS
<400> 3
   gatccgccac cgggttgaat cctccgggcg gactgcgcgg acctgaccca tttacctctt 60
   aacggtttca cgccctcttg tactctctct tcaaagttct tttcaacttt cccttacggt 120
   acttgttgac tatcggtctc gtgccggtat ttagccttaa atggagttta ccacccgctt 180
   tgggctgcat tcccaagcaa cccgactcct ggaagacccg ggccaa 226
<210> 4
   <211> 223
   <212> DNA
   <213> HOMO SAPIENS
<400> 4
   ttggcccggg tcttccagga gtcgggttgc ttgggaatgc agcccaaagc gggtggtaaa 60
   ctccatttaa ggctaaatac cggcacgaga ccgatagtca acaagtaccg taagggaaag 120
   ttgaaaagaa ctttgaagag agagtacaag agggcgtgaa accgttaaga ggtaaatggg 180
   tcaggtccgc gcagtccgcc cggaggattc aacccggtgg cgg 223
<210> 5
   <211> 107
   <212> DNA
   <213> HOMO SAPIENS
<400> 5
   gtggggaacc tggcgctaaa ccattcgtag acgacttgct tctgggtcgg ggtttcgtac 60
   gtagcagagc agctccctcg ctgcgatcta ttgaaagtca gatctcc 107
<210> 6
   <211> 160
   <212> DNA
   <213> HOMO SAPIENS
<400> 6
   cgcagacccg ctcgggggtc gcgtaactag ttagcatgcc agagtctcgt tcgttatcgg 60
   aattaaccag acaaatcgct ccaccaacta agaacggcca tgcaccacca cccacggaat 120
   cgagaaagag ctatcaatct gtcaatcctg tccgtaaatc 160
<210> 7
   <211> 105
   <212> DNA
   <213> HOMO SAPIENS
<400> 7
   agatctgact ttcaatagat cgcagcgagg gagctgctct gctacgtacg aaaccccgac 60
   ccagaagcaa gtcgtctacg aatggtttag cgccaggttc cccac 105
<210> 8
   <211> 159
   <212> DNA
   <213> HOMO SAPIENS
<400> 8
   gatttacgga caggattgac agattgatag ctctttctcg attccgtggg tggtggtgca 60
   tggccgttct tagttggtgg agcgatttgt ctggttaatt ccgataacga acgagactct 120
   ggcatgctaa ctagttacgc gacccccgag cgggtctgc 159
<210> 9
   <211> 271
   <212> DNA
   <213> HOMO SAPIENS
<400> 9
   aacactcagc taagagcatc gagggggcgc caagaggcaa ggggcgggga cgggcagtgg 60
   ctcgcctcat ggcggactgc ccgcccgctc ccaagatcca actaggagct ttttaactgc 120
   agcaacttta atatacgcta ttggagctgg aattaccgcg gctgctggca ctaaacttgc 180
   cctccaatgg atcttcgtta aaggatttaa agtggactca ttccaattac aggacctcga 240
   aagagtcctg tattgttatg tttcatcact a 271
<210> 10
   <211> 143
   <212> DNA
   <213> HOMO SAPIENS
<400> 10
   gtccctctta atcatggcct cagttccata aaccaacaaa atagaaccgc ggtcctattc 60
   cattattcct agctgcggta tccaggcggc ttggacctgc tttgaacact ctaatttttt 120
   caaagtaaac gctttgggcc gcg 143
<210> 11
   <211> 212
   <212> DNA
   <213> HOMO SAPIENS
<400> 11
   ccacgtaggg taggcacacg ctgagccagt cagtgtagcg cgcgtgcagt cccggacatc        60
   taagggcatc acagatctgt tattgctcaa tttcgggtgg ctgaaggcca cttgtccctc       120
   taagaagttg ggggacgccg accgctcggg ggtcacgtaa ctagttagca tgccagagtc       180
   tcgttggtta ttggaattaa ccagacaaat ca                                     212
<210> 12
   <211> 588
   <212> DNA
   <213> HOMO SAPIENS
<400> 12
   tagtgatgaa acataacaat acaggactct ttcgaggtcc tgtaattgga atgagtccac        60
   tttaaatcct ttaacgaaga tccattggag ggcaagttta gtgccagcag ccgcggtaat       120
   tccagctcca atagcgtata ttaaagttgc tgcagttaaa aagctcctag ttggatcttg       180
   ggagcgggcg ggcagtccgc catgaggcga gccactgccc gtccccgccc cttgcctctt       240
   ggcgccccct cgatgctctt agctgagtgt tccgcggccc aaagcgttta ctttgaaaaa      300
   attagagtgt tcaaagcagg tccaagccgc ctggataccg cagctaggaa taatggaata       360
   ggaccgcggt tctattttgt tggtttatgg aactgaggcc atgattaaga gggacagctg       420
   ggggcatttg tattgcaccg ctagaggtga aattcttgga ccggcgcagt ttggaccaga       480
   gtgaaagcat ttgccaagaa tgttttcatt aatcaagaat gaaagtcgga ggttcgaaga       540
   cgatcagata ccgtggtagt tctgaccata aacgatgccg actggcaa                    588
<210> 13
   <211> 137
   <212> DNA
   <213> HOMO SAPIENS
<400> 13
   gatttgtctg gttaattcca ataaccaacg agactctggc atgctaacta gttacgtgac        60
   ccccgagcgg tcggcgtccc ccaacttctt agagggacaa gtggccttca gccacccgaa       120
   attgagcaat aacagat                                                      137
<210> 14
   <211> 266
   <212> DNA
   <213> HOMO SAPIENS
<400> 14
   agaatccccg ccccgcggtg gggcgcggga aatgtggcgt acggaagacc cactccccgg 60
   cgccgctcgt gtggggccca agtccttctg atcgaggccc gcgacctcag atcagacgtg 120
   gcgacccgct gaatttaagc atatttgtca gcggaggaaa agaaactaac caggattccc 180
   tcagtaatgg cgagtgaaca gggaagagcc cagcgccgaa tccccgcccc gcggtggggc 240
   gcgggaaatg tggcgtacgg aaaaat 266
<210> 15
   <211> 167
   <212> DNA
   <213> HOMO SAPIENS
<400> 15
   atttttccgt acgccacatt tcccgcgccc caccgcgggg cggggattcg gcgctgggct 60
   cttccctgtt cactcgccat tactgaggga atcctggtta gtttcttttc ctccgctgac 120
   aaatatgctt aaattcagcg ggtcgccacg tctgatctga ggtcgcg 167
<210> 16
   <211> 171
   <212> DNA
   <213> HOMO SAPIENS
<400> 16
   tgactgctgg cgatggccgg gtatgggcct gatgctccag tgccatccat tttcagggct 60
   agttgattcg gcaggtgagt tgttacacac tccttagtgg attctgactt ccatggccac 120
   cgtcctgctg tctatatcaa ccaacacctt ttctggggtc tgatgagcga g 171
<210> 17
   <211> 164
   <212> DNA
   <213> HOMO SAPIENS
<400> 17
   ctcgctcatc agaccccaga aaaggtgttg gttgatatag acagcaggac ggtggccatg 60
   gaagtcagaa tccactaagg agtgtgtaac aactcacctg ccgaatcaac tagccctgaa 120
   aatggatggc actggagcat caggcccata cccggccatc gcca 164
<210> 18
   <211> 121
   <212> DNA
   <213> HOMO SAPIENS
<400> 18
   cttggaataa tggaatagga ccacagttct attttgttgg ttttcggaac tgaggccatg 60
   attaagaggg acggccgggg gcattcatat tgcaccgcta gaggtgaaat tcttggactg 120
   g 121
<210> 19
   <211> 97
   <212> DNA
   <213> HOMO SAPIENS
<400> 19
   gtgcagatct tggtggtagt agcaaatttt caaatgagaa ctttgaaggc cgaagtggag 60
   aagggttcca tgtgaacagc agttgaacat gggtccc 97
<210> 20
   <211> 245
   <212> DNA
   <213> HOMO SAPIENS
<400> 20
   atcgccagtt ggcatcgttt atggtcggaa ttacaacggt atctgatcgt cttcgaacct 60
   ccgactttcg ttcttgatta atgaaaacat tcttggcaaa tgctttcgct ctggtctaaa 120
   ctgccagtcc aagaatttca cctctagcgg tgcaatatga atgcccccgg ccgtccctct 180
   taatcatggc ctcagttccg aaaaccaaca aaatagaact gtggtcctat tccattattc 240
   caagc 245
<210> 21
   <211> 95
   <212> DNA
   <213> HOMO SAPIENS
<400> 21
   gggacccatg ttcaactgct gttcacatgg aacccttctc cacttcggcc ttcaaagttc 60
   tcatttgaaa atttgctact accaccaaga tctgc 95
<210> 22
   <211> 216
   <212> DNA
   <213> HOMO SAPIENS
<400> 22
   tttgcgtgcg gccgggcgct tggcgccaga agcgagagcc cctcagggct cgcccccccg 60
   cctcaccggg tcagtgaaaa aacgatcaga gtagtggtat ttcaccggcg gcccgcaggg 120
   ccggcagacc ccgccctgga cccctcgcgg ggacaccggg gggcgccggg ggcctcccac 180
   ttattctaca cctctcacgt ctcttcaccg tgccag 216
<210> 23
   <211> 229
   <212> DNA
   <213> HOMO SAPIENS
<400> 23
   gcttgactct agtctggcac ggtgaagaga cgtgagaggt gtagaataag tgggaggccc 60
   ccggcgcccc ccggtgtccc cgcgaggggt ccagggcggg gtctgccggc cctgcgggcc 120
   gccggtgaaa taccactact ctgatcgttt tttcactgac ccggtgaggc gggggggcga 180
   gccctgaggg gctctcgctt ctggcgccaa gcgcccggcc gcacgcaaa 229
<210> 24
   <211> 592
   <212> DNA
   <213> HOMO SAPIENS
<400> 24
   tctacctacc tacctggttg atcctgccag tagcatatgc ttgtctcaaa gattaagcca 60
   tgcatgtcta agtacgcacg gccggtacag tgaaactgcg aatggctcat taaatcagtt 120
   atggttcctt tggtcgctcg ctcctctcct acttggataa ctgtggtaat tctagagcta 180
   atacatgccg acgggcgctg acccccttcg cgggggggat gcgtgcattt atcagatcaa 240
   aaccaacccg gtcagcccct ctccggcccc ggccgggggg cgggcgccgg cggctttggt 300
   gactctagat aacctcgggc tgatcgcacg ccccccgtgg cggcgacgac ccattcgaac 360
   gtctgcccta tcaactttcg atggtagtcg ccgtgcctac catggtgacc acgggtgacg 420
   gggaatcagg gttcgattcc ggagagggag cctgagaaac ggctaccaca tccaaggaag 480
   gcagcaggcg cgcaaattac ccactcccga cccggggagg tagtgacgaa aaataacaat 540
   acaggactct ttcgaggccc tgtaattgga atgagtccac tttaaatcta at 592
<210> 25
   <211> 670
   <212> DNA
   <213> HOMO SAPIENS
<400> 25
   atttaaagtg gactcattcc aattacaggg cctcgaaaga gtcctgtatt gttatttttc 60
   gtcactacct ccccgggtcg ggagtgggta atttgcgcgc ctgctgcctt ccttggatgt 120
   ggtagccgtt tctcaggctc cctctccgga atcgaaccct gattccccgt cacccgtggt 180
   caccatggta ggcacggcga ctaccatcga aagttgatag ggcagacgtt cgaatgggtc 240
   gtcgccgcca cggggggcgt gcgatcagcc cgaggttatc tagagtcacc aaagccgccg 300
   gcgcccgccc cccggccggg gccggagagg ggctgaccgg gttggttttg atctgataaa 360
   tgcacgcatc ccccccgcga agggggtcag cgcccgtcgg catgtattag ctctagaatt 420
   accacagtta tccaagtagg agaggagcga gcgaccaaag gaaccataac tgatttaatg 480
   agccattcgc agtttcactg taccggccgt gcgtacttag acatgcatgg cttaatcttt 540
   gagacaagca tatgctactg gcaggatcaa ccaggtaggt aggtagagcg cggcgaggcc 600
   ccgacgcggc cggacggccg gccggggggc ctcgcgagga cgggcccggc gccccgcaag 660
   cgaggaggac 670
<210> 26
   <211> 210
   <212> DNA
   <213> HOMO SAPIENS
<400> 26
   gtgttgagat ttacaccctc tcccccggat tttcaagggc cagcgagagc tcaccggatg 60
   ccgccagaac cgcgacgctt tccaaggcac gggcccctct ctcggggcga acccattcca 120
   gggcgccctg cccttcacaa agaaaagaga actctccccg gggctcccgc cggcttctcc 180
   gggatcggtc acgttaccgc actggaagaa 210
<210> 27
   <211> 207
   <212> DNA
   <213> HOMO SAPIENS
<400> 27
   ttcttccagt gcggtaacgt gaccgatccc ggagaagccg gcgggagccc cggggagagt 60
   tctcttttct ttgtgaaggg cagggcgccc tggaatgggt tcgccccgag agaggggccc 120
   gtgccttgga aagcgtcgcg gttctggcgg catccggtga gctctcgctg gcccttgaaa 180
   atccggggga gagggtgtaa atctcaa 207
<210> 28
   <211> 94
   <212> DNA
   <213> HOMO SAPIENS
<400> 28
   ccgaaggtag tgagttatct caattgattg ttcacagtca gttacagatc gaactccttg      60
   ttctactctt tccccccttc tcactactgc actt                                   94
<210> 29
   <211> 99
   <212> DNA
   <213> HOMO SAPIENS
<400> 29
   gtcaagtgca gtagtgagaa ggggggaaag agtagaacaa ggagttcgat ctgtaactga      60
   ctgtgaacaa tcaattgaga taactcacta ccttcggac                             99

## Description

### FIELD OF INVENTION

The present invention relates to the general fields of nucleic acid analysis in human or other animal subjects, particularly the procurement and analysis of high quality nucleic acids from a biological sample, and in particular, from microvesicles.

### BACKGROUND

Small microvesicles shed by cells are known as "exosomes" (Thery et al., 2002). Exosomes are reported as having a diameter of approximately 30-100 nm and are shed from many different cell types under both normal and pathological conditions (Thery et al., 2002). Exosomes are classically formed from the inward invagination and pinching off of the late endosomal membrane. This results in the formation of a multivesicular body (MVB) laden with small lipid bilayer vesicles (∼40-100 nm in diameter), each of which contains a sample of the parent cell's cytoplasm (Stoorvogel et al., 2002). Fusion of the MVB with the cell membrane results in the release of these exosomes from the cell, and their delivery into the blood, urine or other bodily fluids.

Another category of cell-derived vesicles are known as "shedding microvesicles" (Cocucci et al., 2009). These microvesicles, formed by directly budding off of the cell's plasma membrane, are more heterogeneous in size than exosomes, and like exosomes, also contain a sample of the parent cell's cytoplasm. Exosomes and shedding microvesicles co-isolate using ultracentrifugation and ultrafiltration isolation techniques and will, therefore, be collectively referred to here as microvesicles.

Recent studies reveal that nucleic acids within microvesicles have a role as biomarkers. For example, Skog et. al. describes, among other things, the use of nucleic acids extracted from microvesicles in GBM patient serum for medical diagnosis, prognosis and therapy evaluation (Skog et al., 2008). The use of nucleic acids extracted from microvesicles is considered to potentially circumvent the need for biopsies, highlighting the enormous diagnostic potential of microvesicle biology (Skog et al., 2008). Urine exosomes may be used for isolating biomarkers for prostate cancer (Milsson et al, 2009, 100, p. 1603-1607).

In research and development, as well as commercial applications of nucleic acid biomarkers, it is desirable to extract high quality nucleic acids from biological samples in a consistent and reliable manner. Described herein are compositions of high quality nucleic acid extractions from microvesicles and other biological samples, methods of making such extractions, and methods of using these high quality nucleic acids in various applications.

### BRIEF SUMMARY OF THE INVENTION

In one aspect, the invention is a method of obtaining and analyzing RNA from a urine sample, comprising the steps of:
(a) obtaining a urine sample and pre-processing the urine sample by filtration through a 0.8µm filter;
(b) obtaining a microvesicle fraction from the pre-processed urine sample by ultracentrifugation or filtration concentration;
(c) washing the microvesicle fraction;
(d) extracting RNA from the microvesicle fraction;
(e) measuring the quality of the RNA from the microvesicle fraction by determining the quantity of 18S and 28S rRNA in the extraction for the purpose of evaluating the quality of the RNA extraction, where detection of 18S and 28S rRNA in the RNA extraction indicates that the RNA extraction is high yield and/or high integrity; and
f) analyzing one or more RNAs from the RNA extraction of step e) which has been evaluated as high yield and/or high integrity.

Also described is a novel nucleic acid extraction from one or more microvesicles isolated from a eukaryotic biological sample, wherein 18S rRNA and 28S rRNA are detectable in the extraction. Preferably, the quantitative ratio of 18S rRNA to 28S rRNA detectable in the novel extractions is within the range of approximately 1:1 to approximately 1:2; and is preferably approximately 1:2. Biological samples from which the novel extraction may be obtained include, among other things, any bodily fluid, preferably urine, serum or plasma, and preferably, are from a mammal, particularly a human. For bodily fluid samples with a protein concentration of less than 10 mg/ml, such as urine, the novel nucleic acid extraction may further comprise a nucleic acid extraction having an RNA Integrity Number (in all cases, as obtained on an Agilent BioAnalyzer or an equivalent thereof) of greater than or equal to 5 and/or may further comprise a nucleic acid yield from 20 ml of biological sample of greater than or equal to 50 pg/ml. Similarly, for bodily fluid samples with a protein concentration of greater than 10 mg/ml, such as serum or plasma, the novel nucleic acid extraction may further comprise an RNA Integrity Number of greater than or equal to 3 and/or may further comprise a nucleic acid yield from 1 ml of biological sample is greater than or equal to 50 pg/ml.

Additionally described is a novel profile of nucleic acid from one or more microvesicles isolated from a eukaryotic biological sample, wherein 18S rRNA and 28S rRNA are detectable in the profile. Preferably, the quantitative ratio of 18S rRNA to 28S rRNA detectable in the novel profile is within the range of approximately 1:1 to approximately 1:2; and is preferably approximately 1:2. Biological samples from which the novel profile may be obtained include, among other things, any bodily fluid, preferably urine, serum or plasma, and preferably, is from a mammal, particularly a human. For bodily fluid samples with a protein concentration of less than 10 mg/ml, such as urine, the novel profile may further comprise an RNA Integrity Number of greater than or equal to 5 and/or may further comprise a nucleic acid yield from 20 ml of biological sample of greater than or equal to 50 pg/ml. Similarly, for bodily fluid samples with a protein concentration of greater than 10 mg/ml, such as serum or plasma, the novel profile may further comprise an RNA Integrity Number of greater than or equal to 3 and/or may further comprise a nucleic acid yield from 1 ml of biological sample is greater than or equal to 50 pg/ml.

Also described is a method of evaluating the quality of a nucleic acid extraction from microvesicles isolated from a eukaryotic biological sample, comprising the steps of: (a) extracting RNA from microvesicles; and (b) measuring the quality of the RNA by determining the quantity of 18S and 28S rRNA in the extraction. Preferably, the quantitative ratio of 18S rRNA to 28S rRNA determined in the novel method is within the range of approximately 1:1 to approximately 1:2; and is preferably approximately 1:2. Biological samples on which the novel method may be performed include, among other things, any bodily fluid, preferably urine, serum or plasma, and preferably, is from a mammal, particularly a human. For bodily fluid samples with a protein concentration of less than 10 mg/ml, such as urine, the novel method may further result in the extraction of nucleic acid having an RNA Integrity Number of greater than or equal to 5 and/or may further result in a nucleic acid yield from 20 ml of biological sample of greater than or equal to 50 pg/ml. Similarly, for bodily fluid samples with a protein concentration of greater than 10 mg/ml, such as serum or plasma, the novel method may further result in the extraction of nucleic acid having an RNA Integrity Number of greater than or equal to 3 and/or may further result in a nucleic acid yield from 1 ml of biological sample is greater than or equal to 50 pg/ml.

Further described is a method of obtaining nucleic acid from a biological sample, comprising the steps of: (a) obtaining a biological sample; (b) performing an extraction enhancement operation on the biological sample; and (c) extracting nucleic acid from the biological sample. The extraction enhancement operation is comprised of: (a) the addition of one or more enhancement agents to the biological sample; or (b) the performance of one or more enhancement steps prior to nucleic acid extraction; or (c) a combination of enhancement agents and enhancement steps. The enhancement agents may include: (i) RNase inhibitor; (ii) protease; (iii) reducing agent; (iv) decoy substrate, such as synthetic RNA; (v) soluble receptor; (vi) small interfering RNA; (vii) RNA binding molecule, such as anti-RNA antibody, chaperone protein, or an RNase inhibitory protein; (ix) RNase denaturing substance, such as high osmolarity solution or detergent. The extraction enhancement steps may include: (x) washing; (xi) size-separating RNase from the sample; (xii) effecting RNase denaturation through a physical change, such as by decreasing temperature, or executing a freeze/thaw cycle. The novel method may be performed on a biological sample including, among other things, any bodily fluid, preferably urine, serum or plasma, and preferably, is from a mammal, particularly a human. A derivative may be obtained from the biological sample and subjected to the extraction enhancement operation before extracting nucleic acid. Preferably, the derivative is a microvesicle fraction from the biological sample. The microvesicle fraction may be obtained by a filtration concentration technique, however other known isolation techniques may be utilized as well. The derivative may be treated with a ribonuclease, deoxyribonuclease, or a combination thereof, prior to performance of the enhancement extraction operation. The extraction enhancement operation may include the addition of an RNase inhibitor to the biological sample, or to the derivative, prior to extracting nucleic acid; preferably the RNase inhibitor has a concentration of greater than 0.027 AU (1X) for a sample equal to or more than 1µl; alternatively, greater than or equal to 0.135 AU (5X) for a sample equal to or more than 1µl; alternatively, greater than or equal to 0.27 AU (10X) for a sample equal to or more than 1µl; alternatively, greater than or equal to 0.675 AU (25X) for a sample equal to or more than 1µl; and alternatively, greater than or equal to 1.35 AU (50X) for a sample equal to or more than 1µl, wherein the 1X protease concentration refers to an enzymatic condition wherein 0.027 AU or more protease is used to treat microvesicles isolated from 1µl or more bodily fluid; the 5X protease concentration refers to an enzymatic condition wherein 0.135 AU or more protease is used to treat microvesicles isolated from 1µl or more bodily fluid; the 10X protease concentration refers to an enzymatic condition wherein 0.27 AU or more protease is used to treat microvesicles isolated from 1µl or more bodily fluid; the 25X protease concentration refers to an enzymatic condition wherein 0.675 AU or more protease is used to treat microvesicles isolated from 1µl or more bodily fluid; the 50X protease concentration refers to an enzymatic condition wherein 1.35 AU or more protease is used to treat microvesicles isolated from 1µl or more bodily fluid. Preferably, the RNase inhibitor is a protease.

Also described is a novel kit for obtaining nucleic acids from microvesicles, comprising in one or more containers: (a) a nucleic acid extraction enhancement agent; (b) DNase, RNase, or both; and (c) a lysis buffer. The novel kit may further comprise instructions for using the kit. The nucleic acid extraction enhancing agent may include: (a) RNase inhibitor; (b) protease; (c) reducing agent; (d) decoy substrate; (e) soluble receptor; (f) small interfering RNA; (g) RNA binding molecule; (h) RNase denaturing substance; or (i) any combination of any of the foregoing agents as a mixture or individually.

Further described is a novel method of analyzing RNA from microvesicles, comprising the steps of: (a) obtaining a sample of microvesicles; (b) treating the sample with DNase to eliminate all or substantially all of any DNA located outside of or on the surface of the microvesicles in the sample; (c) extracting RNA from the sample; and (d) analyzing the extracted RNA. The novel method may be performed on a biological sample including, among other things, any bodily fluid, preferably urine, serum or plasma, and preferably, is from a mammal, particularly a human.

Additionally described is a novel method for diagnosing, monitoring, or treating a subject, comprising the steps of: (a) isolating a microvesicle fraction from a urine sample from a subject; (b) detecting the presence or absence of a biomarker within the microvesicle fraction; wherein the biomarker is selected from the group consisting of (i) a species of nucleic acid, (ii) the level of expression of a nucleic acid, (iii) a nucleic acid variant, and (iv) any combination of any of the foregoing; and wherein the biomarker is associated with the presence or absence of a disease or other medical condition, or the viability of a treatment option. The biomarker may be an mRNA transcript; for instance, the mRNA transcript may be selected from the group consisting of: NPHS2 (podocin), LGALS1 (Galectin-1), HSPG2 (heparin sulfate proteoglycan); CUBN (cubilin), LRP2 (megalin), AQP1 (aquaporin 1), CA4 (carbonic anydrase 4), CLCN5 (chloride channel protein 5), BDKRB1 (bradykinin B1 receptor), CALCR (calcitonin receptor), SCNN1D (amiloride-sensitive sodium channel subunit delta), SLC12A3 (thiazide-sensitive sodium-chloride cotransporter), AQP2 (aquaporin 2), ATP6V1B1 (V-ATPase B1 subunit), SLC12A1 (kidney-specific Na-K-Cl symporter via RT-PCR of RiboAmped mRNA); more preferably, the mRNA transcript is AQP2 (aquaporin 2) or ATP6V1B1 (V-ATPase B1 subunit). The biomarker and disease or other medical condition may be selected from the group consisting of: (a) NPHS2 (podocin) and glomerular disease, such as steroid-resistant nephritic syndrome; (b) CUBN (cubilin) and proteinuria , such as in Imerslund-Gräsbeck syndrome; and (c) AQP2 (aquaporin 2) and diabetes insipidus.

Also described is an isolated polynucleotide molecule comprising a first nucleotide sequence that is at least 90% identical to a second nucleotide sequence selected from the group consisting of SEQ ID NOS: 1-29; an isolated polynucleotide comprising a segment of a nucleotide sequence selected from SEQ ID NOS: 1-29; or an isolated polynucleotide comprising a sequence of at least 13 nucleotides that are the same as any 13-nucleotide sequence in any one of SEQ ID NOS: 1-29. In particular, the foregoing polynucleotide molecules may be a deoxyribonucleotide or a ribonucleotide. Further described is a vector comprising any of the foregoing isolated nucleic acid molecules. Also described is a host cell comprising any of the foregoing vectors or any of the foregoing isolated nucleic acid molecules.

Additionally described is a novel method of assessing the quality of a nucleic acid extraction from a biological sample, comprising: (a) providing a biological sample; (b) obtaining an extraction of nucleic acids from the biological sample; (c) measuring the amount of a polynucleotide molecule comprising a segment having a nucleotide sequence selected from SEQ NOS: 1-29 in the extraction; and (d) comparing the amount of the polynucleotide molecule against a standard to assess the quality of the nucleic acid extraction. The novel method may be performed on any biological sample, for example, a bodily fluid, in particular, urine, serum or plasma, preferably from a mammal such as a human. This novel method may be used in conjunction with any of the foregoing novel nucleic acid extractions or novel extraction methods. In particular, the standard used to assess the quality of the nucleic acid extraction may be derived by measuring the amount of a polynucleotide molecule comprising a segment having the nucleotide sequence selected from SEQ NOS: 1-29 in nucleic acid extractions from more than 5 biological samples.

### BRIEF DESCRIPTION OF THE DRAWINGS

FIGURE 1, frames a to f, are electron microscopy pictures of urinary multivesicular bodies. Multivesicular bodies (MVB) can be identified in various regions of the nephron and collecting duct (see arrows). Podo - podocyte, PT - proximal tubule, TDL - thin descending limb, TAL - thick ascending limb, CD-PC - collecting duct principal cell, CD-IC - collecting duct intercalated cell. Scale bar = 200 nm for a, c, d, e, f; 500 nm for b.
FIGURE 2 is an electron microscopy picture of isolated urinary microvesicles. Human urinary microvesicles isolated via differential ultracentrifugation and imaged via TEM using phosphotungstic acid as a stain. The scale bar = 200 nm.
FIGURE 3 is a plot depicting RNA profiles generated with a method of 100kDa MWCO filters. An Agilent BioAnalyzer was used to generate the plot.
FIGURE 4 is a plot depicting RNA profiles generated with a method of ultracentrifugation. An Agilent BioAnalyzer was used to generate the plot.
FIGURE 5 is a pair of plots depicting RNA profiles generated with a three step pre-processing method of x300g spin, x17,000g spin, and 0.8 µm filtration (**A**), or with a one step pre-processing method of only a 0.8 µm filtration (**B**), in each case followed by ultracentrifugation. An Agilent BioAnalyzer was used to generate the plots.
FIGURE 6 is a pair of plots depicting RNA profiles generated with a three-step pre-processing method of x300g spin, x17,000g spin, and 0.8 µm filtration (**A**), or with a one step pre-processing method of only a 0.8 µm filtration (**B**), in each case followed by filtration concentration. An Agilent BioAnalyzer was used to generate the plots.
FIGURE 7 is a flow chart depicting a new method of nucleic acid extraction from urine with an extraction enhancement operation.

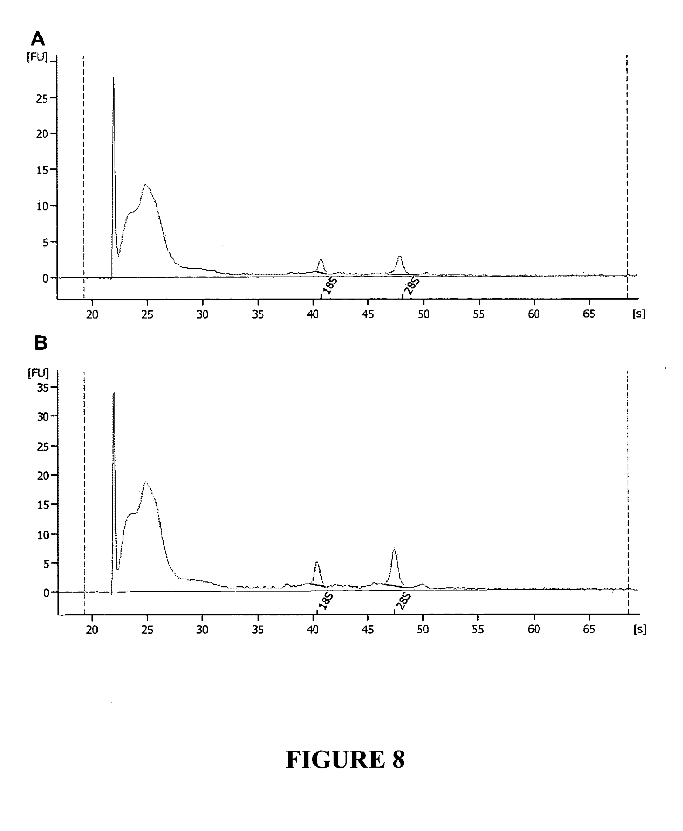

FIGURE 8 is a pair of plots depicting RNA profiles generated with methods using 5X versus 10X concentrated proteases. Microvesicles were isolated via filtration concentrators from 20ml urine samples. Plot **A** represents the profile obtained with 5X protease. Plot **B** represents the profile obtained with 10X protease. A 1X protease concentration refers to an enzymatic condition wherein 0.027 AU or more protease is used to treat microvesicles isolated from 1µl or more bodily fluid. A 5X protease concentration refers to an enzymatic condition wherein 0.135 AU or more protease is used to treat microvesicles isolated from 1µl or more bodily fluid. One mAU is the protease activity that releases folin-positive amino acids and peptides corresponding to 1 µmol tyrosine per minute.
FIGURE 9 is a pair of plots depicting RNA profiles generated with methods using 25X versus 50X concentrated proteases. Microvesicles were isolated via filtration concentrators from 40ml urine samples. Plot A represents the profile obtained with 25X protease. Plot B represents the profile obtained with 50X protease. 1X protease refers to 0.027 AU. One mAU is the protease activity that releases folin-positive amino acids and peptides corresponding to 1 µmol tyrosine per minute.
FIGURE 10 is a plot depicting the RNA profile of melanoma serum, Sample 1. RNA was extracted from 1 ml serum with a method using a RNase inhibitor, Superase-In (Ambion, Inc). The final concentration of the RNase inhibitor is 1.6 units/µl microvesicle suspension buffer.
FIGURE 11 is a plot depicting the RNA profile of melanoma serum, Sample 2. RNA was extracted from 1 ml serum with a method using a RNase inhibitor, Superase-In (Ambion, Inc). The final concentration of the RNase inhibitor is 1.6 units/µl microvesicle suspension buffer.
FIGURE 12 is a plot depicting the RNA profile of melanoma serum, Sample 3. RNA was extracted from 1 ml serum with a method using a RNase inhibitor, Superase-In (Ambion, Inc). The final concentration of the RNase inhibitor is 1.6 units/µl microvesicle suspension buffer.
FIGURE 13 is a plot depicting the RNA profile of melanoma serum, Sample 4. RNA was extracted from 1 ml serum with a method using a RNase inhibitor, Superase-In (Ambion, Inc). The final concentration of the RNase in inhibitor is 1.6 units/µl microvesicle suspension buffer.
FIGURE 14 is a plot depicting the RNA profile of a melanoma serum, Sample 5. RNA was extracted from 1 ml serum with a method using a RNase inhibitor, Superase-In (Ambion, Inc). The final concentration of the RNase inhibitor is 3.2 units/µl microvesicle suspension buffer.
FIGURE 15 is a plot depicting the RNA profile of a melanoma serum, Sample 6. RNA was extracted from 1 ml serum with a method using a RNase inhibitor, Superase-In (Ambion, Inc). The final concentration of the RNase inhibitor is 3.2 units/µl microvesicle suspension buffer.
FIGURE 16 is a plot depicting the RNA profile of normal serum, Sample 7. RNA was extracted from 1 ml serum with a method using a RNase inhibitor, Superase-In (Ambion, Inc). The final concentration of the RNase inhibitor is 1.6 units/µl microvesicle suspension buffer.
FIGURE 17 is a plot depicting the RNA profile of a normal serum, Sample 8. RNA was extracted from 1 ml serum with a method using a RNase inhibitor, Superase-In (Ambion, Inc). The final concentration of the RNase inhibitor is 1.6 units/µl microvesicle suspension buffer.
FIGURE 18 is a plot depicting the RNA profile of normal serum, Sample 9. RNA was extracted from 1 ml serum with a method using a RNase inhibitor, Superase-In (Ambion, Inc). The final concentration is of the RNase inhibitor 1.6 units/µl microvesicle suspension buffer.
FIGURE 19 is a plot depicting the RNA profile of normal serum, Sample 10. RNA was extracted from 1 ml serum with a method using a RNase inhibitor, Superase-In (Ambion, Inc). The final concentration of the RNase inhibitor is 1.6 units/µl microvesicle suspension buffer.
FIGURE 20 is a plot depicting the RNA profile of normal serum, Sample 11. RNA was extracted from 1 ml serum with a method using a RNase inhibitor, Superase-In (Ambion, Inc). The final concentration of the RNase inhibitor is 3.2 units/µl microvesicle suspension buffer.
FIGURE 21 is a plot depicting the RNA profile of normal serum, Sample 12. RNA was extracted from 1 ml serum with a method using a RNase inhibitor, Superase-In (Ambion, Inc). The final concentration of the RNase inhibitor is 3.2 units/µl microvesicle suspension buffer.
FIGURE 22 is a flow chart depicting a new method of nucleic acid extraction from a biological sample using an extraction enhancement operation.

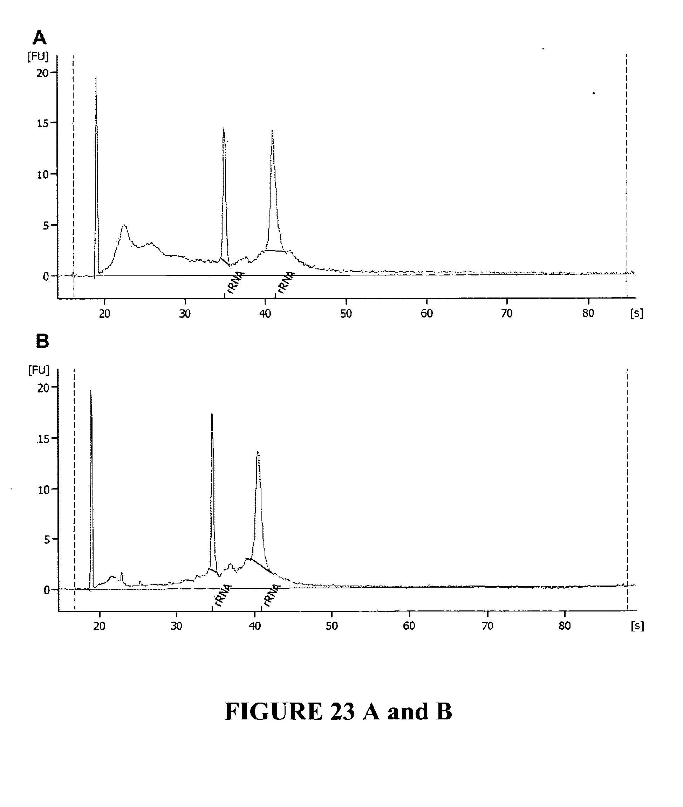

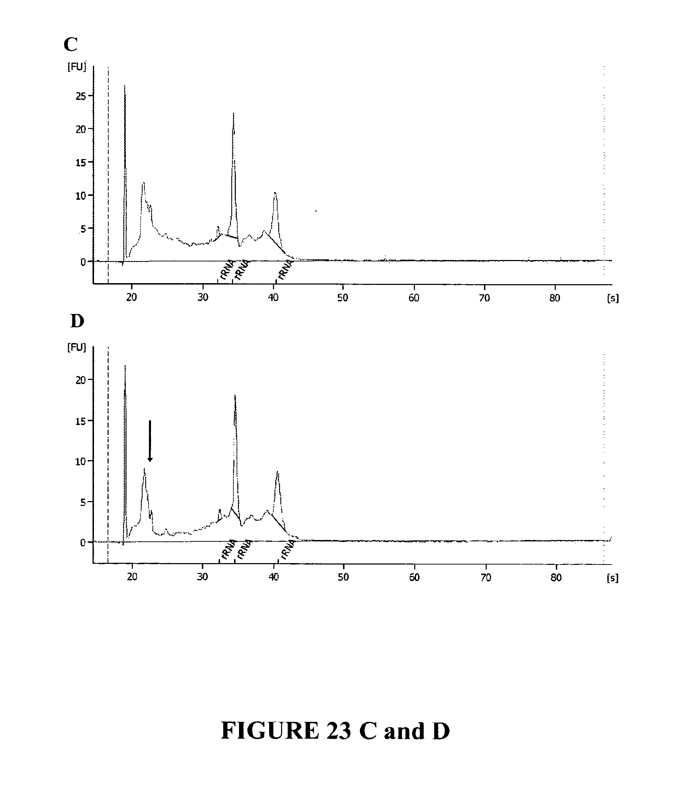

FIGURE 23 is a pair of plots depicting RNA profiles generated with methods with or without DNase treatment. DNA not located inside the microvesicles was removed by DNase digestion of the microvesicle pellet isolated from urine samples prior to lysis and nucleic acid extraction. **A** - profile without DNase digestion using RNeasy Micro Kit. **B** - profile with DNase digestion using RNeasy Micro Kit. **C**- profile without DNase digestion using MirVana Kit. **D** - profile with DNase digestion using MirVana Kit. Note the changes in small RNA peak height and area indicated by the arrow in. **D** suggests that there are some carry-over of DNA into the sample following phenol/chloroform based extraction
FIGURE 24 is a pair of plots depicting RNA profiles generated with methods with or without DNase treatment. DNA not located inside the microvesicles was removed by DNase digestion of the microvesicle pellet isolated from serum samples prior to lysis and nucleic acid extraction. **A** - profile without DNase digestion. **B** - profile with DNase digestion. **C** - A pseudo gel showing "apoptotic body"-like ladders which might co-isolate with serum-derived microvesicles.
FIGURE 25 is a pair of plots depicting RNA profiles generated with methods with or without RNase treatments. RNA not located inside the micro-vesicles was removed by RNase digestion of the microvesicle pellet isolated from urine samples prior to lysis and nucleic acid extraction. **A**- profile without RNase digestion. **B**- profile with RNase digestion.
FIGURE 26 is a pair of plots depicting RNA profiles generated from urinary microvesicles and rat kidney tissue. **A** - profile from rat kidney tissue. **B** - profile from urinary microvesicles.
FIGURE 27 is a pair of plots depicting RNA profiles generated from urinary microvesicles and rat kidney tissue using methods that can enrich small RNA extraction. **A-**profile from rat kidney tissue. **B**- profile from urinary microvesicles.
FIGURE 28 is a pair of plots depicting RNA profiles generated from whole urine exclusive of microvesicles, which are not captured by the isolation technique, with or without DNase treatments. **A**-nucleic acids isolated from whole urine without DNase treatment. **B**-nucleic acids isolated from whole urine with DNase treatments.
FIGURE 29 is a pair of plots depicting RNA profiles generated from urinary microvesicles. **A**- nucleic acids isolated from urinary microvesicles without DNase treatment. **B**- nucleic acids isolated from microvesicles with DNase treatments.
FIGURE 30 is a pair of plots depicting RNA profiles generated from nucleic acids extracted from the pellet formed during the 300g spin. **A**- nucleic acids isolated from 300g spin pellets without DNase treatment. **B**- nucleic acids isolated from 300g spin pellets with DNase treatment.
FIGURE 31 is a pair of plots depicting RNA profiles generated from nucleic acids extracted from the pellet formed during the 17,000g spin. **A**- nucleic acid profile from 17,000g spin pellet without DNase treatment. **B**- nucleic acid profile from 17,000g spin pellet with DNase treatment.
FIGURE 32 is a pair of plots depicting RNA profiles generated from microvesicles that underwent RNase and DNase digestion on the outside prior to microvesicle lysis, with or without intra-microvesicular RNase digestion. **A**- nucleic acid profile without intra-microvesicular RNase digestion. **B**- nucleic acid profile with intra-microvesicular RNase digestion.

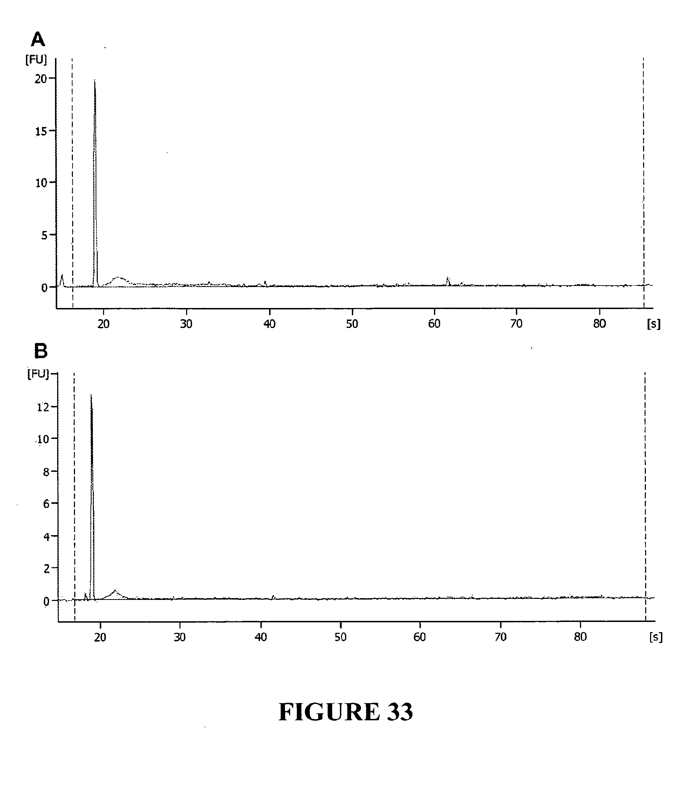

FIGURE 33 is a pair of plots depicting RNA profiles generated from microvesicles that underwent RNase and DNase digestion on the outside prior to microvesicle lysis and intra-microvesicular RNase digestion, without or without intra-microvesicular DNase digestion. **A**- nucleic acid profile without intra-microvesicular DNase digestion. **B**- nucleic acid profile with intra-microvesicular DNase digestion. On plot **B**, the peak just after 20s is reduced compared the matching peak in plot **A**. The reduction suggests that a small amount of DNase digestible material is present within exosomes.
FIGURE 34 **A**) are BioAnalyzer generated 'Pseudo gel' profiles of the positive identification of RiboAmp amplified mRNA transcripts for beta-actin and GAPDH in urinary microvesicles by RT-PCR; **B**) is an illustration of the nephron and collecting duct highlighting its six functionally distinct regions. 1. Glomerulus; 2. Proximal Tubule; 3. Thin Descending Limb; 4. Medullary Thick Ascending Limb; 5. Distal Convoluted Tubule; 6. Collecting Ducts.
FIGURE 35 represents BioAnalyzer generated pseudo gel profiles of the identification of mRNA transcripts encoding specific genes from regions 1 and 2 of the nephron and collecting duct detected by RT-PCR of RiboAmped mRNA from urinary microvesicles, specifically: **1.** Glomerulus: NPHS2 - podocin, LGALS1 - Galectin-1 , HSPG2 - heparan sulfate proteoglycan **2.** Proximal Tubule: CUBN - cubilin, LRP2 - megalin, AQP1 - aquaporin 1, CA4 - carbonic anhydrase 4, CLCN5 - chloride channel protein 5.

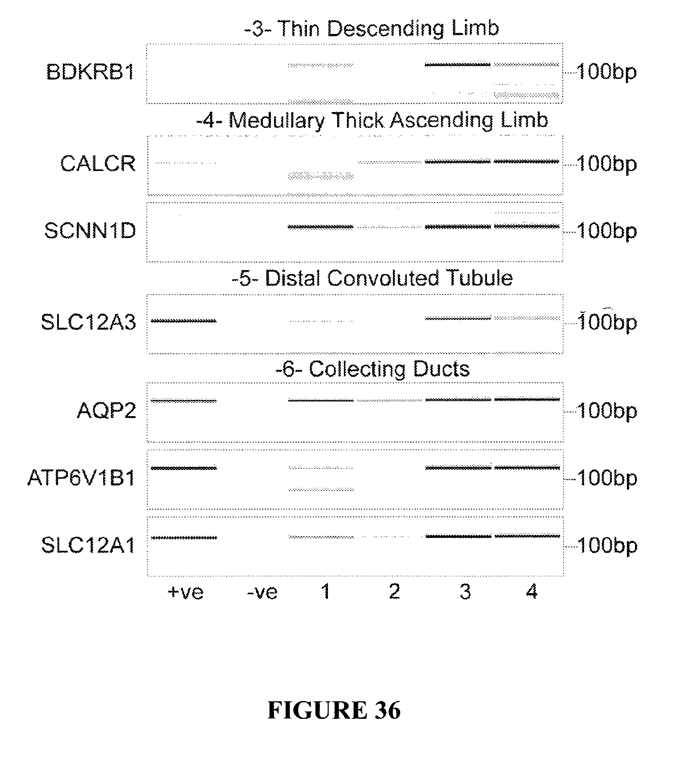

FIGURE 36 represents BioAnalyzer generated pseudo gel profiles of the identification of mRNA transcripts encoding specific genes from regions 3-6 of the nephron and collecting duct detected by RT-PCR of RiboAmped mRNA from urinary microvesicles, specifically: **3.** Thin Descending Limb: BDKRB1 - bradykinin B1 receptor. **4.** Medullary Thick Ascending Limb: CALCR - calcitonin receptor, SCNN1D - amiloride-sensitive sodium channel subunit delta. **5.** Distal Convoluted Tubule: SLC12A3 - thiazide-sensitive sodium-chloride cotransporter. **6.** Collecting Ducts: AQP2 - aquaporin 2, ATP6V1B1 - vATPase B1 subunit, SLC12A1 - Kidney-specific Na-K-Cl symporter.
FIGURE 37 **A**) is a pair of BioAnalyzer pseudo gels depicting the expression of the V-ATPase B1 subunit and AQP2 mRNA by RT-PCR in V-ATPase B1 KO (B1 -/-) and wild type (B1 +/+) mice; **B**) is a pair of charts depicting the expression of the V-ATPase B2 subunit in urinary microvesicles and kidney cells from V-ATPase B1 KO (B1 -/-) and wild type (B1 +/+) mice by real-time PCR analysis. "NS" - not statistically significant.
FIGURE 38 is a trio of plots depicting RNA profiles generated from urinary microvesicles. Urinary microvesicles were not washed or treated with any extraction enhancer before the microvesicle membranes were broken for nucleic acid extraction. Three samples were used in this group of extractions. The profiles are shown in **A**, **B** and **C**, respectively.
FIGURE 39 is a trio of plots depicting RNA profiles generated from urinary microvesicles. Urinary microvesicles were not washed but were treated with a RNase inhibitor, RNase-In (Promega), before the microvesicle membranes were broken for nucleic acid extraction. Three samples were used in this group of extractions. The profiles are shown in **A**, **B** and **C**, respectively.
FIGURE 40 is a trio of plots depicting RNA profiles generated from urinary microvesicles. Urinary microvesicles were washed but were not treated with any RNase inhibitor before the microvesicle membranes were broken for nucleic acid extraction. Three samples were used in this group of extractions. The profiles are shown in **A**, **B** and **C**, respectively.
FIGURE 41 is a trio of plots depicting RNA profiles generated from urinary microvesicles. Urinary microvesicles were washed and treated with RNase inhibitor before microvesicle membranes were broken for nucleic acid extraction. Three samples were used in this group of extractions. The profiles are shown in **A**, **B** and **C**, respectively.
FIGURE 42 is a list of chromosome regions in which there were more than 500 transcript hits in a deep sequencing analysis of RNA extracted from urinary microvesicles ("spikes"). The numbers indicate the start and end point of each chromosomal region. For example, "chr1.-1.91625366.91625741" refers to the region on human chromosome 1 between nucleotide nos. 91625366 and 91625741. The corresponding SEQ ID NOS are also indicated.
FIGURE 43 is a list of primers used for PCR reaction to amply sequences in 10 chromosome regions as indicated. For example, "chr1.-1.91625366.91625741" refers to the region on human chromosome 1 between nucleotide nos. 91625366 and 91625741. The primer pair used to amplify this region was "tccagctcacgttccctatt 1L and ccaggtggggagtttgact 1 R". Primers run from 5' to 3' as they go from left to right.
FIGURE 44 is a pair of BioAnalyzer pseudo gels depicting the result of PCR amplification of 10 spike-rich chromosome regions. The numbering of the lanes at the top of each frame corresponds to the numbering of the chromosome regions shown in Figure 43. In **A**, a nucleic acid extraction from urinary microvesicles was used as a template for the PCR. In **B**, a nucleic acid extraction from renal tissue was used as a template for the PCR.
FIGURES 45-73 are plots depicting the spikes in 29 chromosome regions. The regions are indicated at the top of each plot. For example, the plot in Figure 46 refers to the region of "chr1.-1.91625366.91625741," which is the region on human chromosome 1 between nucleotide nos. 91625366 and 91625741.

### DETAILED DESCRIPTION

Microvesicles are shed by eukaryotic cells, or budded off of the plasma membrane, to the exterior of the cell. These membrane vesicles are heterogeneous in size with diameters ranging from about 10 nm to about 5000 nm. The small microvesicles (approximately 10 to 1000 nm, and more often approximately 10 to 200 nm in diameter) that are released by exocytosis of intracellular multivesicular bodies are referred to in the art as "exosomes." The compositions, methods and uses described herein are equally applicable to microvesicles of all sizes; preferably 10 to 800 nm; and more preferably 10 to 200 nm.

In some of the literature, the term "exosome" also refers to protein complexes containing exoribonucleases which are involved in mRNA degradation and the processing of small nucleolar RNAs (snoRNAs), small nuclear RNAs (snRNAs) and ribosomal RNAs (rRNA) (Liu et al., 2006; van Dijk et al., 2007). Such protein complexes do not have membranes and are not "microvesicles" or "exosomes" as those terms are used here in.

The present invention is partly based on the discovery that adverse factors can prevent an effective extraction of nucleic acids from a biological sample and that novel and unexpected agents and steps may be used to mitigate or remove the adverse factors, thereby dramatically improving the quality of the extracted nucleic acids. As such, described herein are novel methods for extracting high quality nucleic acids from a biological sample. The high quality extractions obtained by the novel methods described herein are characterized by high yield and high integrity, making the extracted nucleic acids useful for various applications in which high quality nucleic acid extractions are preferred.

Broadly described, the novel methods include, for example, the steps of obtaining a biological sample, mitigating or removing the adverse factors that prevent an effective extraction of nucleic acids from a biological sample, and extracting nucleic acids from the biological sample followed, optionally, by nucleic acid analysis.

Applicable biological samples include, for example, a cell, a group of cells, fragments of cells, cell products including for example microvesicles, cell cultures, bodily tissues from a subject, or bodily fluids. The bodily fluids can be fluids isolated from anywhere in the body of the subject, preferably a peripheral location, including but not limited to, for example, blood, plasma, serum, urine, sputum, spinal fluid, pleural fluid, nipple aspirates, lymph fluid, fluid of the respiratory, intestinal, and genitourinary tracts, tear fluid, saliva, breast milk, fluid from the lymphatic system, semen, cerebrospinal fluid, intra-organ system fluid, ascitic fluid, tumor cyst fluid, amniotic fluid and combinations thereof.

A biological sample may sometimes come from a subject. The term "subject" is intended to include all animals shown to or expected to have microvesicles. The subject may be a mammal, a human or nonhuman primate, a dog, a cat, a horse, a cow, other farm animals, or a rodent (e.g. mouse, rat, guinea pig, etc.). The term "subject" and "individual" are used interchangeably herein.

A biological sample may optionally be processed to obtain a biological sample derivative before, after, or at the same time as, carrying out the step of mitigating or removing the adverse effects. The biological sample derivative may be a cell, cell debris, a membrane vesicle, or a microvesicle.

A biological sample is sometimes pre-processed before a biological sample derivative such as a microvesicle is obtained. In some instances, the pre-processing step is preferred. For example, a urine sample may be pre-processed to obtain urinary microvesicles. The pre-processing may be achieved by techniques known in the art such as low speed centrifugation and pre-filtration. For example, urine samples may undergo a first centrifugation step of 300g to get rid of large particles in the samples. Urine samples may undergo a second centrifugation step of 17,000g to get rid of smaller particles in the samples. After the second centrifugation step, urine samples may further undergo a pre-filtration step, e.g., a 0.8um pre-filtration step. Alternatively, urine samples may be pre-processed by a - pre-filtration step without first undergoing the one or more of the centrifugation steps.

Membrane vesicles, e.g., microvesicles, may be isolated from a biological sample. In some instances, such isolation may be carried out without pre-processing the biological sample. In other instances, such isolation may be carried out after the biological sample is pre-processed. The isolation step may be advantageous for high quality nucleic acid extraction from a biological sample. For example, the isolation may give rise to advantages such as: 1) the opportunity to selectively analyze disease- or tumor-specific nucleic acids, which may be obtained by isolating disease- or tumor-specific microvesicles apart from other microvesicles within the fluid sample; 2) significantly higher yield of nucleic acid species with higher integrity as compared to the yield/integrity obtained by extracting nucleic acids directly from the fluid sample; 3) scalability, e.g. to detect nucleic acids expressed at low levels, the sensitivity can be increased by pelleting more microvesicles from a larger volume of serum; 4) purer nucleic acids in that protein and lipids, debris from dead cells, and other potential contaminants and PCR inhibitors are excluded from the microvesicle pellets before the nucleic acid extraction step; and 5) more choices in nucleic acid extraction methods as microvesicle pellets are of much smaller volume than that of the starting serum, making it possible to extract nucleic acids from these microvesicle pellets using small volume column filters.

Methods of isolating microvesicles from a biological sample are known in the art. For example, a method of differential centrifugation is described in a paper by Raposo et al. (Raposo et al., 1996), a paper by Skog et. al.(Skog et al., 2008) and a paper by Nilsson et. al.(Nilsson et al., 2009). Methods of anion exchange and/or gel permeation chromatography are described in US Patent Nos. 6,899,863 and 6,812,023. Methods of sucrose density gradients or organelle electrophoresis are described in U.S. Patent No. 7,198,923. A method of magnetic activated cell sorting (MACS) is described in a paper by Taylor and Gercel-Taylor (Taylor and Gercel-Taylor, 2008). A method of nanomembrane ultrafiltration concentration is described in a paper by Cheruvanky et al. (Cheruvanky et al., 2007). Further, microvesicles can be identified and isolated from bodily fluid of a subject by a newly developed microchip technology that uses a unique microfluidic platform to efficiently and selectively separate tumor-derived microvesicles (Chen et al.).

The microvesicles isolated from a bodily fluid may be enriched for those originating from a specific cell type, for example, lung, pancreas, stomach, intestine, bladder, kidney, ovary, testis, skin, colorectal, breast, prostate, brain, esophagus, liver, placenta, fetus cells. Because the microvesicles often carry surface molecules such as antigens from their donor cells, surface molecules may be used to identify, isolate and/or enrich for microvesicles from a specific donor cell type (Al-Nedawi et al., 2008; Taylor and Gercel-Taylor, 2008). In this way, microvesicles originating from distinct cell populations can be analyzed for their nucleic acid content. For example, tumor (malignant and non-malignant) microvesicles carry tumor-associated surface antigens and may be detected, isolated and/or enriched via these specific tumor-associated surface antigens. In one example, the surface antigen is epithelial-cell-adhesion-molecule (EpCAM), which is specific to microvesicles from carcinomas of lung, colorectal, breast, prostate, head and neck, and hepatic origin, but not of hematological cell origin (Balzar et al., 1999; Went et al., 2004). In another example, the surface antigen is CD24, which is a glycoprotein specific to urine microvesicles (Keller et al., 2007). In yet another example, the surface antigen is selected from a group of molecules such as CD70, carcinoembryonic antigen (CEA), EGFR, EGFRvIII and other variants, Fas ligand, TRAIL, transferrin receptor, p38.5, p97 and HSP72. Additionally, tumor specific microvesicles may be characterized by the lack of surface markers, such as CD80 and CD86.

The isolation of microvesicles from specific cell types can be accomplished, for example, by using antibodies, aptamers, aptamer analogs or molecularly imprinted polymers specific for a desired surface antigen. The surface antigen may be specific for a cancer type. The surface antigen may be specific for a cell type which is not necessarily cancerous. One example of a method of microvesicle separation based on cell surface antigen is provided in U.S. Patent No. 7,198,923. As described in, e.g., U.S. Patent Nos. 5,840,867 and 5,582,981, WO/2003/050290 and a publication by Johnson et al. (Johnson et al., 2008), aptamers and their analogs specifically bind surface molecules and can be used as a separation tool for retrieving cell type-specific microvesicles. Molecularly imprinted polymers also specifically recognize surface molecules as described in, e.g., US Patent Nos. 6,525,154, 7,332,553 and 7,384,589 and a publication by Bossi et al. (Bossi et al., 2007) and are a tool for retrieving and isolating cell type-specific microvesicles.

In instances when the intended biological derivative is a membrane vesicle such as a microvesicle, a step of removing nucleic acids that are not inside the microvesicle is sometimes performed. Methods of removing nucleic acids are well known in the art. For example, to remove such nucleic acids from a sample, an enzyme digestion step may be performed. Such enzymes may be a type of ribonuclease that catalyzes the enzymatic digestion of ribonucleic acids or a type of deoxyribonuclease that catalyzes the enzymatic digestion of deoxyribonucleic acids.

The novel nucleic acid extraction methods may include a step of removing or mitigating adverse factors that prevent high quality nucleic acid extraction from a biological sample. Such adverse factors are heterogeneous in that different biological samples may contain various species of such adverse factors. In some biological samples, factors such as excessive extra-microvesicle DNA may affect the quality of nucleic acid extractions from such samples and contaminate DNA extracted from within microvesicle. In other samples, factors such as excessive endogenous RNase may affect the quality of nucleic acid extractions from such samples. Many agents and methods may be used to remove these adverse factors. These methods and agents are referred to collectively as an "extraction enhancement operation."

In some instances, the extraction enhancement operation may involve the addition of nucleic acid extraction enhancement agents to the biological sample or derivative. To remove adverse factors such as endogenous RNases, such extraction enhancement agents as defined here may include, but are not limited to, a commercially available RNase inhibitor such as Superase-In (Ambion Inc.), RNaseIN (Promega Corp.), or other agents that function in a similar fashion; a protease; a reducing agent; a decoy substrate such as a synthetic RNA; a soluble receptor that can bind RNase; a small interfering RNA (siRNA); a RNA binding molecule, such as an anti-RNA antibody, or a chaperone protein; a RNase denaturing substance, such as a high osmolarity solution, a detergent, or a combination thereof. These enhancement agents may exert their functions in various ways, for example, but not limited to, through inhibiting RNase activity (e.g., RNase inhibitors), through a ubiquitous degradation of proteins (e.g., proteases), or through a chaperone protein (e.g., a RNA-binding protein) that binds and protects RNAs. In all instances, such extraction enhancement agents remove or mitigate some or all of the adverse factors in the biological sample that would otherwise prevent or interfere with the high quality extraction nucleic acids from the biological sample.

In other instances, the extraction enhancement operation may involve the performance of one or more process steps. Such processes include extensive or substantially thorough washing of nucleic acid-containing components of the sample, such as microvesicles; size separation of RNases from the biological sample; denaturation of proteins in the biological sample by various techniques including, but not limited to, generating a particular pH condition, a temperature condition, (e.g., the maintenance of a decreasing or lower temperature), freeze/thaw cycles, and combinations thereof.

One surprising manifestation of the use of extraction enhancement operations, as described herein, is the ability to detect in an extraction of nucleic acid from microvesicles the existence of significant quantities of ribosomal RNA (rRNA). No prior studies are known to have demonstrated the detection of 18S and 28S rRNA in microvesicle nucleic acid extractions. On the contrary, prior studies suggested that no or little rRNA is present in nucleic acid extracts from microvesicles (Skog et al., 2008; Taylor and Gercel-Taylor, 2008; Valadi et al., 2007).

The performance of an extraction enhancement operation will improve the quality of extracted RNA in terms of RNA integrity number (RIN). Designed by Agilent Technologies (http://www.chem.agilent.com/en-us/products/instruments/lab-on-a-chip/pages/gp14975.aspx, accessed July 15, 2010), the RNA integrity number (RIN) is the product of a software tool designed to estimate the integrity of total RNA samples. The software automatically assigns an integrity number to an eukaryote total RNA sample. Using this tool, sample integrity is not determined by the ratio of the 18S and 28S ribosomal bands, but by the entire electrophoretic trace of the RNA sample. This includes the presence or absence of degradation products. The assigned RIN is independent of sample concentration, instrument, and analyst, and can serve as a standard for RNA integrity.

The performance of an extraction enhancement operation will improve the quantity or yield of extracted nucleic acid. For example, using an extraction enhancement operation, as described herein, one may obtain a nucleic acid yield of greater than or equal to 50 pg/ml from a 20 ml low protein biological sample such as urine. Alternatively, one may obtain a nucleic acid yield of greater than or equal to 50 pg/ml from 1 ml of a high protein biological sample, such as serum or plasma.

Novel high quality nucleic acid extractions obtained by the methods described herein may display a combination of the detection of 18S and 28S rRNA, preferably in a ratio of approximately 1:1 to approximately 1:2; and more preferably, approximately 1:2; a RNA integrity number of greater than or equal to 5 for a low protein biological sample, or greater than or equal to 3 for a high protein biological sample; and a nucleic acid yield of greater than or equal to 50 pg/ml from a 20 ml low protein biological sample or a 1 ml high protein biological sample.

High quality RNA extractions are highly desirable because RNA degradation can seriously affect downstream assessment of the extracted RNA, such as in gene expression and mRNA analysis, as well as analysis of non-coding RNA such as small RNA and micro RNA. The novel methods described herein enable one to extract high quality nucleic acids from a biological sample such as microvesicles so that an accurate analysis of gene expression and mutational level within the exosomes can be carried out. For example, when increased concentrations of protease (5X, 10X) are used as an extraction enhancing agent, the amount and integrity of RNA isolated from urinary microvesicles is increased significantly.

Also provided are methods of extracting high quality small RNA from a biological sample such as urine. Small RNA, such as miRNA is particularly susceptible to degradation and loss during the process of nucleic acid extraction. In the novel methods here disclosed, a high concentration of protease is used to remove or mitigate adverse factors that prevent high quality extraction of small RNAs. A method to extract nucleic acid, particularly small RNA, may use 25X and 50X protease as extraction enhancing agent and is able to obtain significantly increased amounts of small RNA. As used herein, expressions such as 5X, 10X, 25X and 50X mean 5 times, 10 times, etc. the activity level of protease currently used or recommended in commercially available nucleic acid extraction kits such as the QIAamp MinElute Virus Spin Kit.

When the adverse factors affecting extraction have been removed or mitigated, nucleic acid molecules can be isolated from a biological sample using any number of procedures that are well-known in the art. Persons of skill will select a particular isolation procedure as being appropriate for the particular biological sample. Examples of methods for extraction are provided in the Examples section herein. In some instances, with some techniques, it may also be possible to analyze the nucleic acid without extraction from the microvesicle.

The extracted nucleic acids, including DNA and/or RNA, may be analyzed directly without an amplification step. Direct analysis may be performed with different methods including, but not limited to, nanostring technology. NanoString technology enables identification and quantification of individual target molecules in a biological sample by attaching a color coded fluorescent reporter to each target molecule. This approach is similar to the concept of measuring inventory by scanning barcodes. Reporters can be made with hundreds or even thousands of different codes allowing for highly multiplexed analysis. The technology is described in a publication by Geiss et al. (Geiss et al., 2008).

It may be beneficial or otherwise desirable to amplify the nucleic acid of the microvesicle prior to analyzing it. Methods of nucleic acid amplification are commonly used and generally known in the art, many examples of which are described herein. If desired, the amplification can be performed such that it is quantitative. Quantitative amplification will allow quantitative determination of relative amounts of the various nucleic acids, to generate a profile as described below.

In one embodiment, the extracted nucleic acid is RNA. The RNA is then preferably reverse-transcribed into complementary DNA (cDNA) before further amplification. Such reverse transcription may be performed alone or in combination with an amplification step. One example of a method combining reverse transcription and amplification steps is reverse transcription polymerase chain reaction (RT-PCR), which may be further modified to be quantitative, e.g., quantitative RT-PCR as described in US Patent No. 5,639,606.

Nucleic acid amplification methods include, without limitation, polymerase chain reaction (PCR) (US Patent No. 5,219,727) and its variants such as in situ polymerase chain reaction (US Patent No. 5,538,871), quantitative polymerase chain reaction (US Patent No. 5,219,727), nested polymerase chain reaction (US Patent No. 5,556,773), self-sustained sequence replication and its variants (Guatelli et al., 1990), transcriptional amplification system and its variants (Kwoh et al., 1989), Qb Replicase and its variants (Miele et al., 1983), cold-PCR (Li et al., 2008), or any other nucleic acid amplification methods, followed by the detection of the amplified molecules using techniques well known to those of skill in the art. Especially useful are those detection schemes designed for the detection of nucleic acid molecules if such molecules are present in very low numbers.

The analysis of nucleic acids present in the microvesicles is quantitative and/or qualitative. For quantitative analysis, the amounts (expression levels), either relative or absolute, of specific nucleic acids of interest within the microvesicles are measured with methods known in the art (described below). For qualitative analysis, the species of specific nucleic acids of interest within the microvesicles, whether wild type or variants, are identified with methods known in the art.

Also described is a novel composition of matter, a nucleic acid extraction from microvesicles in which 18S and 28S rRNA is detectable in the extraction. Such nucleic acid extractions may be achieved using the novel nucleic acid extraction method described herein. A high quality nucleic acid extraction from microvesicles in a biological sample is desirable in many instances. In some instances, a tissue sample is not easily accessible. For example a brain tumor sample can not usually be obtained without brain surgery. Instead, a microvesicle sample from the brain tumor patient serum is easily accessible. In order to analyze nucleic acids in brain tumor cells, it is easier to analyze nucleic acids in serum microvesicles that are secreted by brain tumor cells. Therefore, in instances where nucleic acids in microvesicles secreted by tissue cells are used to substitute nucleic acids from tissue cells, it is desirable to obtain high quality nucleic acids which, like those obtained from tissue cells directly, contain detectable quality controls, such as 18S and 28S rRNA. In other instances, high quality small RNA is desirable. Nucleic acid extractions disclosed herein contain such high quality small RNA together with 18S and 28S rRNA. Such high quality small RNA is important for the accurate assessment of nucleic acids for various purposes, e.g., the expression level of a particular miRNA.

Also described is a novel, high-quality profile of nucleic acids from microvesicles in a biological sample. Such profiles are generated by analyzing nucleic acid extractions that contain 18S and 28S rRNA. Such profiles may be obtained with the novel methods disclosed herein. High quality nucleic acid profiles are highly desirable for many uses, such as for use as a biomarker for a medical condition or therapy selection. It is desirable in that such profiles are consistent between samples. Such consistency can hardly be achieved without high quality nuclei acid extractions. A profile of nucleic acids can be obtained by analyzing nucleic acids in microvesicles that are secreted by those cells of origin. Such microvesicles can be isolated from an easily accessible biological sample, e.g., urine, serum or plasma. Such profiles of nucleic acids many include small RNAs, messenger RNA, microRNA, non-coding RNAs or a combination thereof. Such profiles of nucleic acids may be combined with other biomarkers to more accurately achieve certain results.

The profile of nucleic acids for instance can be a collection of genetic aberrations, which is used herein to refer to the nucleic acid amounts as well as nucleic acid variants within the microvesicles. Specifically, genetic aberrations include, without limitation, over-expression of a gene (e.g., oncogenes) or a panel of genes, under-expression of a gene (e.g., tumor suppressor genes such as p53 or RB) or a panel of genes, alternative production of splice variants of a gene or a panel of genes, gene copy number variants (CNV) (e.g. DNA double minutes) (Hahn, 1993), nucleic acid modifications (e.g., methylation, acetylation and phosphorylations), single nucleotide polymorphisms (SNPs), chromosomal rearrangements (e.g., inversions, deletions and duplications), and mutations (insertions, deletions, duplications, missense, nonsense, synonymous or any other nucleotide changes) of a gene or a panel of genes, which mutations, in many cases, ultimately affect the activity and function of the gene products, lead to alternative transcriptional splice variants and/or changes of gene expression level.

The determination of such genetic aberrations can be performed by a variety of techniques known to the skilled practitioner. For example, expression levels of nucleic acids, alternative splicing variants, chromosome rearrangement and gene copy numbers can be determined by microarray analysis (US Patent Nos. 6,913,879, 7,364,848, 7,378,245, 6,893,837 and 6,004,755) and quantitative PCR. Particularly, copy number changes may be detected with the Illumina Infinium II whole genome genotyping assay or Agilent Human Genome CGH Microarray (Steemers et al., 2006). Nucleic acid modifications can be assayed by methods described in, e.g., US Patent No. 7,186,512 and patent publication WO/2003/023065. Particularly, methylation profiles may be determined by Illumina DNA Methylation OMA003 Cancer Panel. SNPs and mutations can be detected by hybridization with allele-specific probes, enzymatic mutation detection, chemical cleavage of mismatched heteroduplex (Cotton et al., 1988), ribonuclease cleavage of mismatched bases (Myers et al., 1985), mass spectrometry (US Patent Nos. 6,994,960, 7,074,563, and 7,198,893), nucleic acid sequencing, single strand conformation polymorphism (SSCP) (Orita et al., 1989), denaturing gradient gel electrophoresis (DGGE)(Fischer and Lerman, 1979a; Fischer and Lerman, 1979b), temperature gradient gel electrophoresis (TGGE) (Fischer and Lerman, 1979a; Fischer and Lerman, 1979b), restriction fragment length polymorphisms (RFLP) (Kan and Dozy, 1978a; Kan and Dozy, 1978b), oligonucleotide ligation assay (OLA), allele-specific PCR (ASPCR) (US Patent No. 5,639,611), ligation chain reaction (LCR) and its variants (Abravaya et al., 1995; Landegren et al., 1988; Nakazawa et al., 1994), flow-cytometric heteroduplex analysis (WO/2006/113590) and combinations/modifications thereof. Notably, gene expression levels may be determined by the serial analysis of gene expression (SAGE) technique (Velculescu et al., 1995). In general, the methods for analyzing genetic aberrations are reported in numerous publications, not limited to those cited herein, and are available to skilled practitioners. The appropriate method of analysis will depend upon the specific goals of the analysis, the condition/history of the patient, and the specific cancer(s), diseases or other medical conditions to be detected, monitored or treated.

It should be understood that this invention is not limited to the particular methodologies, protocols and reagents, described herein, which may vary. The terminology used herein is for the purpose of describing particular embodiments only, and is not intended to limit the scope of the present invention, which is defined solely by the claims.

Examples of the presently disclosed subject matter are set forth below. Other features, objects, and advantages of the presently disclosed subject matter will be apparent from the detailed description, figures, examples and claims. Methods, devices, and materials substantially similar or equivalent to those described herein can be used in the practice or testing of the presently disclosed subject matter. Exemplary methods, devices, uses and materials are now described.

### Microvesicles in Urine

### Example 1: Renal cells contain multivesicular bodies

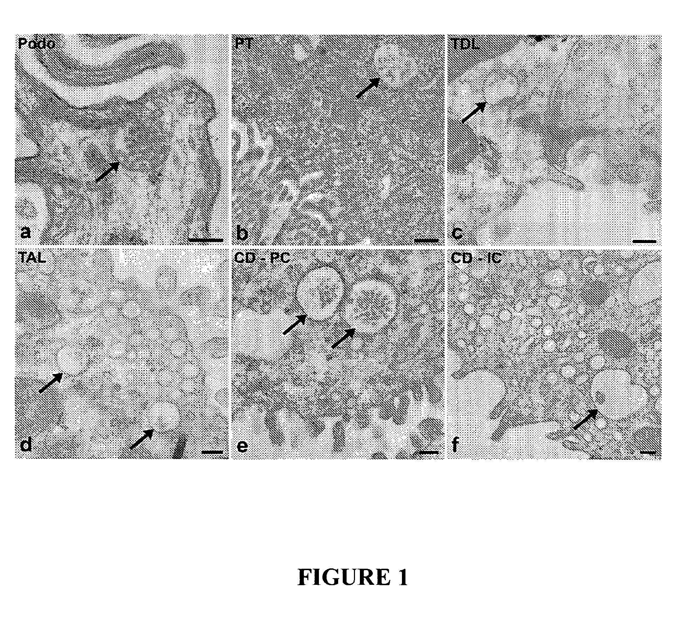

To examine whether renal cells shed microvesicles, we used Transmission Electron Microscopy (TEM) to determine whether renal cells contain multivesicular bodies that can give rise to microvesicles. Rat kidney was fixed by intravascular perfusion with 2.0% glutaraldehyde in 0.1 M sodium cacodylate buffer, pH 7.4 (Electron Microscopy Sciences, PA), and kidney slices were further fixed overnight at 4°C. The sample slices were rinsed in 0.1 M sodium cacodylate buffer, post-fixed in 1.0% osmium tetroxide in cacodylate buffer for 1 h at room temperature, rinsed in buffer again, then rinsed in distilled water (dH₂O) and stained, *en bloc,* in an aqueous solution of 2.0% uranyl acetate for 1 hour at room temperature. The samples were rinsed in distilled water and dehydrated through a graded series of ethanol to 100%. The samples were infiltrated with Epon resin (Ted Pella, CA) by overnight immersion in a 1:1 solution of Epon:ethanol. The following day samples were placed in fresh Epon for several hours and embedded in Epon overnight at 60°C. Thin sections were cut on a Reichert Ultracut E ultramicrotome, collected on formvar-coated grids, stained with uranyl acetate and lead citrate. Samples were examined in a JEOL JEM 1011 transmission electron microscope at 80 kV. Images were collected using an AMT (Advanced Microscopy Techniques, MA) digital imaging system. As shown in **Figure 1**, transmission electron microscope (TEM) images of MVBs are seen in rat renal tissue cells. Multivesicular bodies (MVB) can be identified in various regions of the nephron and collecting duct, including the podocyte, the proximal tubule, thin descending limb, thick ascending limb, collecting duct principal cell, and collecting duct intercalated cell. This demonstrates that exosomes can indeed be released from various regions of the nephron as well as both intercalated and principal cells of the collecting duct.

### Example 2: Microvesicles exist in urine

To examine microvesicles themselves, we examined human urinary microvesicles by TEM. Human urine was obtained under the approved IRB guidelines of the Massachusetts General Hospital. Urine was then pre-processed by a method consisting of three steps: centrifugation of the urine at 300g for 10 min at 4°C, centrifugation of the supernatant at 17,000g for 20 min at 4°C, and filtration of the supernatant through a 0.8 µm filter (cellulose nitrate membrane filter unit, Nalgene, NY). Alternatively, urine was pre-processed by a one-step filtration directly through a 0.8 µm filter without any pre-centrifugation steps. In either case, the filtrate then underwent ultracentrifugation at 118,000g for 70 min at 4°C, the supernatant was removed and the microvesicle-containing pellet was washed in PBS and re-pelleted at 118,000g for 70 min at 4°C.

Instead of ultracentrifugation, filtration concentration was also used to isolate microvesicles from pre-processed samples. The filtrate concentrator (100kDa MWCO) (Millipore, MA) was prepared according to the manufacturer's instructions. Pre-processed filtrate was added to the filtration concentrator and centrifuged at 4,000g for 4 min at RT. A 15 ml PBS wash step was included.

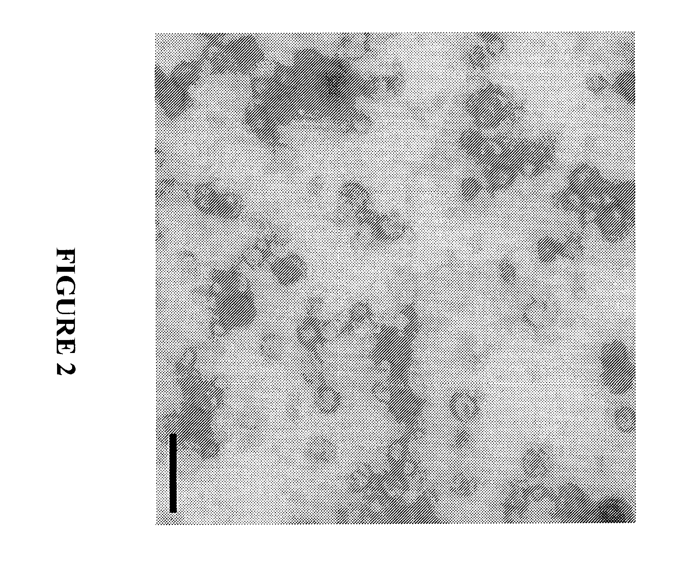

Microvesicle pellets were fixed, 1:1 with 4% paraformaldehyde in dH₂O. Ten (10) µl drops were pipetted onto formvar-coated 200 mesh gold grids and drawn off after one minute. Samples were rinsed 2 times with drops of dH₂O. Aqueous 2.0% phosphotungstic acid (PTA) was applied (10 µl) for 10 sec, drawn off and rinsed once with dH₂O Samples were examined in a JEOL JEM 1011 transmission electron microscope at 80 kV. Images were collected using an AMT (Advanced Microscopy Techniques, MA) digital imaging system. As shown in **Figure 2**, the pellet was indeed rich in microvesicles. The microvesicles sometimes aggregate together or remain singular in the TEM image.

### Improved Methods for Nucleic Acid Extraction from a Biological Sample

### Example 3: Ultracentrifugation is replaceable with filtration concentrator for purposes of microvesicle isolation

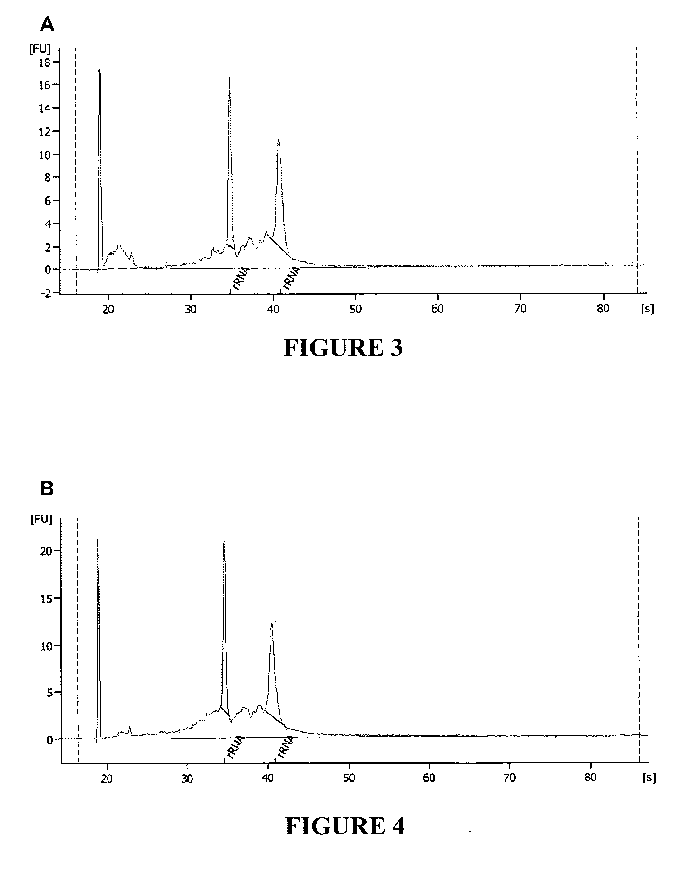

We show here that filtration concentrators can yield viable microvesicles for RNA extraction similar to the ultracentrifugation method. We pre-processed 75 ml urine by centrifugation at 300g for 10 minutes at 4°C and 17,000g for 20 minutes at 4°C, and then filtered through a 0.8 µm filter as detailed in Example 2. We then isolated microvesicles via a 100kDa MWCO filtration concentrator (Millipore, MA) and via ultracentrifugation both with RNase digestion, respectively, and with and without DNase digestion to remove extra-microvesicular nucleic acid contamination. As shown in **Figures 3 and 4**, the ultracentrifugation method and filtration concentration method yielded similar RNA concentrations from the 75 ml urine samples with ultracentrifugation **(Figure 4)** at 410 ± 28 pg/µl, and filtration concentrator **(Figure 3)** at 381 ± 47 pg/µl (Mean ± SD). There is no statistically significant difference between the two yields. These data demonstrate that the use of filtration concentrators is a reliable method for isolating urinary microvesicles for RNA analysis.

### Example 4: Sample pre-processing with only a 0.8um pre-filtration step is sufficient for purpose of microvesicle isolation

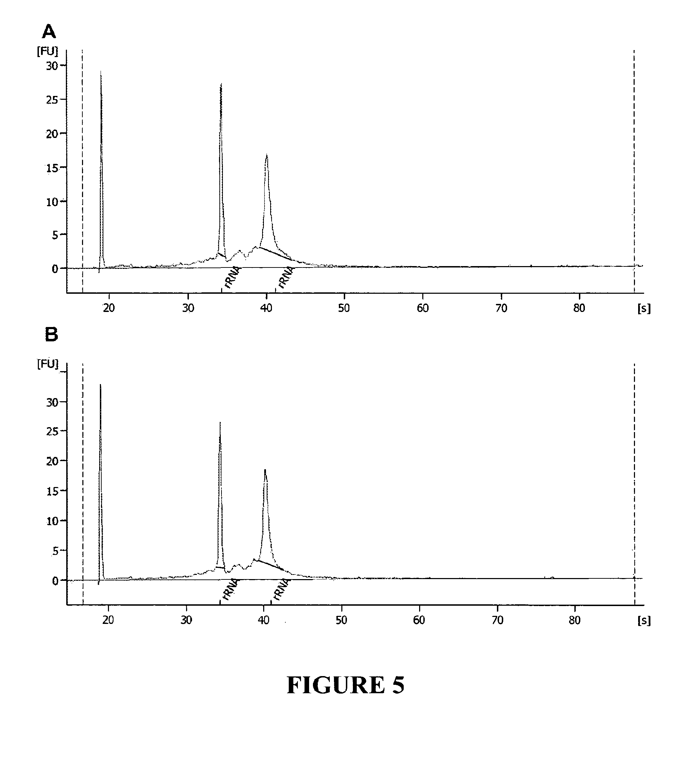

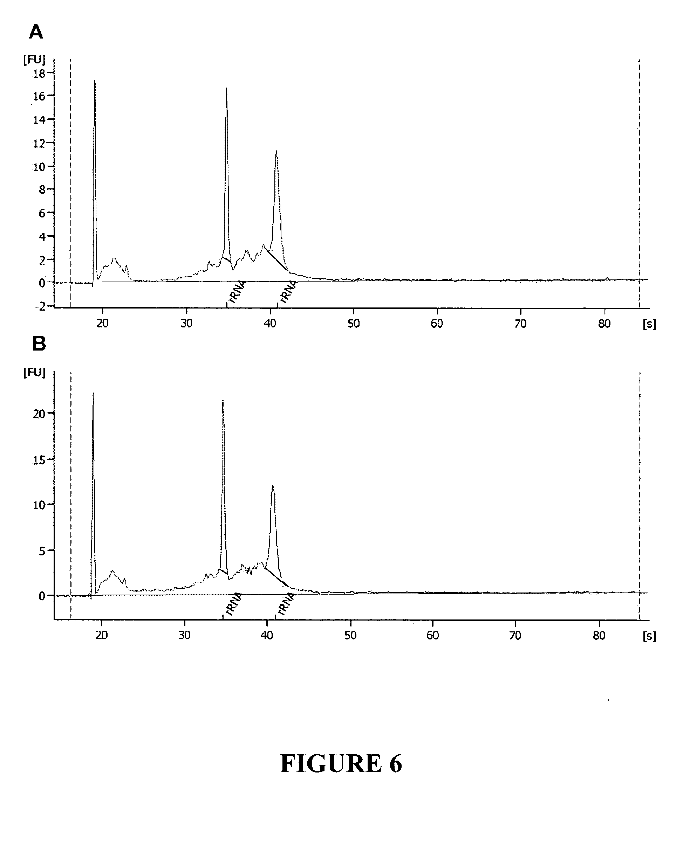

Further, we found that the low speed centrifugation steps at 300g and 17,000g could be eliminated because urine pre-processing with just a 0.8 µm pre-filtration step was as effective as methods including the low speed centrifugation steps. As shown in **Figures 5** **and** **6**, the nucleic acid profile using a method of low speed centrifugation coupled with 0.8 µm pre-filtration (**A**) is the same as the profile using a method of only 0.8 µm pre-filtration (**B**).

### Example 5: Nucleic acid extraction from urinary microvesicles with methods that include removal or mitigation of adverse factors

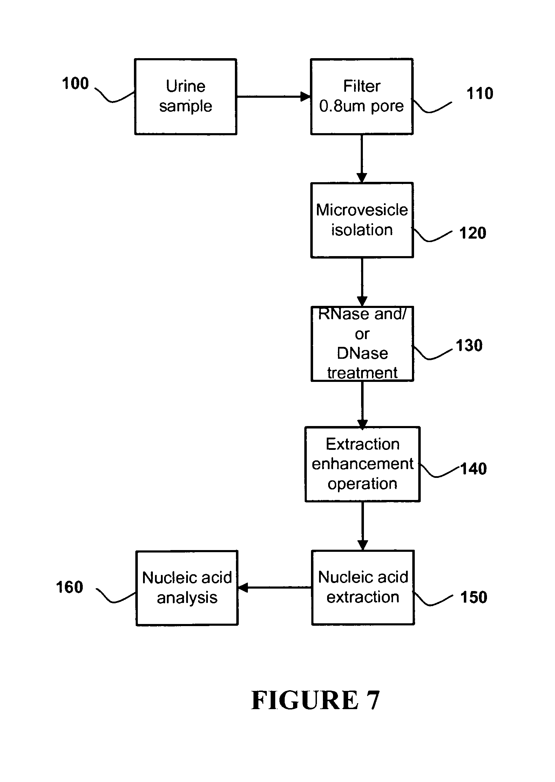

We used an improved method for nucleic acid extraction from microvesicles. In this method, we removed adverse factors for high quality nucleic acid extractions before breaking microvesicular membranes. As shown in **Figure 7**, urine samples **100** were pre-processed by filtering through a 0.8 µm filter membrane **110.** The microvesicles in the filtrates were then isolated either by ultracentrifugation or by filtration concentration **120**, with details similar to those described in Example 2. The isolated microvesicles were then subjected to RNase and/or DNase digestion to remove nucleic acids not contained inside the microvesicles **130.** Specifically, the microvesicles were resuspended in 1 µl/ml RNase A (DNase and protease free) (Fermentas, MD) in PBS and incubated for 1 hr at 37°C. The samples were re-pelleted at 118,000g for 70 min in PBS. For DNase I digestion the pellet was resuspended in 500 µl PBS and DNase I (RNase free)(Qiagen, CA) diluted in RDD buffer (according to manufacturer's instructions) and incubated at room temperature for 10 min. The samples were re-pelleted at 118,000g for 70 min in PBS. For RNase A and DNase I digestion of microvesicles isolated via filtration concentrators, the same concentration of RNase and DNase was used and incubations were carried out in the filtration concentrators. A step of extraction enhancement **140** was then performed, e.g., three resuspension/wash steps with 15 ml of PBS alone, or coupled with a protease treatment. After microvesicles were isolated, digested with nucleases and treated with protease, nucleic acids were extracted **150.** The RNA extraction was performed using the RNeasy Micro kit (Qiagen, CA) according to the manufacturer's instructions. Briefly, 350 µl RLT buffer (with 10 µl betamercaptoethanol per ml RLT) was used to lyse exosomes and 16 µl nuclease-free water was used for elution. The RNeasy Plus Micro kit (Qiagen, CA) is designed to remove genomic DNA (gDNA) and was carried out according to the manufacturer's instructions and eluted in 16 µl nuclease-free water. For small RNA isolation using the RNeasy Micro kit or RNeasy Plus Micro kit, the miRNA isolation method was followed according to the manufacturer's instructions. Isolated RNA was analyzed **160** on a RNA Pico 6000 chip (Agilent, CA) using a Agilent BioAnalyzer (Agilent, CA) which generated an electrophoretic profile and corresponding 'pseudo gel' of the sample.

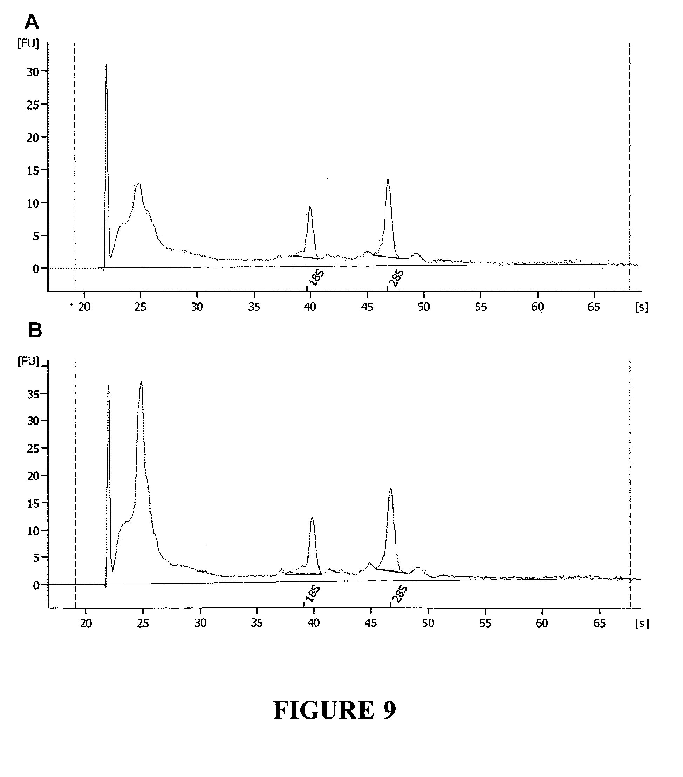

As shown in **Figures 8** **and** **9**, the quality (in terms of yield and integrity) of RNA extraction from urine microvesicles increases as more protease is used to treat microvesicles before breaking the membrane of microvesicles. In **Figure 8**, nucleic acids from microvesicles in 20 ml urine were extracted using the above improved method except that the RNA extraction was performed using the Qiagen Qiamp minelute virus spin kit. The urine sample was concentrated with filtration concentration and eluted in 200 µl PBS. Here, we define 1X protease as 0.027 AU; 5X protease as 0.135 AU; 10X protease as 0.27 AU; 25X protease as 0.675 AU; and 50X as 1.35 AU. 18S and 28S rRNA peaks of greater integrity were observed when the concentration of protease was increased from 5X (**A**) to 10X (**B**). Likewise, as shown in **Figure 9**, at higher concentrations of protease, 25X (**A**) and 50X (**B**), we observed 18S and 28S rRNA of higher integrity, in addition to increased small/miRNA levels. These data suggest that the addition of protease can increase the yield and integrity of 18S and 28S rRNA as well as small RNA and microRNA. We suspect that the effect of protease may be due to its ability to digest away RNases and other inhibitory factors.

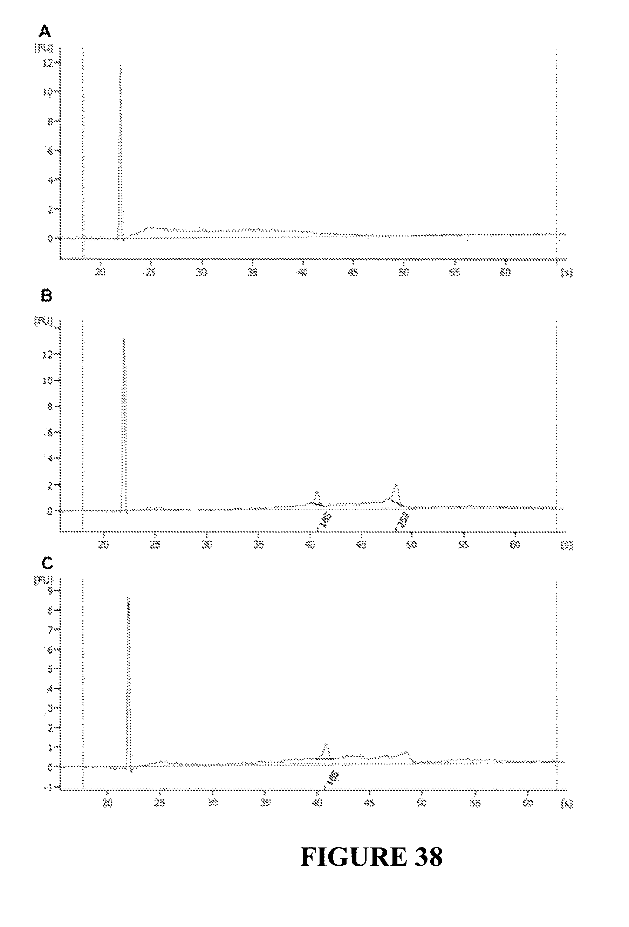

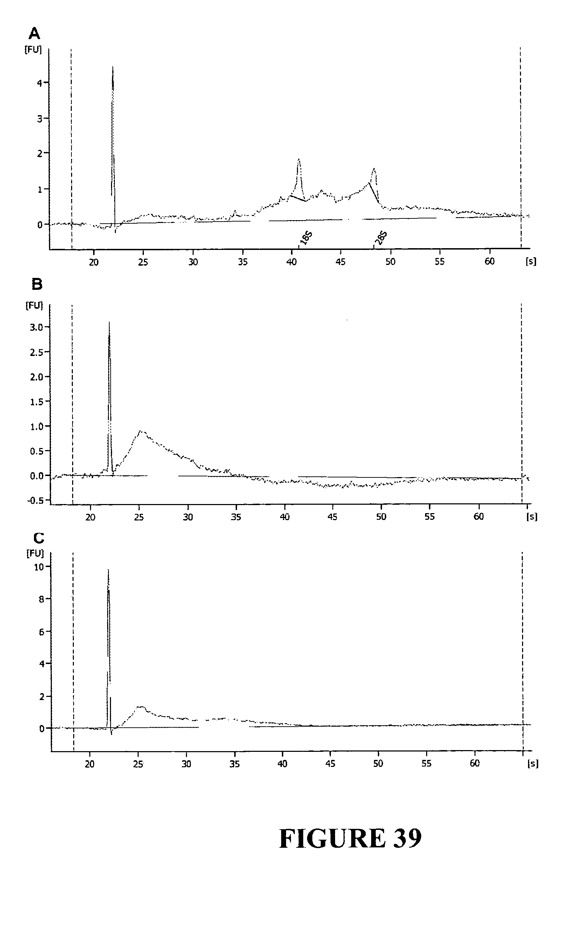

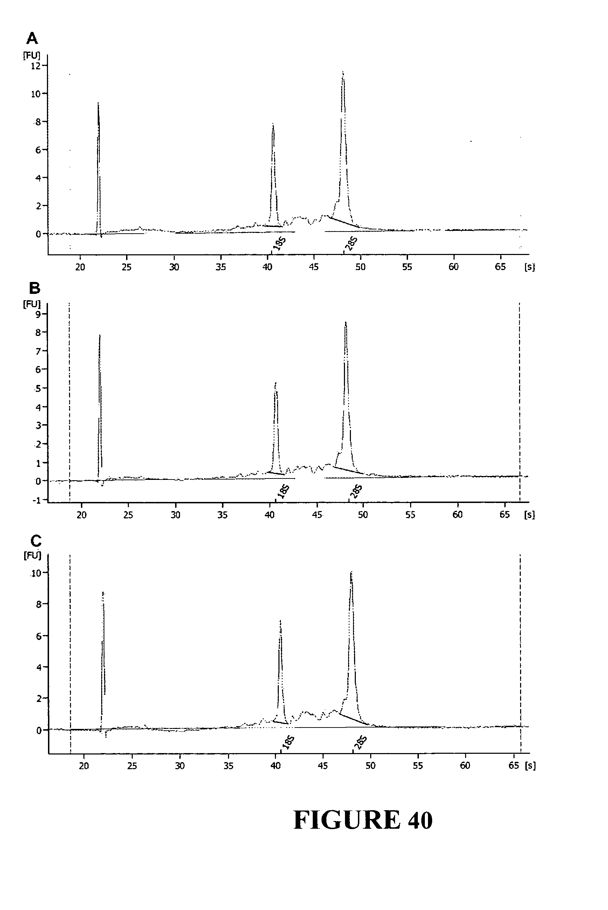

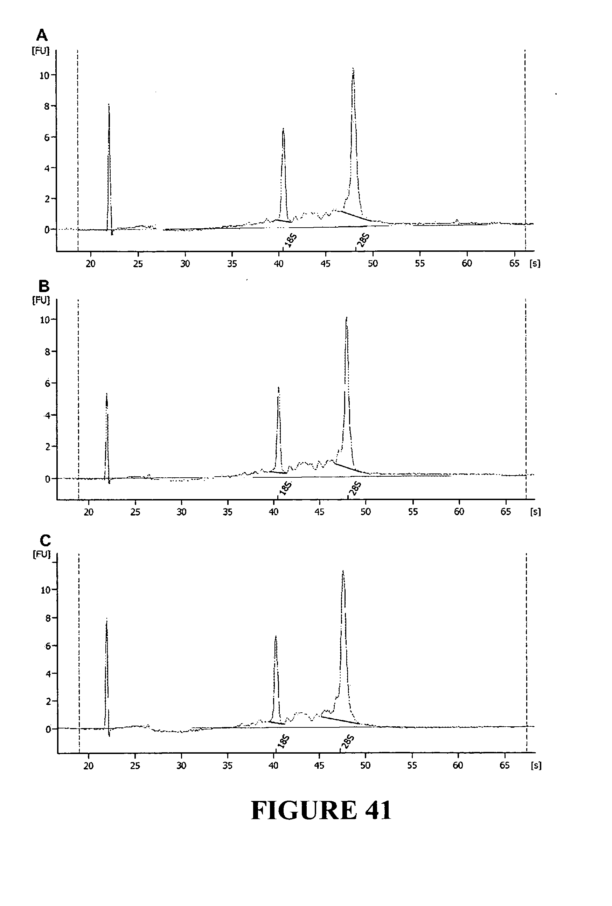

The addition of a step of washing microvesicles multiple times also dramatically improves the quality of nucleic acids extracted from urinary microvesicles. The washing step can effectively remove adverse factors that prevent high quality nucleic acid extraction from urinary microvesicles. Urine samples of 20 ml each are used for four groups of nucleic acid extraction tests following the above method with except some modifications. For group 1, isolated microvesicles were directly used for nucleic acid extraction without any intervening steps. For group 2, microvesicles were treated with RNase inhibitors before nucleic acid extraction. For group 3, microvesicles were washed without any RNase inhibitor treatment before nucleic acid extraction. For group 4, microvesicles were washed and treated with RNase inhibitors. As shown in **Figure 38**, for group 1 tests, the quality of the extracted nucleic acids was very poor with RIN of 3.63±2.3 and RNA concentration of 101.3 ± 27.6 pg/µl. For group 2 tests (**Figure 39**), the quality of the extracted nucleic acids was roughly similar to that in group 1 tests with RIN of 1.83 ± 2.2 and RNA concentration of 101.6 ± 88 pg/µl. For group 3 tests (**Figure 40**)**,** the quality of the extracted nucleic acids was improved dramatically with RIN of 9.2 ± 0.0 and RNA concentration of 347.7 ±97.7 pg/µl. For group 4 tests (**Figure 41**), the quality of the extracted nucleic acids was similar to that in group 3 with RIN of 7.43 ±0.2 and RNA concentration of 346.3 ±32.7 pg/µl. These data showed that without washing steps, the extraction quality from microvesicles were inconsistent, with a relatively higher variation than those extractions where microvesicles had been washed before nucleic acid extraction.

### Example 6: Use of RNase inhibitors for nucleic acid extraction from serum microvesicles

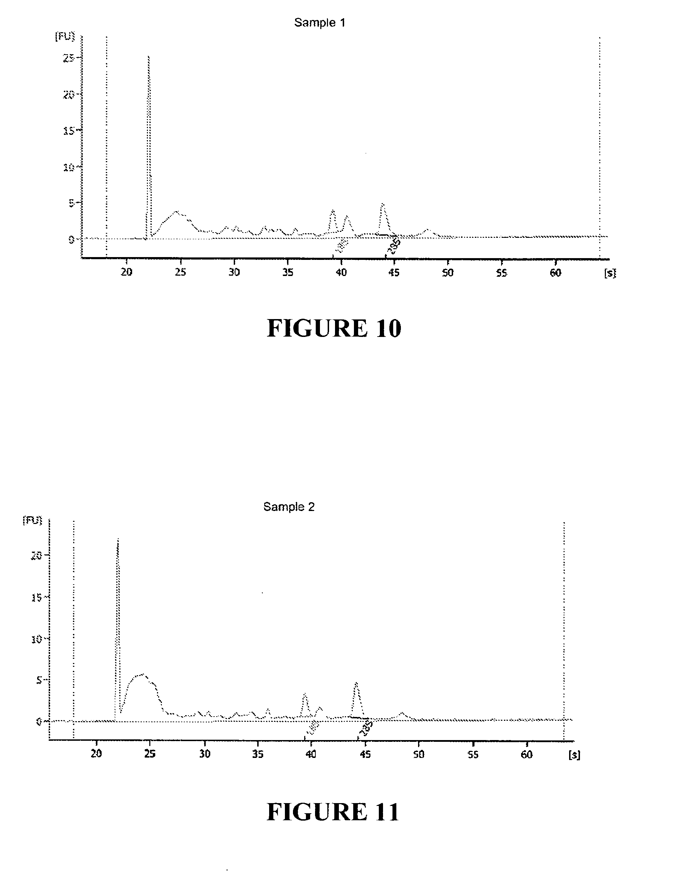

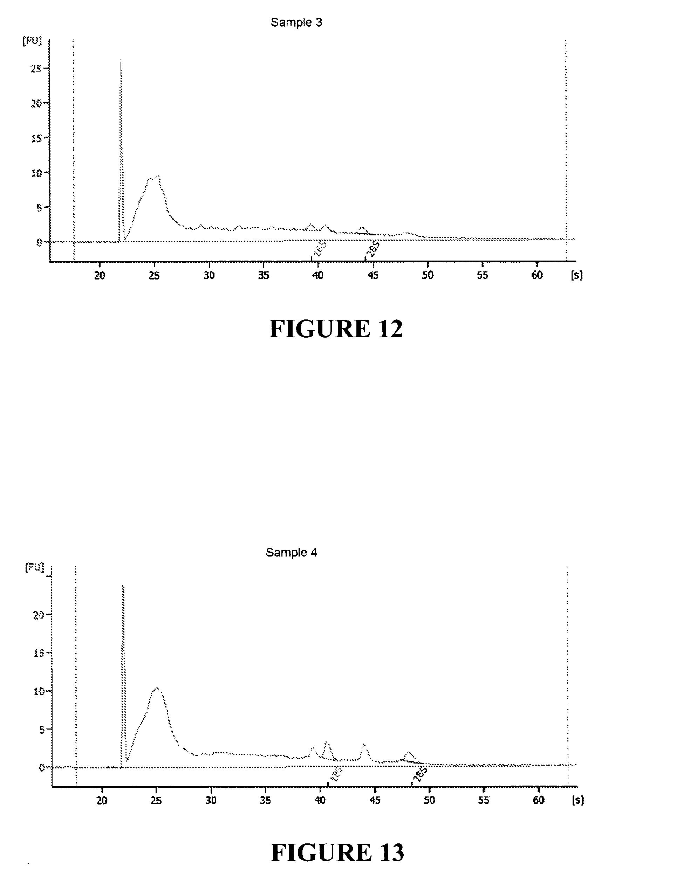

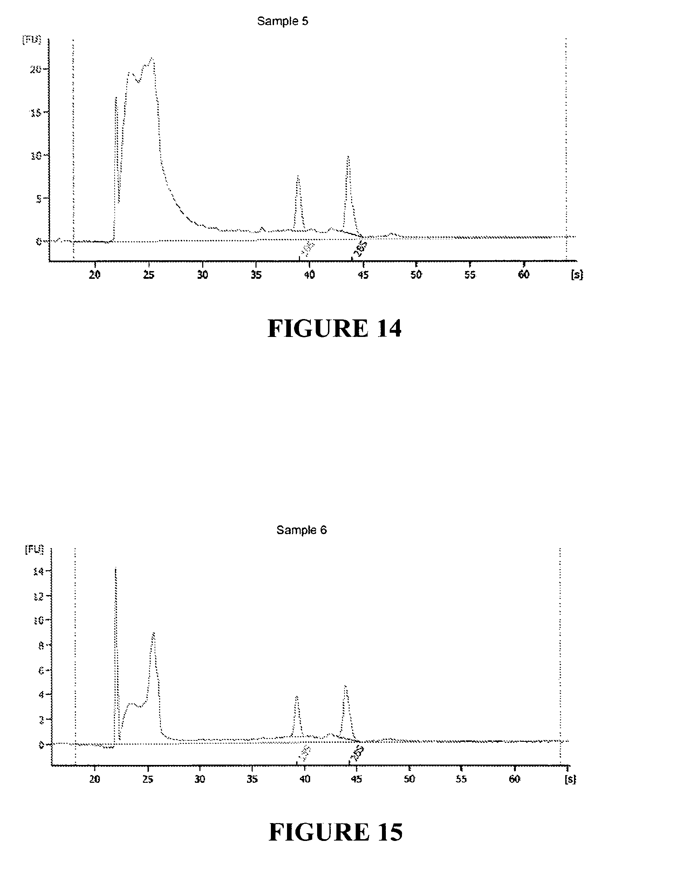

With the above improved method, high quality nucleic acid extractions can also be obtained from serum microvesicles. Here, we obtained serum from both melanoma and normal patient sera and used RNase inhibitor cocktail SUPERase-In™ (Ambion, Inc.) to treat microvesicle pellets by resuspension. In one batch of tests, we isolated microvesicles from four duplicates of 1 ml melanoma serum samples and treated the microvesicle pellets with 1.6 units SUPERase-In/µl at a final concentration. The microvesicle isolation method was ultracentrifugation and the microvesicle pellets were treated with DNase for 20 minutes at room temperature. As shown in **Figures 10-13**, the quality of RNA extraction from the four melanoma serum samples was low and inconsistent, with the RNA yield being 543 pg/µl, 607 pg/µl, 1084 pg/µl, 1090 pg/µl, and the RNA integrity assessed by the 28s/18s ratio being 1.7, 1.8, 1.3, and 0.6, respectively. In another batch of tests, we isolated microvesicles from two duplicates of 1 ml melanoma serum samples and treated the microvesicle pellets with 3.2 units SUPERase-In µ/l at a final concentration. As shown in **Figures 14 and 15**, the quality of RNA extraction from the two melanoma serum samples treated with 3.2 units SUPERase-In/µl was generally better than the quality of RNA extraction from those treated with 1.6 units SUPERase-In/µl. The RNA yield for the two melanoma serum samples is 3433pg/µl and 781pg/µl and the 28S/18S ratio is 1.4 and 1.5, respectively.

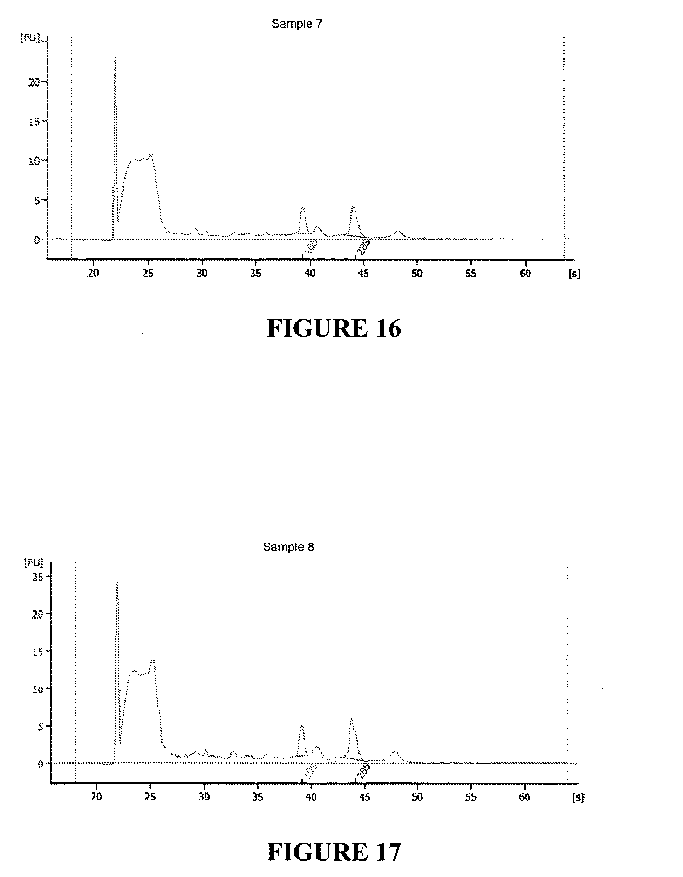

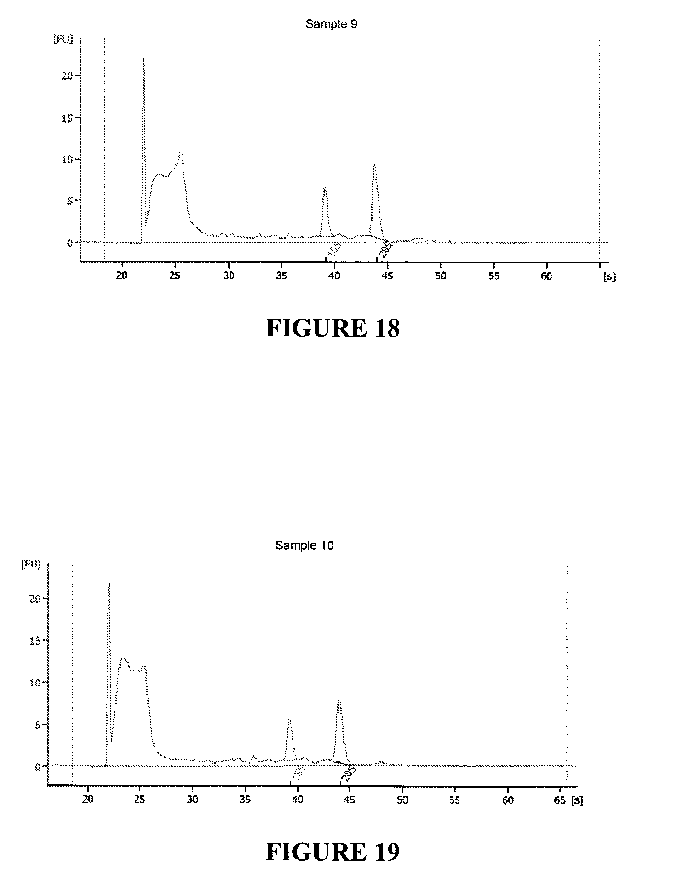

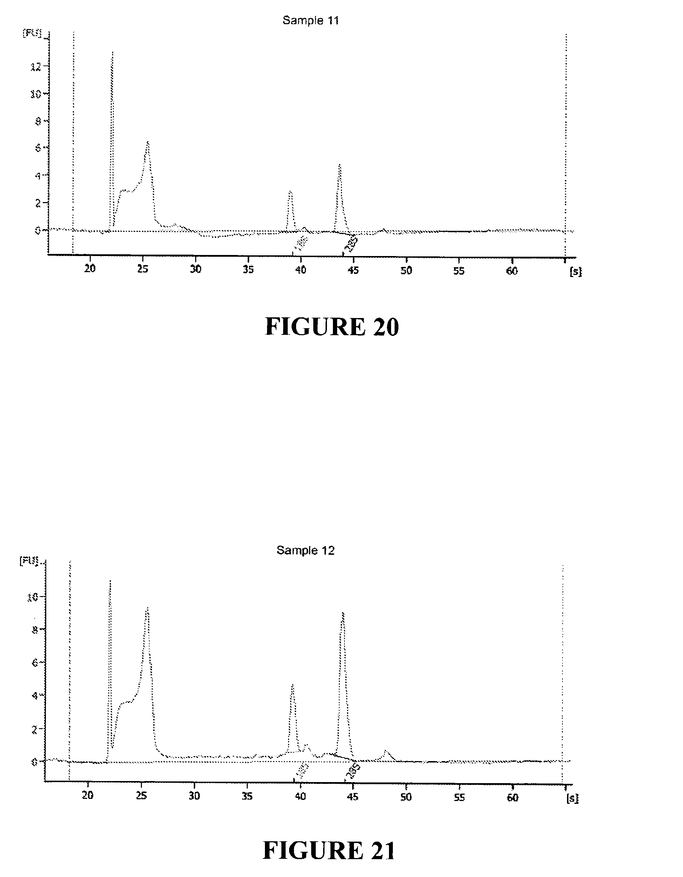

Further, we tested four duplicates of 1 ml normal serum samples at 1.6 units SUPERase-In/µl and 2 duplicates of 1 ml normal serum samples at 3.2 SUPERase-In/µl. As shown in **Figures 16-19**, the quality of RNA extraction at 1.6 units SUPERase-In/µl is low with the RNA yield being 995 pg/µl, 1257 pg/µl, 1027 pg/µl, and 1206 pg/µl, and the 28S/18S ratio being 1.3, 1.6, 1.6, 1.8, respectively. In contrast, as shown in **Figures 20 and 21**, the quality of RNA extraction at 3.2 units SUPERase-In/ul increases with the RNA yield being 579 pg/µl and 952 pg/µl, and the 28S/18S ratio being 1.6 and 2.3, respectively.

### Example 7: Use of extraction enhancement agents for nucleic acid extraction from a biological sample

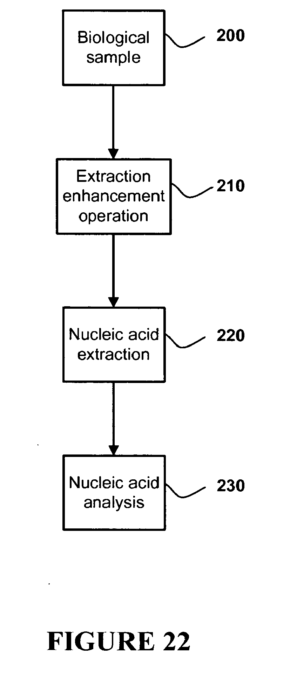

In Examples 3 and 4, our test results suggest that treatment with extraction enhancers can increase the quality of RNA extraction from microvesicles. It is expected that such extraction enhancers will have similar effects on other biological samples. As shown in **Figure 22**, a novel method of nucleic acid extraction requires a step of performing an extraction enhancement operation on the biological sample. Such method may be exemplified in the following conceived nucleic acid extraction experiment. A doctor prescribes a test of a tumor biomarker for a patient. A 5 ml blood is then drawn from the patient. The blood sample **200** is sometimes pre-processed to get the blood serum. An enhancement operation **210** is then performed, e.g., an appropriate amount of extraction enhancer is added to the serum and the mixture is incubated for 30 minutes at 37°C. Nucleic acids from the treated serum are then extracted using regular extraction methods such as detailed in Example 5 **220** and analyzed using Agilent BioAnalyzer **230**. Such extraction is expected to produce high quality nucleic acids from the biological sample.

### Nucleic Acids From Urinary Microvesicles As Biomarkers

### Example 8: Urinary microvesicles are contaminated by free, extra-microvesicle, non-cellular DNA

We separated a urine sample into two 25 ml duplicate samples and isolated microvesicles from the two sub-samples by differential centrifugation as detailed above. In one sub-sample, we treated the microvesicles with DNase and extracted nucleic acids from the treated microvesicles as detailed above. In another sub-sample, we did not treat the microvesicles with DNase and extracted nucleic acids from the untreated microvesicles. As shown in **Figure 23**, free, extra-microvesicle, non-cellular DNA contaminated isolated urinary microvesicles. When RNeasy micro kit was used for the nucleic acid extraction (**Figure 23** **A and B**), the result showed that more nucleic acids were seen in the untreated sample (**A**) than the treated sample (**B**) since the peak in (**A**) was generally higher than the peak in (**B**).

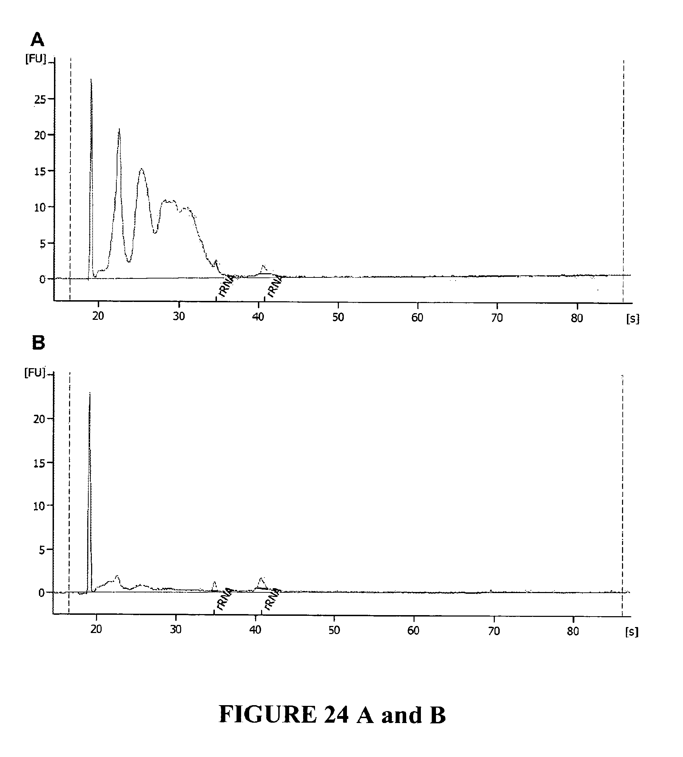

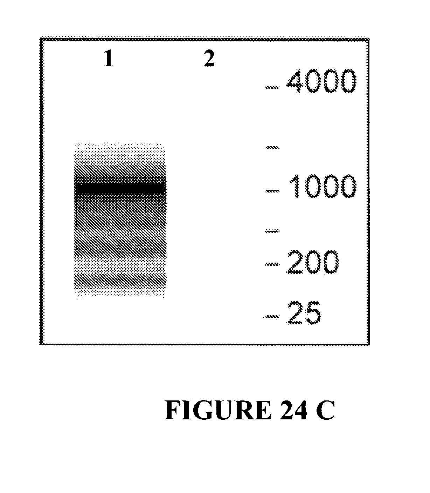

In another test, we performed a similar test except that serum samples were used instead of urine samples. As shown in **Figure 24**, free, extra-microvesicle, non-cellular DNA also contaminated the isolated serum microvesicles. More nucleic acids were seen in the DNase untreated sample (**A**) than the DNase treated sample (**B**) since the peak in (**A**) was generally higher than the peak in (**B**). Similarly, when MirVana kit was used for the nucleic acid extraction, as shown in Figure 23 (**C**) and (**D**), the result also showed that more nucleic acids were seen in the untreated sample (**C**) than the treated sample (**D**) since the peak in (**C**) was generally higher than the peak in (**D**). The extra nucleic acids from the untreated sample were likely from DNase susceptible "apoptotic bodies" because DNase susceptible "apoptotic bodies"-like ladders were seen as shown in the pseudo gel in **Figure 24** **C**. In both the urine and the serum samples, the amount of the free, extra-microvesicle, non-cellular DNA varied between subjects but the size of this DNA was in the range of approximately 25 to 1500 base pairs.

### Example 9: Urinary microvesicles are mostly not contaminated by free, extra-microvesicle, non-cellular RNA

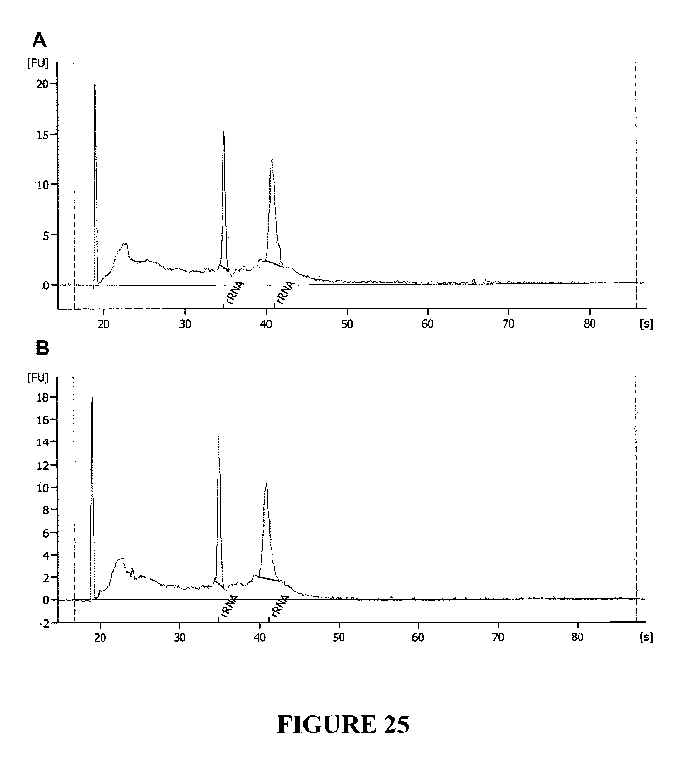

We separated a urine sample into two 25 ml duplicate samples and isolated microvesicles from the two sub-samples by differential centrifugation as detailed above. In one sub-sample, we treated the microvesicles with RNase and extracted nucleic acids from the treated microvesicles as detailed above. In another sub-sample, we did not treat the microvesicles with RNase and extracted nucleic acids from the untreated microvesicles. As shown in **Figure 25**, almost no free, extra-microvesicle, non-cellular RNA contaminated the isolated microvesicles. The curve of the RNase untreated sample (**A**) mostly overlapped with the curve of the RNase treated sample (**B**), suggesting there is no free extra-microvesicle, non-cellular RNA associated with the isolated microvesicles. This may be due to the presence of ribonucleases in urine.

### Example 10: Nucleic acid profiles are similar in urinary microvesicles and renal cells measured by Agilent BioAnalyzer.

We extracted nucleic acids from both urinary microvesicles and renal (kidney) tissues and compared their profiles. The method of extraction from urinary microvesicles was as detailed in Example 5. The rat kidney samples were processed via the RNeasy Mini kit and the RNeasy Plus kit. To determine the amount of small RNAs in the rat kidney sample, they were also processed by both kits using the miRNA isolation method according to the manufacturer's instructions.

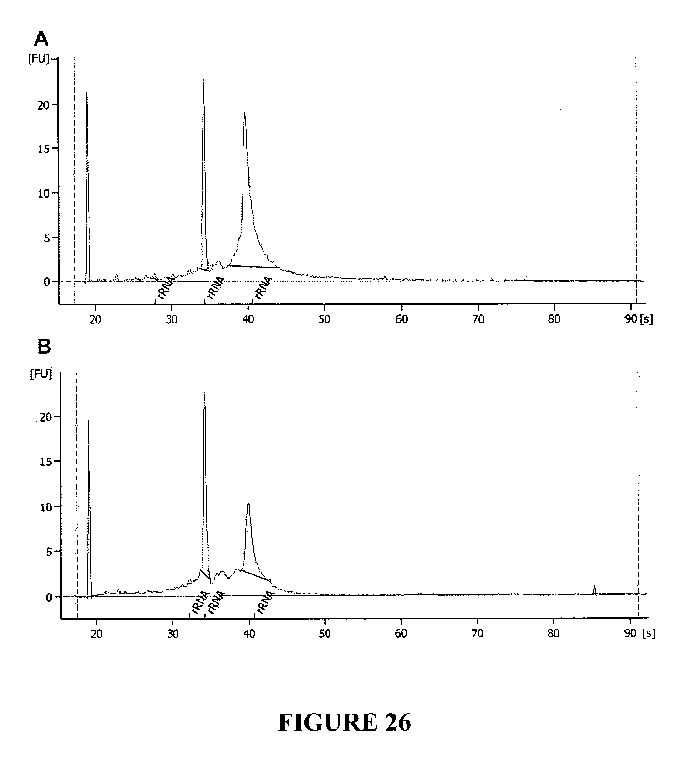

As shown in **Figure 26****,** their profiles (**A-**kidney, **B**-microvesicle) were very similar including the presence and integrity of the 18S and 28S rRNA peaks. Such 18S and 28S rRNA peaks had not been seen in previously reported serum-derived or cell culture media-derived microvesicles.

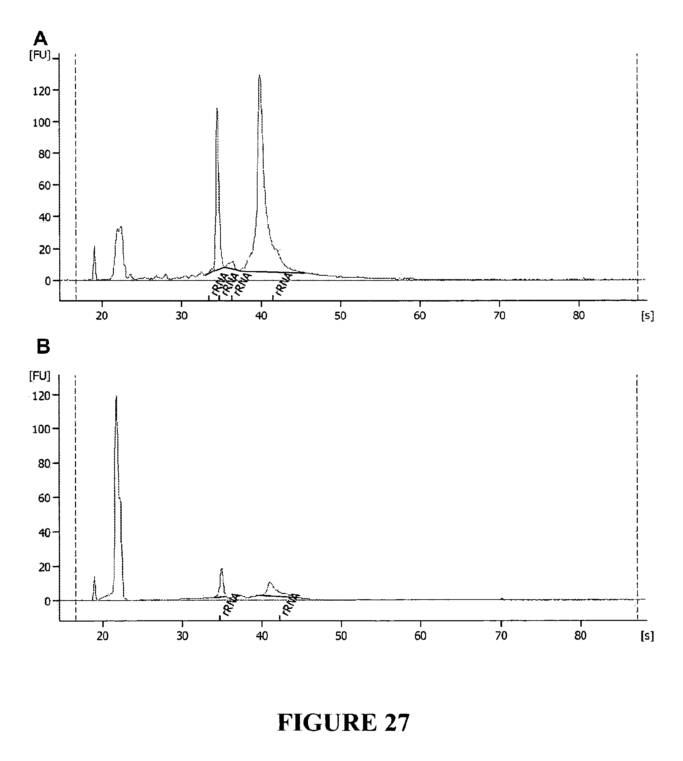

In addition to the similarities in rRNA peaks, urinary microvesicles also contained similar small RNA profiles to those obtained from renal cells. As shown in **Figure** 27, both urinary microvesicles (**B**) and renal tissue (**A**) contained small RNAs (about 25-200 base pairs) and shared similar patterns.

These data suggest that using the novel nucleic acid extraction method disclosed herein, the profiles in urinary microvesicles may be used to examine the profiles in the renal cells from which the microvesicles originated.

### Example 11: RNA profiles in urinary microvesicles are different from those from whole urine.

We discovered that RNA profiles in urinary microvesicles are different from those from whole urine. We used 75 ml duplicate urine samples for the tests. RNA was isolated from urinary microvesicles by first pre-processing the urine by 300g for 10 min at 4°C, centrifugation of the supernatant at 17,000g for 20 min at 4°C, and filtration of the supernatant through a 0.8 µm filter (cellulose nitrate membrane filter unit, Nalgene, NY), followed by the steps as detailed in Example 5. RNA from whole urine was isolated using the ZR urine RNA isolation kit (Zymo Research, CA) according to the manufacturer's instructions. To remove DNA from the Zymo processed sample, the eluted RNA was resuspended in 350 µl RLT buffer and processed via the RNeasy Plus Micro kit, which used DNase to eliminate associated DNA, and eluted in 16 µl nuclease free water.

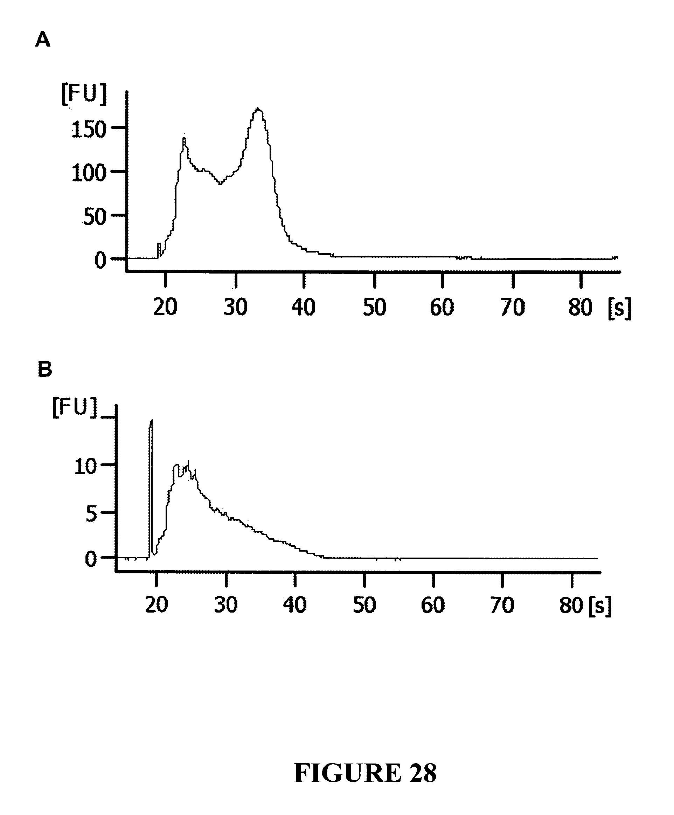

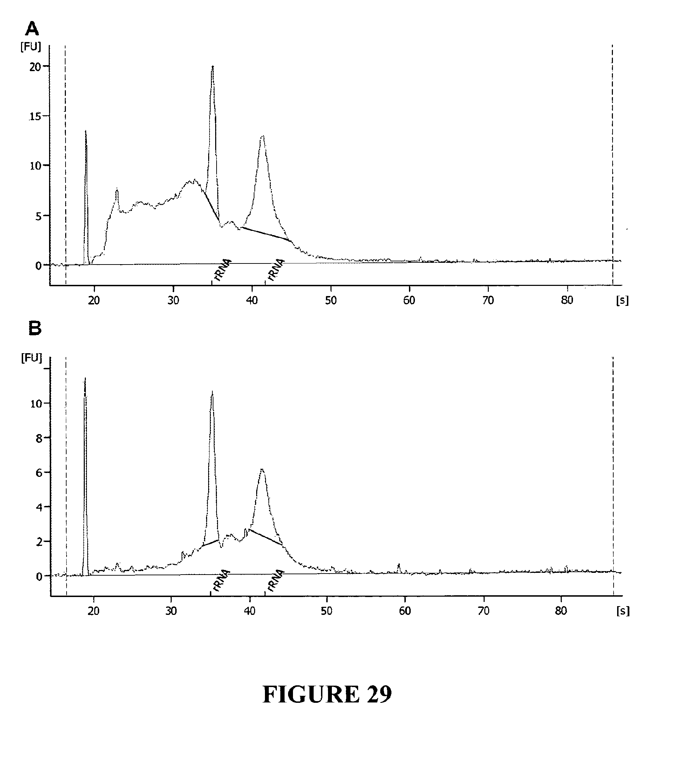

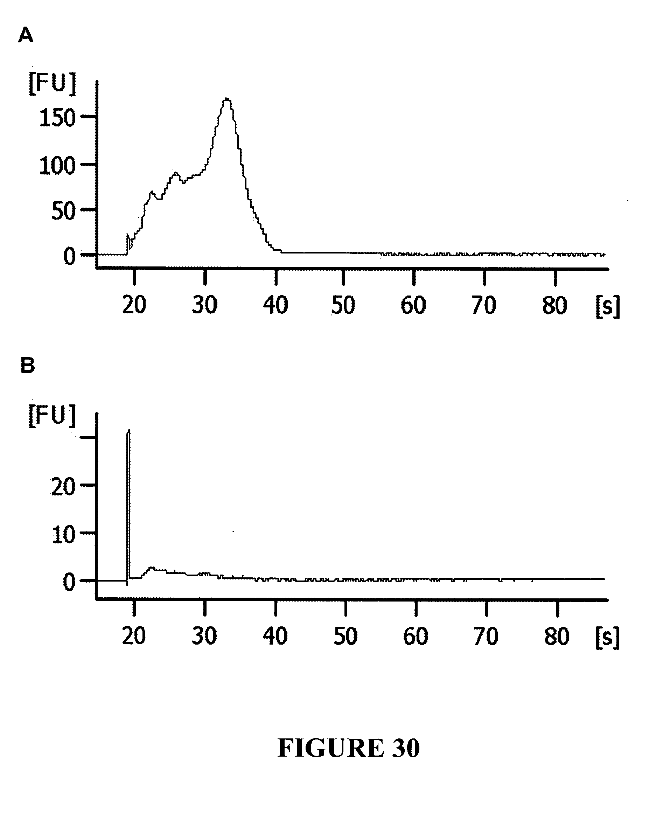

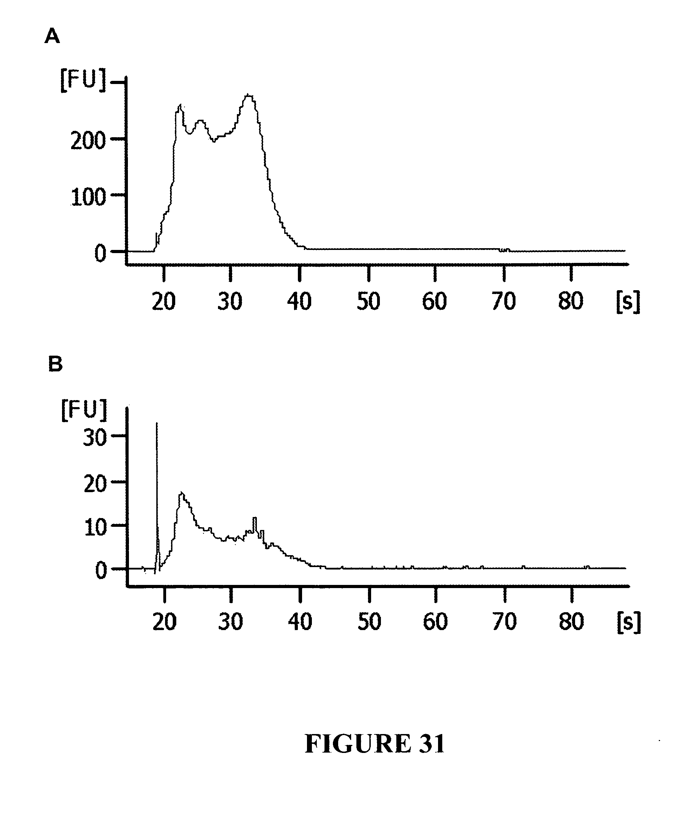

As shown in **Figure 28**, a large amount of nucleic acid could be isolated using the ZR urine RNA isolation kit when no DNase was used (**A**). However, the profile appeared broad and lacked 18S and 28S rRNA peaks. Further, the profile changed significantly (**B**) when DNase was used, suggesting most of the extract was DNA in nature. In contrast, as shown in Figure 29, the RNA profile from microvesicles from the same urine sample was generally very different from the profile from the whole urine extraction. In the microvesicle profile, there were 18S and 28S peaks. In addition, RNA from microvesicles was more abundant than that from the whole urine. DNase digestion of the extractions from microvesicles did not affect the rRNA peak significantly when we compared the profile without DNase treatment (**A**) to the profile with DNase treatment (**B**). The RNA profiles from the 300g pellets (**Figure 30**) and the 17,000g pellets (**Figure 31**) were similar to that from the whole urine extraction. In both of these profiles, the peaks decreased substantially after DNase treatment when we compared the profile without DNase treatment (**A**) to the profile with DNase treatment (**B**). These data suggested that DNA was the predominant species in the extraction, and no 18S and 28S rRNA peaks were detectable. Therefore, together with the data in Example 10, RNA profiles from urinary microvesicles is more similar to renal cell profiles than to profiles from whole urine. Further, the integrity of RNA extraction from microvesicles was at least 10 times better than that from whole urine.

### Example 12: Urinary microvesicles contain both RNA and DNA.

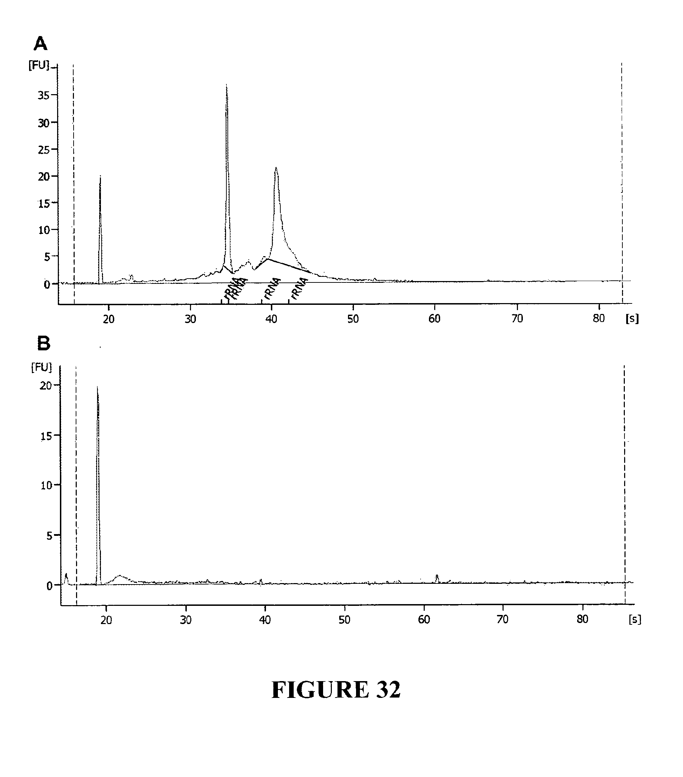

We determined whether urinary microvesicles contained RNA, DNA, or both by treating the pellets first with both RNase and DNase to remove free, extra-microvesicle, non-cellular contaminations followed by RNase and/or DNase digestion of intra-microvesicle nucleic acids during column based nucleic acid isolation. RNase digestion (**B**) almost completely abolished the nucleic acid profile (**Figure 32**) compared to that without RNase digestion (**A**). These data suggest that RNA represents the most abundant nucleic acid within microvesicles. As shown in **Figure 33**, after on-column digestion of the RNase treated samples with DNase (**B**), the peak just after 20s is reduced after further DNase digestion inside in comparison with the peak before further DNase digestion (**A**). This reduction demonstrated that a small amount of DNase-digestible material was present within microvesicles, probably DNA.

### Example 13: Urinary microvesicles contain mRNA transcripts encoding specific genes from various regions of the nephron and collecting duct.

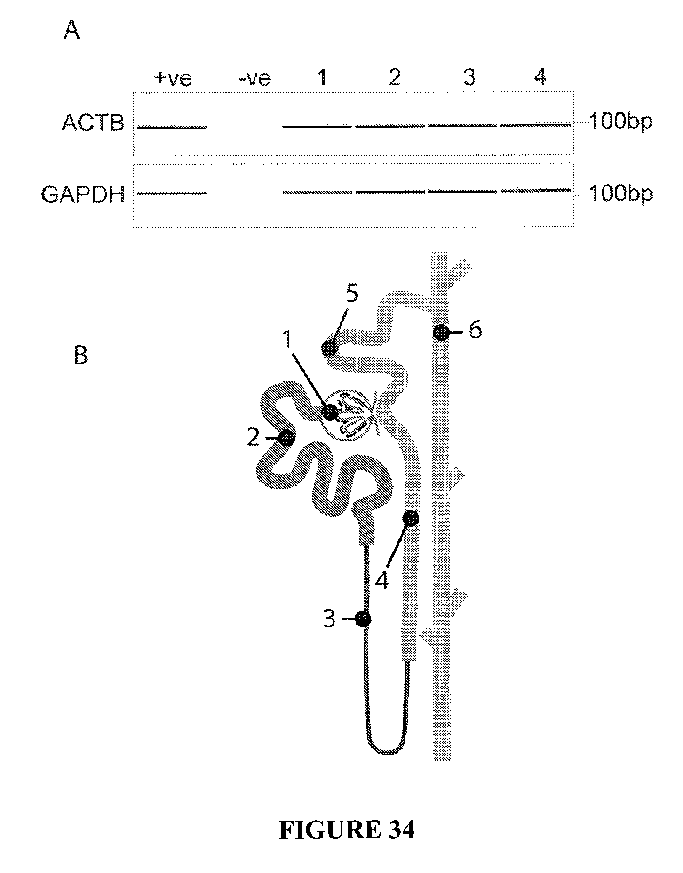

As shown in Example 10, nucleic acid profiles are similar in urinary microvesicles and renal cells measured by Agilent BioAnalyzer. Here we further show that microvesicles contain mRNA transcripts encoding specific genes from various regions of the nephron and collecting duct. Urinary microvesicles were isolated from 200 ml urine from four human subjects (23 to 32 years of age) and were digested with RNase and DNase prior to exosome lysis and RNA extraction as detailed in Example 5. The extracted RNA underwent two rounds of mRNA amplification using RiboAmp (Molecular Devices, CA). For the riboamplification for the first round of the *in vitro* transcription step samples were incubated at 42°C for 4 hours and for the second *in vitro* transcription step samples were incubated at 42°C for 6 hours. Amplified RNA was denatured for 5 minutes at 65 °C and subjected to first strand cDNA synthesis as described in the Qiagen Omniscript protocol (Qiagen, CA). Both GAPDH and beta-actin genes were identified in all samples (**Figure 34A**)**.** We examined 15 transcripts characteristic of various regions of the nephron and collecting duct (**Figure 34B**). These included proteins and receptors implicated in various renal diseases including podocin from the glomerulus, cubilin from the proximal tubule and aquaporin 2 from the collecting duct.

For human samples, the PCR primers used were: ACTB UTR, forward 5'-GAAGTCCCTTGCCATCCTAA-3', reverse '5-GCTATCACCTCCCCTGTGTG-3'; GAPDH EX, forward 5'-ACACCCACTCCTCCACCTTT-3', reverse 5'-TGCTGTAGCCAAATTCGTTG-3'; NPHS2 UTR, forward 5'-AACTTGGTTCAGATGTCCCTTT-3', reverse 5'-CAATGATAGGTGCTTGTAGGAAG-3'; LGALS1 EX, forward 5'-GGAAGTGTTGCAGAGGTGTG-3', reverse 5'-TTGATGGCCTCCAGGTTG-3'; HSPG2 UTR, 5'-AAGGCAGGACTCACGACTGA-3', reverse 5'-ATGGCACTTGAGCTGGATCT-3'; CUBN EX, forward 5'-CAGCTCTCCATCCTCTGGAC-3', reverse 5'-CCGTGCATAATCAGCATGAA-3'; LRP2 EX, forward 5'-CAAAATGGAATCTCTTCAAACG-3', reverse 5'-GTCGCAGCAACACTTTCCTT-3'; AQP1 UTR, forward 5'-TTACGCAGGTATTTAGAAGCAGAG-3', reverse 5'-AGGGAATGGAGAAGAGAGTGTG-3'; CA4 UTR, forward 5'-ATGATGGCTCACTTCTGCAC-3', reverse 5'-TCATGCCTAAAGTCCCACCT-3'; CLCN5 EX, forward 5'-GTGCCTGGTTACACACAACG-3', reverse 5'-AGGATCTTGGTTCGCCATCT-3'; BDKRB1 UTR, forward 5'-GTGGTTGCCTTCCTGGTCT-3', reverse 5'-ATGAAGTCCTCCCAAAAGCA-3'; CALCR UTR, forward 5'-ATTTTGCCACTGCCTTTCAG-3', reverse 5'-ATTTTCTCTGGGTGCGCTAA-3'; SCNN1D UTR, forward 5'-GCGGTGATGTACCCATGCT-3', reverse 5'-CTGAGGTGGCTAGGCTTGA-3'; SLC12A3 EX, forward 5'-AGAACAGAGTCAAGTCCCTTCG-3', reverse 5'-TATGGGCAAAGTGATGACGA-3'; AQP2 UTR, forward 5'-GCAGTTCCTGGCATCTCTTG-3', reverse 5'-GCCTTTGTCCTTCCCTAACC-3'; ATP6V1B1 EX, forward 5'-AGGCAGTAGTTGGGGAGGAG-3', reverse 5'-CGAGCGGTTCTCGTAGGG-3'; SLC12A1 EX, forward 5'-CAGATGCAGAACTGGAAGCA-3', reverse 5'-GGAAGGCTCAGGACAATGAG-3'. "UTR" refers to primers designed in the UTR and "EX" refers primers designed across exons. The PCR protocol was 5 min at 94°C; 40 s at 94°C; 30 s at 55°C; 1 min at 65°C for 30 cycles; and 68°C for 4 min. For mouse samples the primers used were: AQP2: forward 5'-GCCACCTCCTTGGGATCTATT-3', reverse 5'-TCATCAAACTTGCCAGTGACAAC-3'; V-ATPase B1 subunit: forward 5'-CTGGCACTGACCACGGCTGAG -3', reverse 5'-CCAGCCTGTGACTGAGCCCTG -3'. The PCR protocol was 5 min at 94°C; 40 s at 94°C, 30 s at 55°C, 1 min at 65°C for 30 cycles; and 68°C for 4 min.

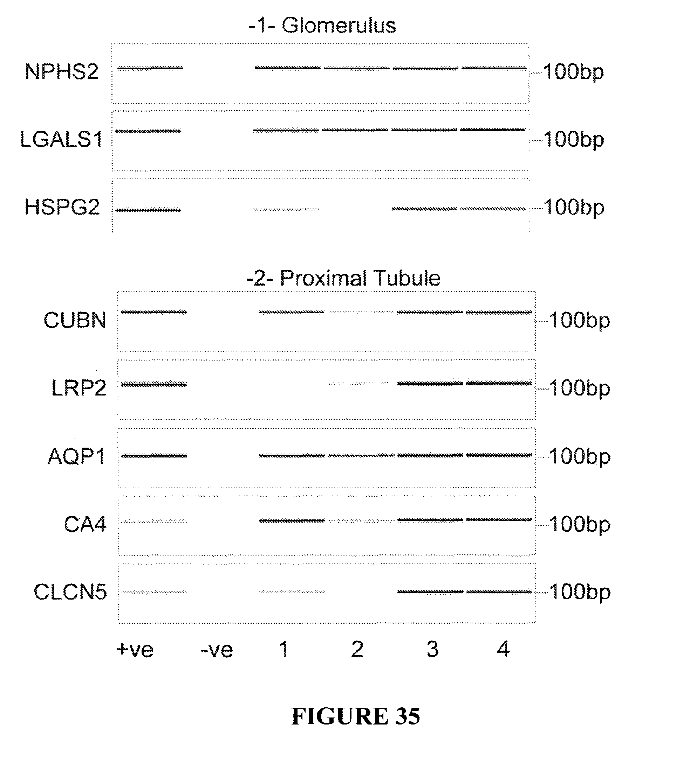

As shown in **Figure 34****, Panel A**, RiboAmp amplified mRNA transcripts for beta-actin and GAPDH were readily detectable in the BioAnalyzer generated pseudo gel in urinary microvesicles from the four human subjects. For clarification purpose, the six regions of the nephron and collecting duct are shown in **Figure 34****, Panel B.** As a result of RT-PCR analysis of RiboAmped mRNA from urinary exosomes, the following transcripts in the six regions are also readily detectable: region 1 Glomerulus: NPHS2 - podocin, LGALS1 - Galectin-1 , and HSPG2 - heparan sulfate proteoglycan (**Figure 35**);b region 2 Proximal Tubule: CUBN - cubilin, LRP2 - megalin, AQP1 - aquaporin 1, CA4 - carbonic anhydrase 4, and CLCN5 - chloride channel protein 5 (**Figure 35**); region 3 Thin Descending Limb: BDKRB1 - bradykinin B1 receptor (**Figure 36**); region 4 Medullary Thick Ascending Limb: CALCR - calcitonin receptor, and SCNN1D - amiloride-sensitive sodium channel subunit delta (**Figure 36**); region 5 Distal Convoluted Tubule: SLC12A3 - thiazide-sensitive sodium-chloride cotransporter (**Figure 36**); region 6 Collecting Ducts: AQP2 - aquaporin 2, ATP6V1B1 - vATPase B1 subunit, and SLC12A1 - Kidney-specific Na-K-Cl symporter (**Figure 36**).

Therefore, mRNA transcripts from all renal regions examined could be identified, suggesting that microvesicles containing mRNA are released from all regions of the nephron and collecting duct, and microvesicles can be a novel non-invasive source of nucleic acid biomarkers for renal diseases.

### Example 14: Some mRNA transcripts inside urinary microvesicles are specific to renal cells

If nucleic acids in microvesicles are used to non-invasively examine renal genes in diseases, the transcripts in microvesicles should be specific to renal cells. Here, we show that mRNA transcripts are specific to renal cells. We used knockout mice in which the V-ATPase B1 subunit is absent. The absence of V-ATPase B1 subunit leads to renal acidosis in the mice (Finberg KE, Wagner CA, Bailey MA, et al., The B1-subunit of the H(+) ATPase is required for maximal urinary acidification. Proc Natl Acad Sci USA 102:13616-13621, 2005*).*

All animal experiments were carried out in accordance with approved animal ethics guidelines at the Massachusetts General Hospital. V-ATPase B1 subunit knockout animals have been described (Finberg KE, Wagner CA, Bailey MA, et al. The B1-subunit of the H(+) ATPase is required for maximal urinary acidification. Proc Natl Acad Sci USA 102:13616-13621, 2005*).* For urine collection, animals were caged in metabolic cages in groups of two (n = 4 animals per group) over a period of 72 hours (sufficient RNA can also be obtained by caging one animal per cage, and urine was collected for microvesicle isolation and analysis as described above for human urine. For kidney extraction, animals were anesthetized using pentobarbital sodium (Nembutal) (Abbott Laboratories, IL) (65 mg/kg body weight i.p.), and kidneys immediately removed and frozen in liquid nitrogen. Using a pestle and mortar in a liquid nitrogen bath the frozen kidney was ground up, resuspended in RNAlater (Qiagen, CA) and stored in 1 ml aliquots at -80°C. For RNA extraction, an aliquot was thawed on ice and 50 µl lysed in 350 µl RLT buffer (with 10 µl beta- mercaptoethanol per ml RLT). Mouse kidney samples were processed via the RNeasy Mini kit (Qiagen, CA) with the inclusion of the DNA digestion step.

For real time PCR analysis, RNA extracted from mouse urinary microvesicles was denatured for 5 minutes at 65 °C and subjected to first strand cDNA synthesis as described in the Qiagen Sensiscript protocol (Qiagen, MD). For the Sensiscript reverse transcription oligo-dT primers were used at a final concentration of 1µM (Applied Biosystems, CA). The resulting cDNA was used in the TaqMan PreAmp Master Mix Kit according to manufactures guide using 14 preamplification cycles (Applied Biosystems, CA). The preamplification product was then diluted 1:20 with 1X TE buffer (Promega, WI). The resulting cDNA was then used as a template for real-time PCR according to Taqman Preamplification guide (Applied Biosystems, CA). Mouse kidney RNA concentration was measured on a SmartSpec 3000 (Bio-Rad, CA) and all samples were diluted to 90 ng/µl. Mouse kidney RNA was denatured for 5 minutes at 65 °C and subjected to first strand cDNA synthesis as described in the Qiagen Omniscript protocol (Qiagen, MD). In the Omniscript reverse transcription oligo-dT primers were used at a final concentration of 1µM (Applied Biosystems, CA) and 1 µl of the resulting cDNA was then used per well in the subsequent real-time PCR reaction. The real-time PCR reaction was carried out using TaqMan Gene Expression Master Mix and Expression Assays (Mouse GAPD Part Number 4352339E and mouse Atp6v1b2 assay id Mm00431996_mH) on an ABI 7300 Real Time PCR System (Applied Biosystems, CA).

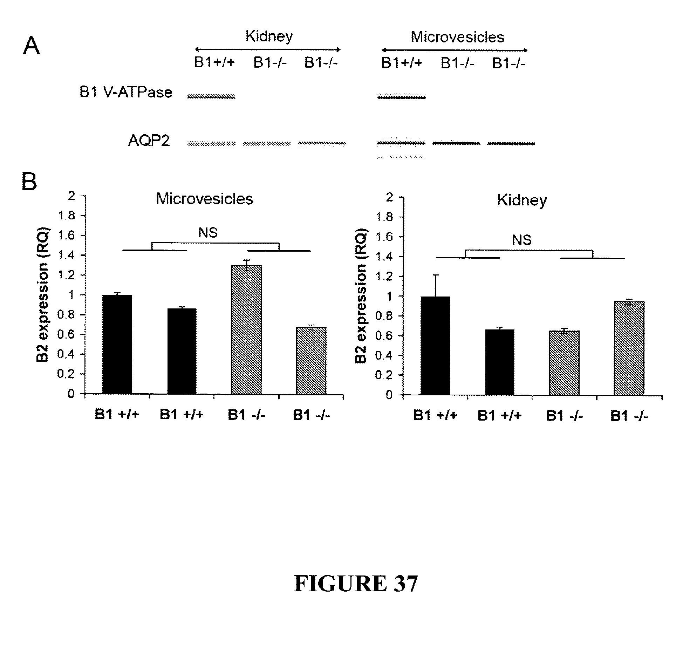

We extracted RNA from kidney tissues as well as from urinary microvesicles from the knockout mice. We examined the expression of the V-ATPase B1 subunit and aquaporin 2 (AQP2) mRNA using RT-PCR. As shown **Figure 37**, **Panel A**, V-ATPase B1 subunit transcript was not detected in both the kidney and urinary microvesicle samples from double mutant mice (B1-/-), which is consistent with the fact that V-ATPase B1 subunit gene was knocked out in these mice. In contrast, V-ATPase B1 subunit transcript was present in the kidney and microvesicle samples from the wild-type mice (B1/+/+). AQP2 mRNA was readily detected in both the kidney and the microvesicle sample from mice with both the B1 knockout or wild-type mice, which is expected because the V-ATPase B1 subunit deletion does not affect the expression of AQP2. Further, as shown in **Figure 37****, Panel B**, the expression level of V-ATPase B2 subunit in microvesicles from the B1 knockout was not statistically different from the level in microvesicles from the wild-type mice. Such was also true for the expression level of V-ATPase B2 subunit in kidney cell from the knockout mice in comparison with the level in kidney cells from the wild-type mice. Therefore, transcripts present in kidney cells can be detected in urinary microvesicles secreted by the kidney cells, and transcripts absent in kidney cells can not be detected in urinary microvesicles secreted by the kidney cell. Accordingly, transcripts in urinary microvesicles are specific to renal cells and are noninvasive biomarkers for renal cells transcripts.

### Example 15: Urinary microvesicles contain non-coding RNA transcripts

Urinary microvesicles were isolated and nucleic acids were extracted according to the above method detailed in Example 5. We performed a deep-sequencing of urinary microvesicle RNAs and found that there were random areas on certain chromosomes that exhibited extreme transcription. When plotted transcript number versus position on the chromosome, these transcripts appeared as "Spikes." These transcripts were more abundantly expressed than well-known endogenous markers such as GAPDH or actin, and were generally in non-coding regions of the chromosome. The relatively high expression levels of these spike sequences suggest that these sequences may also serve important roles in chromosome activation and cellular regulation.

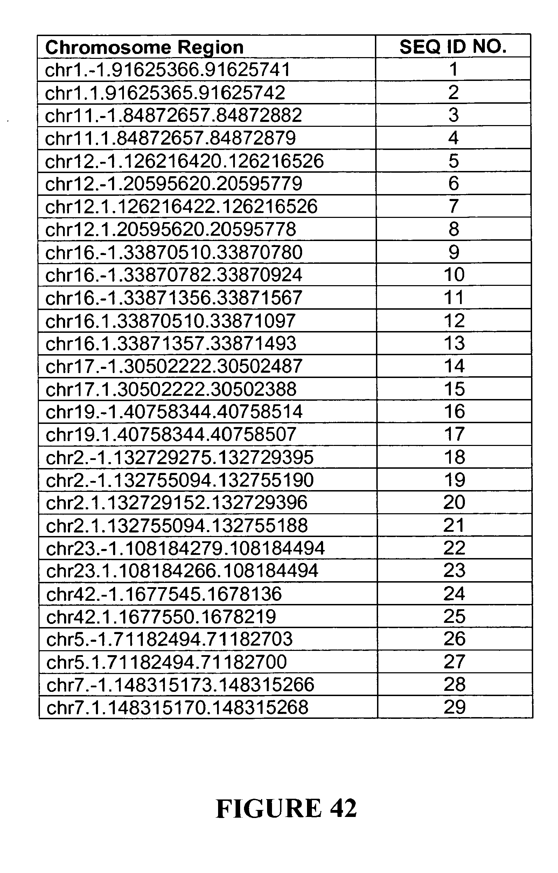

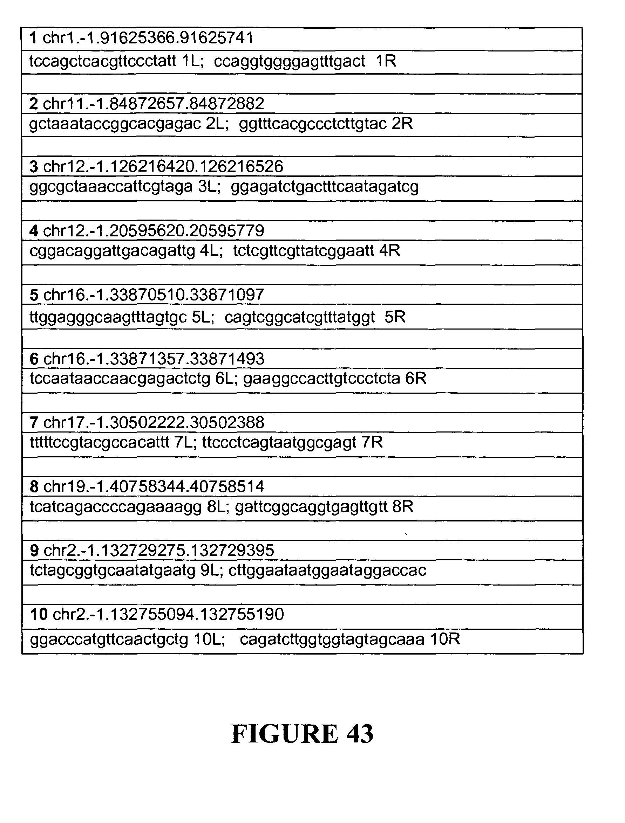

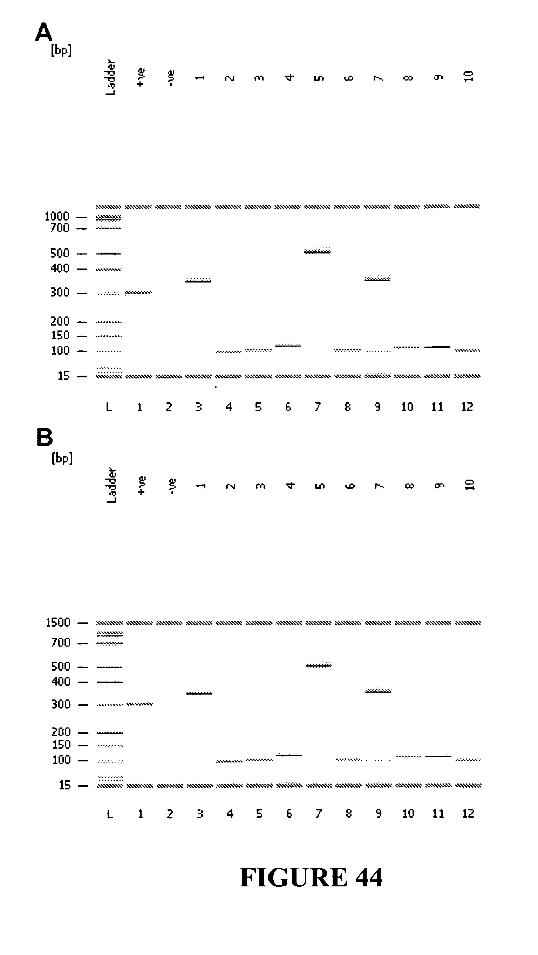

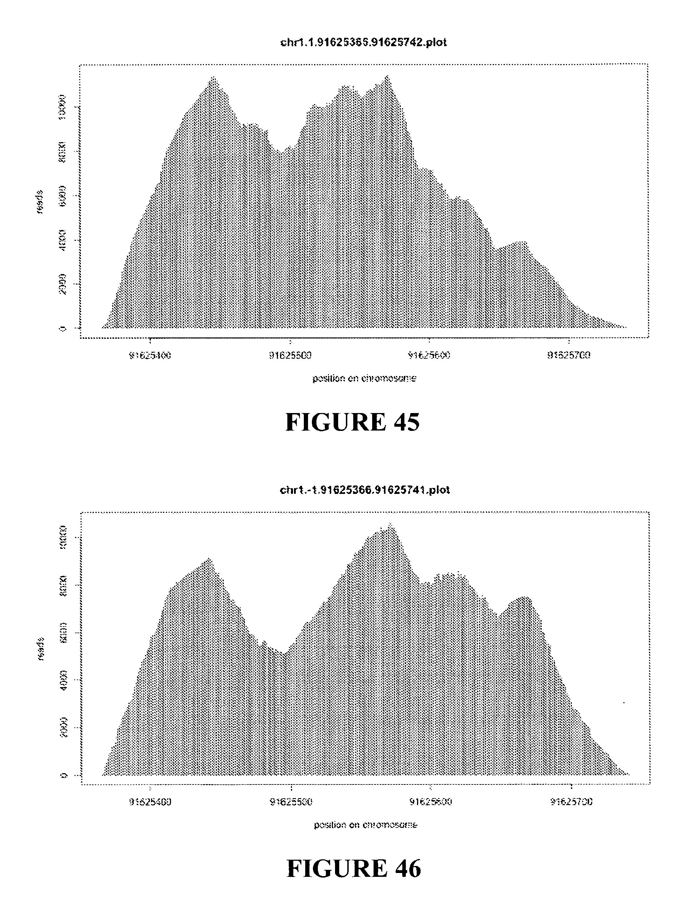

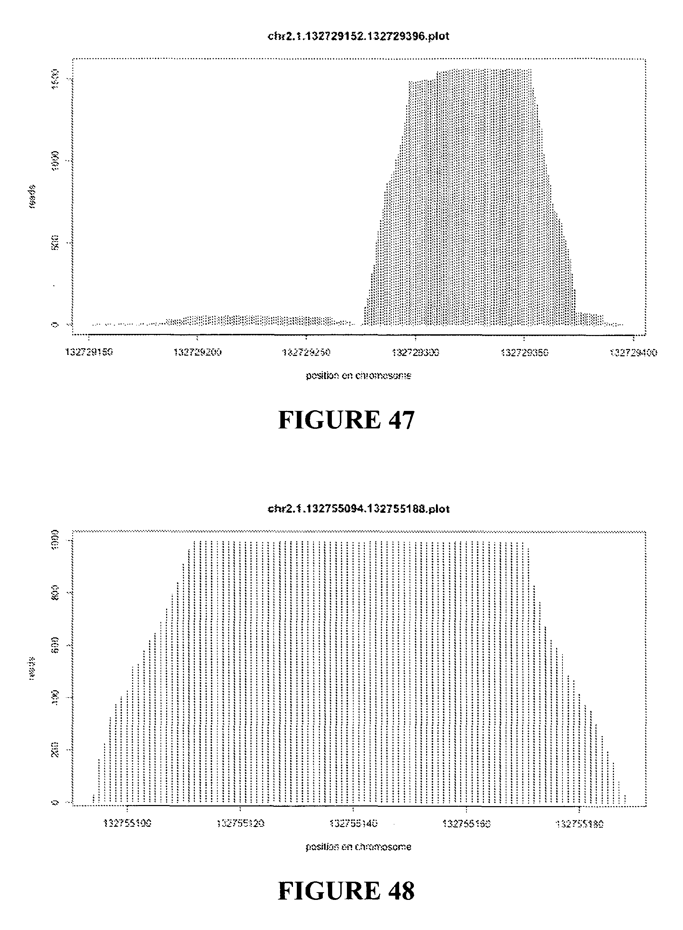

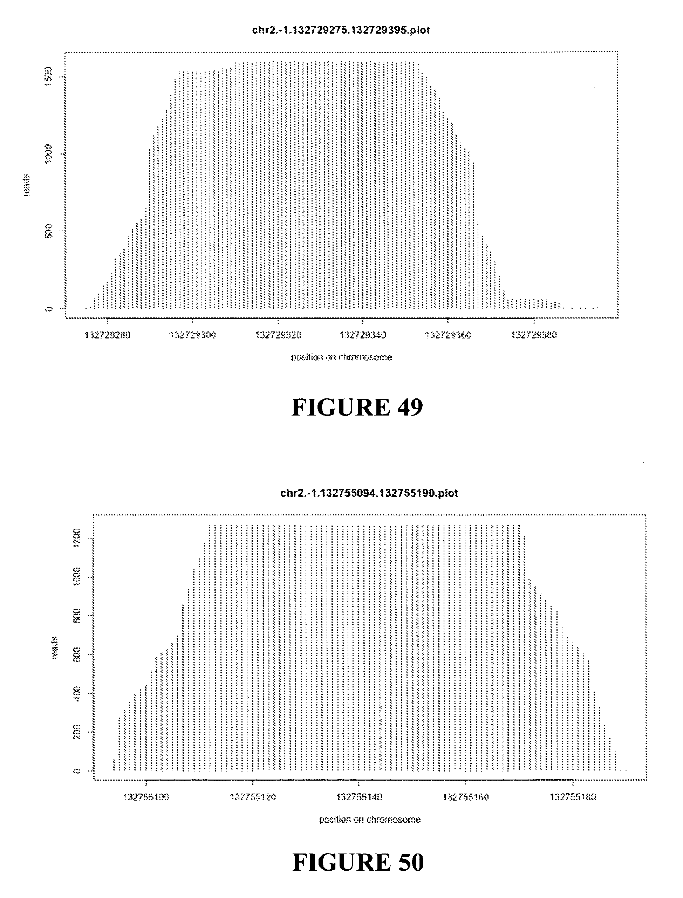

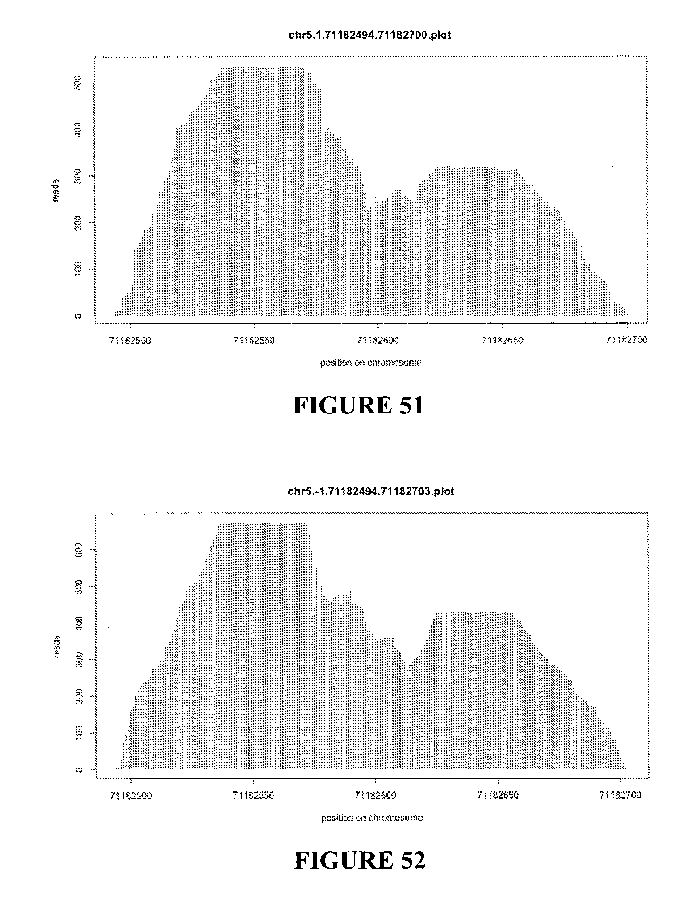

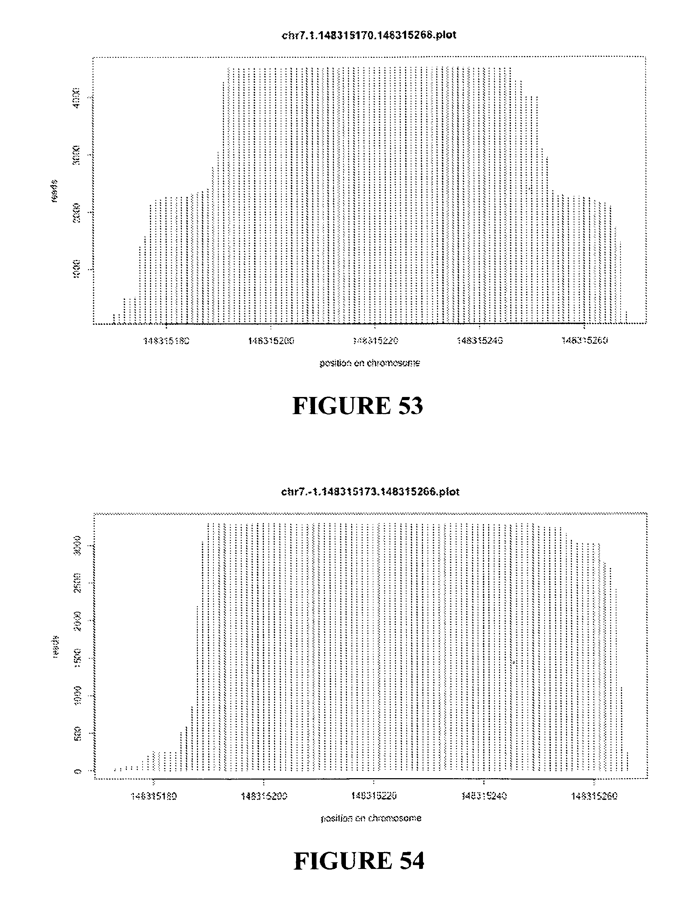

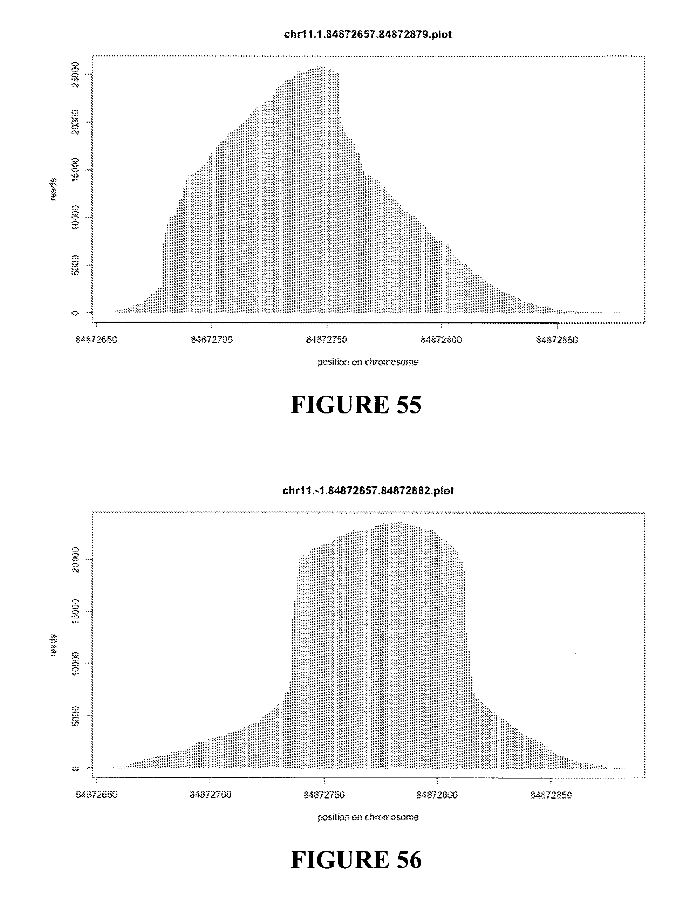

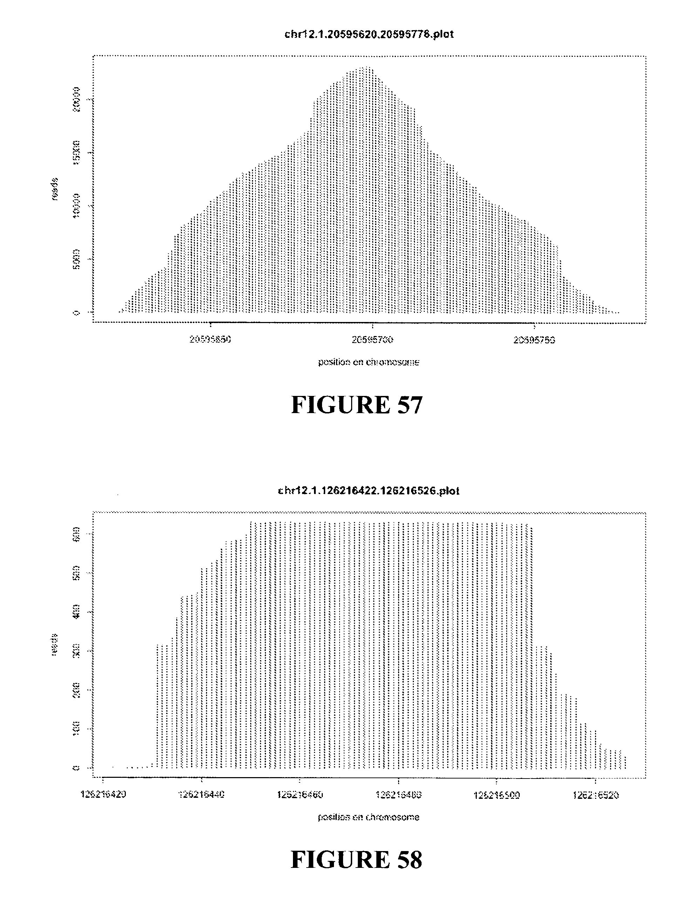

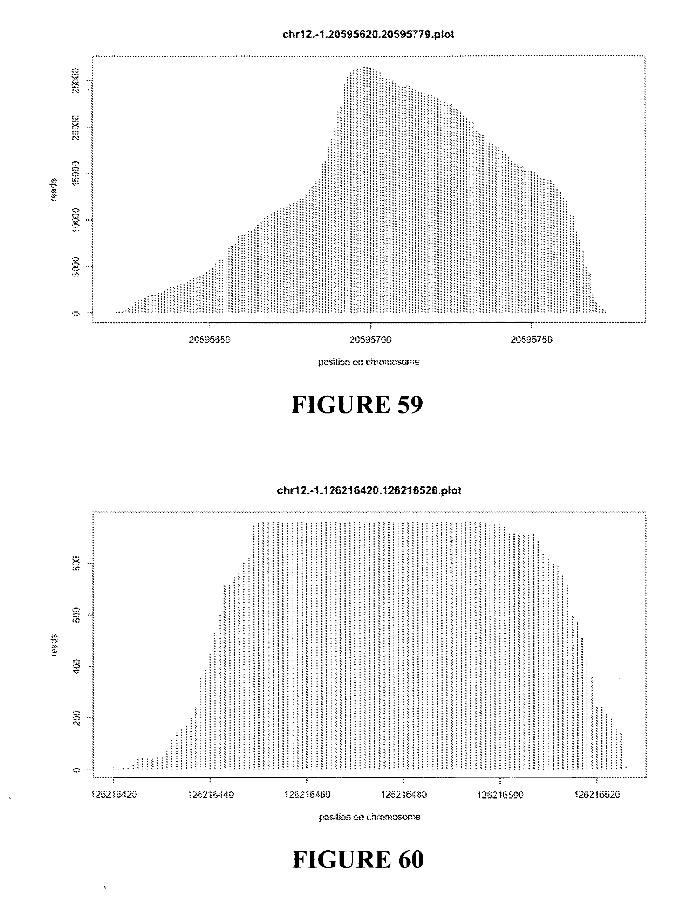

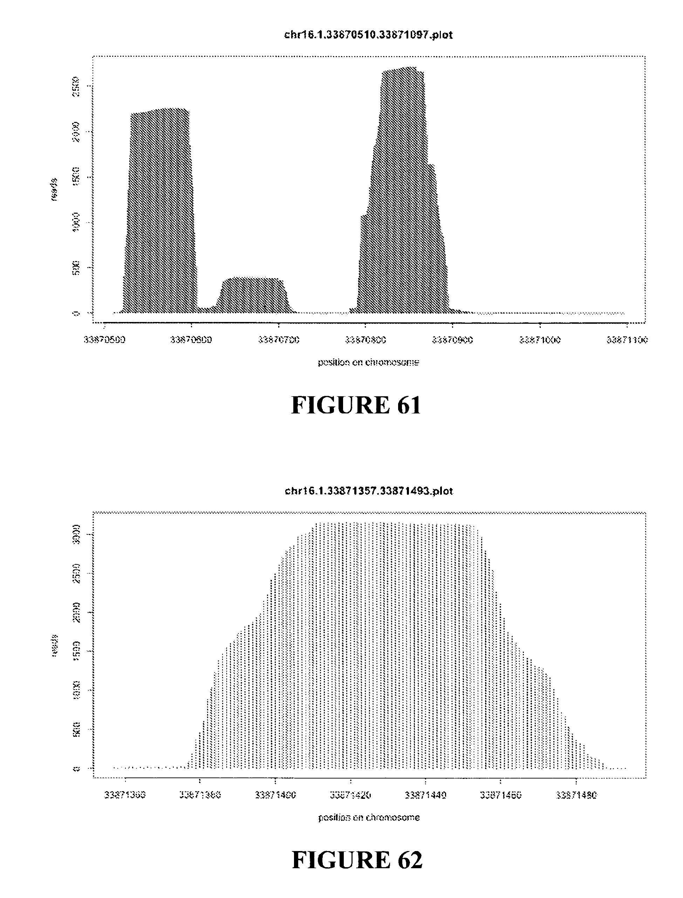

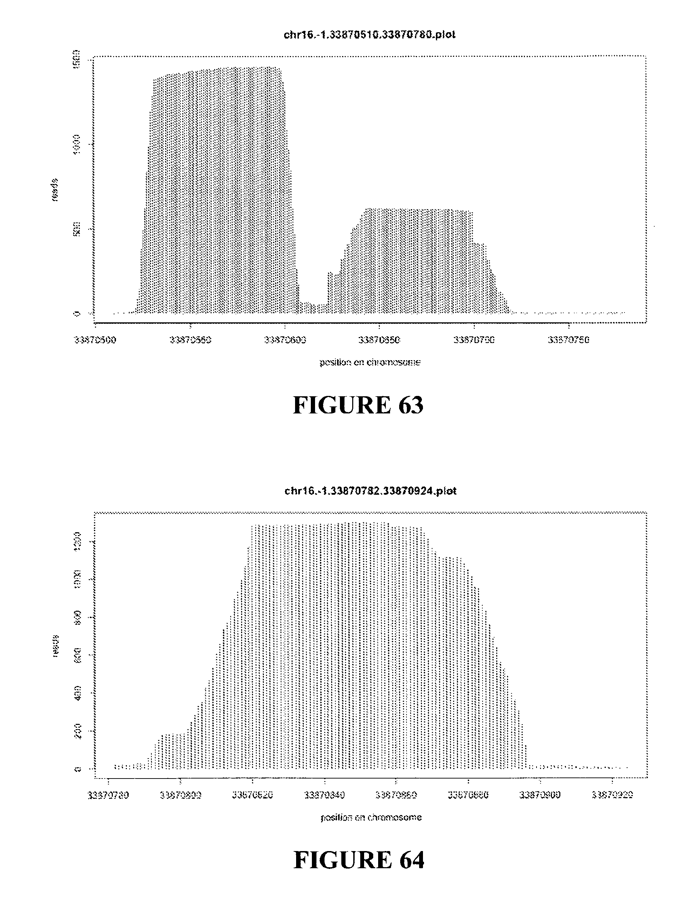

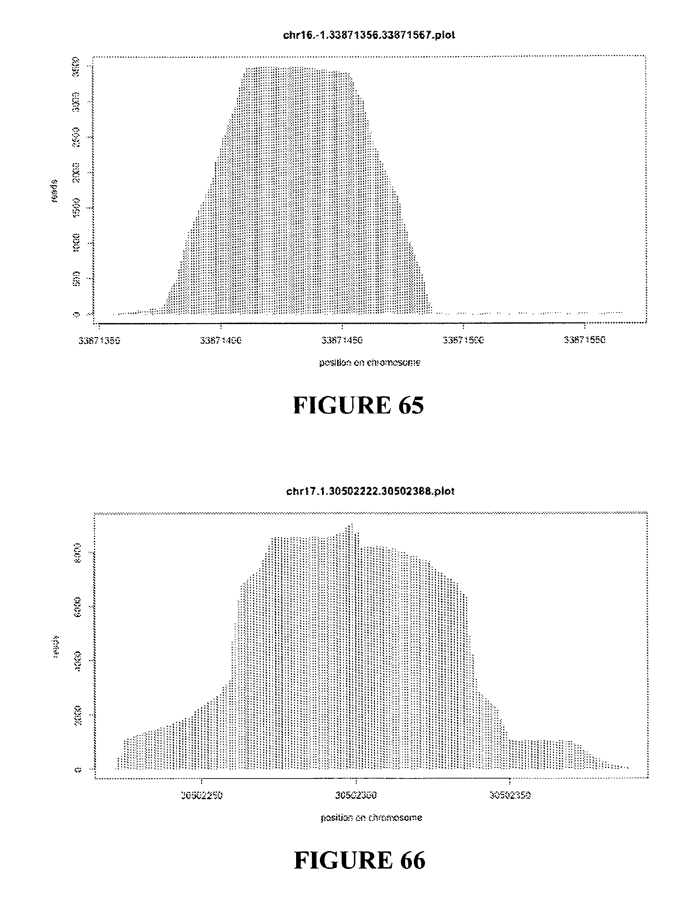

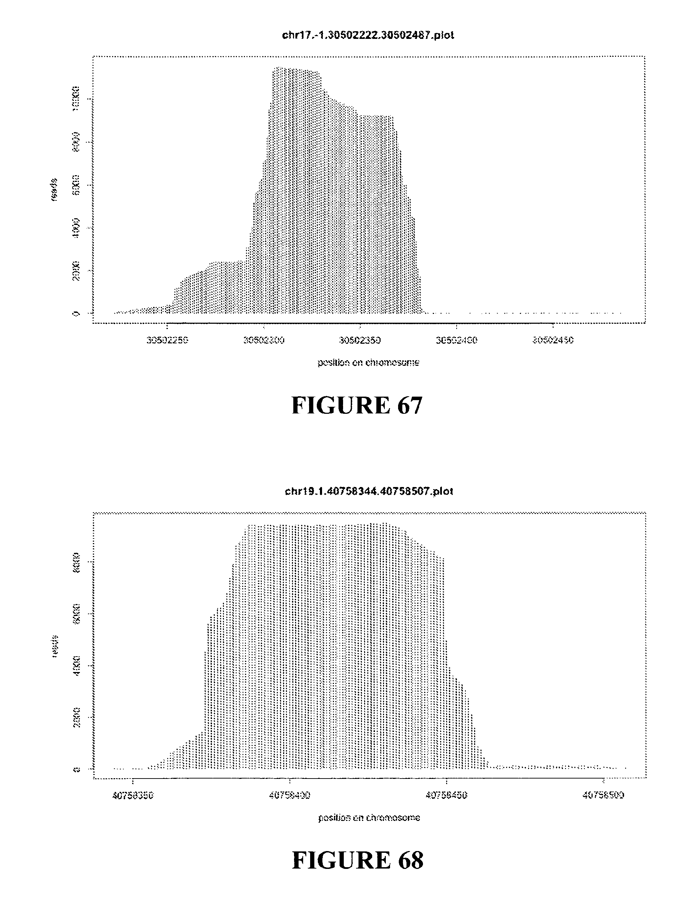

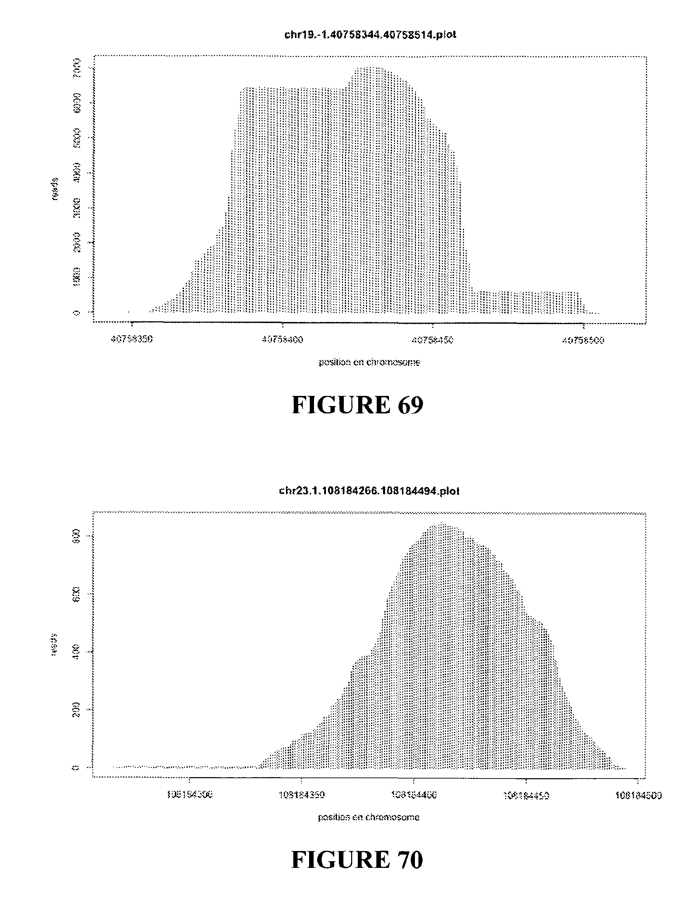

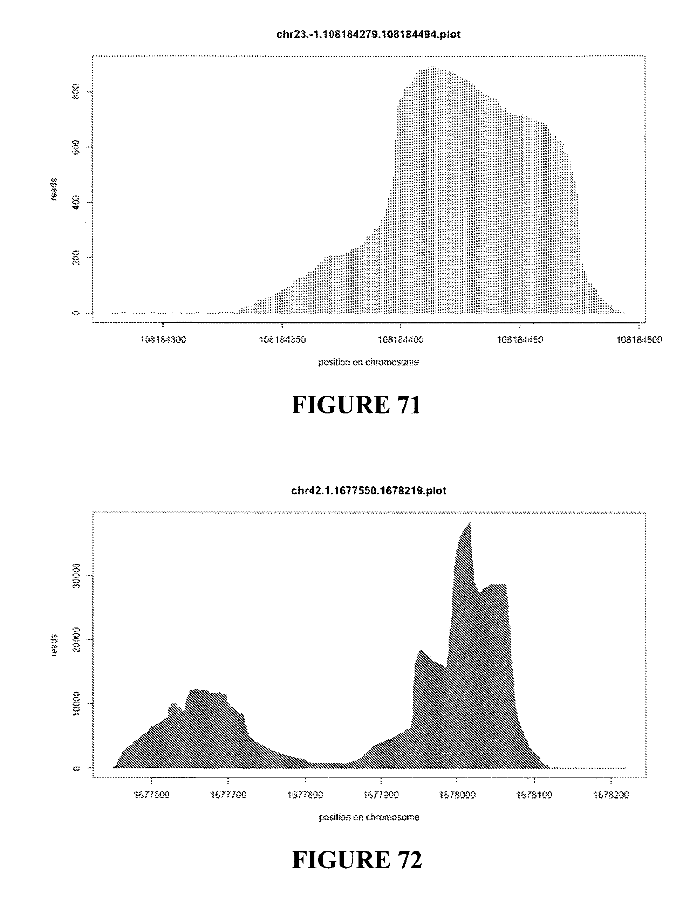

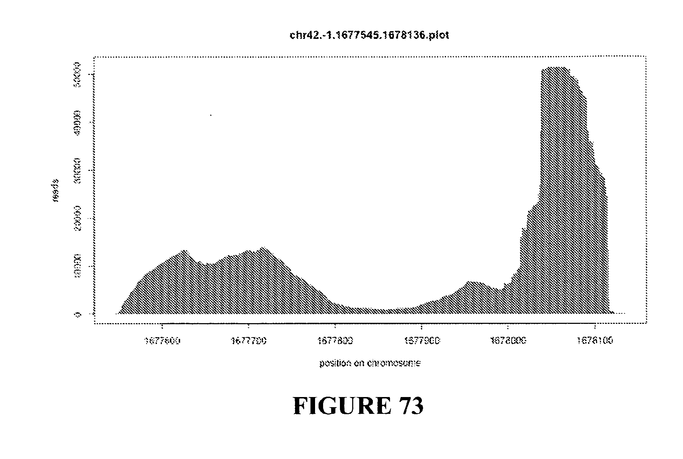

We identified 29 regions where there were more than 500 spikes. The 29 regions are shown in **Figure 42** and correspond to **SEQ ID NOS. 1-29.** The plotted spikes in these 29 regions are shown in **Figures 45-73****.** PCR analysis of the sequences of the most highly expressed spike transcripts demonstrated that they were indeed present within both human urinary microvesicles and human kidney cells, suggesting that these sequences were not an artifact of deep sequencing. The primers used to amply the 10 such spike abundant regions are shown in **Figure 43**. PCR was performed according to the following program: initial denaturing at 95°C for 8 minutes; 30 cycles of three steps of denaturing at 95°C for 40 seconds, annealing at 55°C for 30 seconds, and elongation at 65°C for 1 minute; final elongation at 68 °C for 5 minutes; and on hold at 4°C before BioAnalyzer analysis of the reaction. As shown in **Figure 44**, the amplification of each of these 10 regions gave positive results using templates in both human urinary microvesicles (**A**) and human kidney cells (**B**), suggesting that these spike transcripts were indeed present within microvesicles and human kidney cells.

These abundant spike transcripts can be used to assess the quality of an nucleic acid extraction from a biological sample. For example, the amount of any of the spike transcripts can be used to assess the quality of nucleic acids from urinary microvesicles in place of common markers such as GAPDH or ACTIN polynucleotide molecules. The amount of GAPDH or ACTIN RNA in urinary microvesicles was so low that an extra-amplification step, *e.g.,* a RiboAMP, was required for measuring their amount. In contrast, the amount of any one of the spike transcripts was so high that no extra-amplification step was necessary. Therefore, the use of these spike transcripts can make the assessment of nucleic acid extraction quality more efficient and simpler. Hence, described is a novel method of assessing the quality of a nucleic acid extraction from a biological sample, *e.g.,* a human urine sample. The method can be accomplished by extracting nucleic acids from a biological sample, measuring the amount of any of the spike transcripts, and compare the amount to a standard that has been establish for the particular biological sample. The establishment of such standard can be, for example, an average amount of such spike transcript extracted from 10 normal human urine samples performed by an experienced biotechnology professional.

While the present invention has been disclosed with reference to certain embodiments, numerous modifications, alterations, and changes to the described embodiments are possible without departing from the sphere and scope of the present invention, as defined in the appended claims. Accordingly, it is intended that the present invention not be limited to the described embodiments, but that it has the full scope defined by the language of the following claims, and equivalents thereof.

### REFERENCES:

Abravaya, K., J.J. Carrino, S. Muldoon, and H.H. Lee. 1995. Detection of point mutations with a modified ligase chain reaction (Gap-LCR). Nucleic Acids Res. 23 :675-82.
Al-Nedawi, K., B. Meehan, J. Micallef, V. Lhotak, L. May, A. Guha, and J. Rak. 2008. Intercellular transfer of the oncogenic receptor EGFRvIII by microvesicles derived from tumour cells. Nat Cell Biol. 10:619-24.
Balzar, M., M.J. Winter, C.J. de Boer, and S.V. Litvinov. 1999. The biology of the 17-1A antigen (Ep-CAM). J Mol Med. 77:699-712.
Bossi, A., F. Bonini, A.P. Turner, and S.A. Piletsky. 2007. Molecularly imprinted polymers for the recognition of proteins: the state of the art. Biosens Bioelectron. 22:1131-7.
Chen, C., J. Skog, C.H. Hsu, R.T. Lessard, L. Balaj, T. Wurdinger, B.S. Carter, X.O. Breakefield, M. Toner, and D. Irimia. Microfluidic isolation and transcriptome analysis of serum microvesicles. Lab Chip. 10:505-11.
Cheruvanky, A., H. Zhou, T. Pisitkun, J.B. Kopp, M.A. Knepper, P.S. Yuen, and R.A. Star. 2007. Rapid isolation of urinary exosomal biomarkers using a nanomembrane ultrafiltration concentrator. Am J Physiol Renal Physiol. 292:F1657-61.
Cocucci, E., G. Racchetti, and J. Meldolesi. 2009. Shedding microvesicles: artefacts no more. Trends Cell Biol. 19:43-51.
Cotton, R.G., N.R. Rodrigues, and R.D. Campbell. 1988. Reactivity of cytosine and thymine in single-base-pair mismatches with hydroxylamine and osmium tetroxide and its application to the study of mutations. Proc Natl Acad Sci U S A. 85:4397-401.
Fischer, S.G., and L.S. Lerman. 1979a. Length-independent separation of DNA restriction fragments in two-dimensional gel electrophoresis. Cell. 16:191-200.
Fischer, S.G., and L.S. Lerman. 1979b. Two-dimensional electrophoretic separation of restriction enzyme fragments of DNA. Methods Enzymol. 68:183-91.
Geiss, G.K., R.E. Bumgarner, B. Birditt, T. Dahl, N. Dowidar, D.L. Dunaway, H.P. Fell, S. Ferree, R.D. George, T. Grogan, J.J. James, M. Maysuria, J.D. Mitton, P. Oliveri, J.L. Osborn, T. Peng, A.L. Ratcliffe, P.J. Webster, E.H. Davidson, and L. Hood. 2008. Direct multiplexed measurement of gene expression with color-coded probe pairs. Nat Biotechnol. 26:317-25*.*
Guatelli, J.C., K.M. Whitfield, D.Y. Kwoh, K.J. Barringer, D.D. Richman, and T.R. Gingeras. 1990. Isothermal, in vitro amplification of nucleic acids by a multienzyme reaction modeled after retroviral replication. Proc Natl Acad Sci USA. 87:1874-8.
Hahn, P.J. 1993. Molecular biology of double-minute chromosomes. Bioessays. 15:477-84.
Johnson, S., D. Evans, S. Laurenson, D. Paul, A.G. Davies, P.K. Ferrigno, and C. Walti. 2008. Surface-immobilized peptide aptamers as probe molecules for protein detection. Anal Chem. 80:978-83.
Kan, Y.W., and A.M. Dozy. 1978a. Antenatal diagnosis of sickle-cell anaemia by D.N.A. analysis of amniotic-fluid cells. Lancet. 2:910-2.
Kan, Y.W., and A.M. Dozy. 1978b. Polymorphism of DNA sequence adjacent to human beta-globin structural gene: relationship to sickle mutation. Proc Natl Acad Sci U S A. 75:5631-5.
Keller, S., C. Rupp, A. Stoeck, S. Runz, M. Fogel, S. Lugert, H.D. Hager, M.S. Abdel-Bakky, P. Gutwein, and P. Altevogt. 2007. CD24 is a marker of exosomes secreted into urine and amniotic fluid. Kidney Int. 72:1095-102.
Kwoh, D.Y., G.R. Davis, K.M. Whitfield, H.L. Chappelle, L.J. DiMichele, and T.R. Gingeras. 1989. Transcription-based amplification system and detection of amplified human immunodeficiency virus type 1 with a bead-based sandwich hybridization format. Proc Natl Acad Sci U S A. 86:1173-7.
Landegren, U., R. Kaiser, J. Sanders, and L. Hood. 1988. A ligase-mediated gene detection technique. Science. 241:1077-80.
Li, J., L. Wang, H. Mamon, M.H. Kulke, R. Berbeco, and G.M. Makrigiorgos. 2008. Replacing PCR with COLD-PCR enriches variant DNA sequences and redefines the sensitivity of genetic testing. Nat Med. 14:579-84.
Liu, Q., J.C. Greimann, and C.D. Lima. 2006. Reconstitution, activities, and structure of the eukaryotic RNA exosome. Cell. 127:1223-37.
Miele, E.A., D.R. Mills, and F.R. Kramer. 1983. Autocatalytic replication of a recombinant RNA. J Mol Biol. 171:281-95.
Myers, R.M., Z. Larin, and T. Maniatis. 1985. Detection of single base substitutions by ribonuclease cleavage at mismatches in RNA:DNA duplexes. Science. 230:1242-6.
Nakazawa, H., D. English, P.L. Randell, K. Nakazawa, N. Martel, B.K. Armstrong, and H. Yamasaki. 1994. UV and skin cancer: specific p53 gene mutation in normal skin as a biologically relevant exposure measurement. Proc Natl Acad Sci U S A. 91:360-4.
Nilsson, J., J. Skog, A. Nordstrand, V. Baranov, L. Mincheva-Nilsson, X.O. Breakefield, and A. Widmark. 2009. Prostate cancer-derived urine exosomes: a novel approach to biomarkers for prostate cancer. Br J Cancer. 100:1603-7.
Orita, M., H. Iwahana, H. Kanazawa, K. Hayashi, and T. Sekiya. 1989. Detection of polymorphisms of human DNA by gel electrophoresis as single-strand conformation polymorphisms. Proc Natl Acad Sci USA. 86:2766-70.
Raposo, G., H.W. Nijman, W. Stoorvogel, R. Liejendekker, C.V. Harding, C.J. Melief, and H.J. Geuze. 1996. B lymphocytes secrete antigen-presenting vesicles. J Exp Med. 183:1161-72.
Skog, J., T. Wurdinger, S. van Rijn, D.H. Meijer, L. Gainche, M. Sena-Esteves, W.T. Curry, Jr., B.S. Carter, A.M. Krichevsky, and X.O. Breakefield. 2008. Glioblastoma microvesicles transport RNA and proteins that promote tumour growth and provide diagnostic biomarkers. Nat Cell Biol. 10:1470-6.
Steemers, F.J., W. Chang, G. Lee, D.L. Barker, R. Shen, and K.L. Gunderson. 2006. Whole-genome genotyping with the single-base extension assay. Nat Methods. 3:31-3.
Stoorvogel, W., M.J. Kleijmeer, H.J. Geuze, and G. Raposo. 2002. The biogenesis and functions of exosomes. Traffic. 3:321-30.
Taylor, D.D., and C. Gercel-Taylor. 2008. MicroRNA signatures of tumor-derived exosomes as diagnostic biomarkers of ovarian cancer. Gynecol Oncol. 110:13-21.
Thery, C., L. Zitvogel, and S. Amigorena. 2002. Exosomes: composition, biogenesis and function. Nat Rev Immunol. 2:569-79.
Valadi, H., K. Ekstrom, A. Bossios, M. Sjostrand, J.J. Lee, and J.O. Lotvall. 2007. Exosome-mediated transfer of mRNAs and microRNAs is a novel mechanism of genetic exchange between cells. Nat Cell Biol. 9:654-9.
van Dijk, E.L., G. Schilders, and G.J. Pruijn. 2007. Human cell growth requires a functional cytoplasmic exosome, which is involved in various mRNA decay pathways. RNA. 13:1027-35.
Velculescu, V.E., L. Zhang, B. Vogelstein, and K.W. Kinzler. 1995. Serial analysis of gene expression. Science. 270:484-7.
Went, P.T., A. Lugli, S. Meier, M. Bundi, M. Mirlacher, G. Sauter, and S. Dirnhofer. 2004. Frequent EpCam protein expression in human carcinomas. Hum Pathol. 35:122-8.

## Claims

1. A method of obtaining and analyzing RNA from a urine sample, comprising the steps of:
(a) obtaining a urine sample and pre-processing the urine sample by filtration through a 0.8µm filter;
(b) obtaining a microvesicle fraction from the pre-processed urine sample by ultracentrifugation or filtration concentration;
(c) washing the microvesicle fraction;
(d) extracting RNA from the microvesicle fraction;
(e) measuring the quality of the RNA from the microvesicle fraction by determining the quantity of 18S and 28S rRNA in the extraction for the purpose of evaluating the quality of the RNA extraction, where detection of 18S and 28S rRNA in the RNA extraction indicates that the RNA extraction is high yield and/or high integrity; and
f) analyzing one or more RNAs from the RNA extraction of step e) which has been evaluated as high yield and/or high integrity.

2. The method of claim 1, wherein the microvesicle fraction is obtained by filtration concentration.

3. The method of claim 2, wherein the filtration concentration comprises passing the urine sample through a filter having a pore size of less than or equal to 0.8 µm.

4. The method of any previous claim, wherein the urine sample or microvesicle fraction is treated with a ribonuclease, deoxyribonuclease, or a combination thereof, prior to performance of the washing in step c).

5. The method of any previous claim, wherein step c) comprises the addition of an RNase inhibitor, prior to extracting RNA.

6. The method of claim 5, wherein the RNase inhibitor has a concentration of greater than [1X] concentration; alternatively, greater than or equal to [5X] concentration; alternatively, greater than or equal to [10X] concentration; alternatively, greater than or equal to [25X] concentration; and alternatively, greater than or equal to [50X] concentration.

7. The method of claim 5 or claim 6, wherein the RNase inhibitor is a protease.

8. The method of any previous claim, further comprising determining a quantitative ratio of 18S rRNA to 28S rRNA in the extraction.

9. The method of claim 8, wherein the quantitative ratio of 18S rRNA to 28S rRNA is within the range of approximately 1:1 to approximately 1:2.

10. The method of claim 8 or claim 9, wherein the quantitative ratio of 18S rRNA to 28S rRNA is approximately 1:2.

11. The method of claim 4 comprising:
treating the microvesicle fraction with DNase to eliminate all or substantially all of any DNA located outside of or on the surface of the microvesicles in the fraction; extracting RNA from the sample; and analyzing the extracted RNA.

## Patentansprüche

1. Verfahren zum Erhalten und Analysieren von RNA aus einer Urinprobe, die folgenden Schritte umfassend:
(a) Erhalten einer Urinprobe und Vorverarbeiten der Urinprobe durch Filtrieren durch einen 0,8-µm-Filter;
(b) Erhalten einer Mikrovesikelfraktion aus der vorverarbeiteten Urinprobe durch Ultrazentrifugieren oder Filtrationskonzentrieren;
(c) Waschen der Mikrovesikelfraktion;
(d) Extrahieren von RNA aus der Mikrovesikelfraktion;
(e) Messen der Qualität der RNA aus der Mikrovesikelfraktion durch Ermitteln der Menge von 18S- und 28S-rRNA in der Extraktion, um die Qualität der RNA-Extraktion auszuwerten, wobei das Feststellen von 18S- und 28S-rRNA in der RNA-Extraktion darauf hindeutet, dass die RNA-Extraktion einen hohen Ertrag und / oder eine hohe Integrität aufweist; und
f) Analysieren einer oder mehrerer RNAs aus der RNA-Extraktion aus Schritt e), die als solche mit hohem Ertrag oder hoher Integrität bestimmt worden sind.

2. Verfahren nach Anspruch 1, wobei die Mikrovesikelfraktion durch Filtrationskonzentrieren erhalten wird.

3. Verfahren nach Anspruch 2, wobei das Filtrationskonzentrieren das Durchleiten der Urinprobe durch einen Filter mit einem Porendurchmesser von höchstens 0,8 µm umfasst.

4. Verfahren nach einem der vorhergehenden Ansprüche, wobei die Urinprobe oder die Mikrovesikelfraktion vor dem Waschen in Schritt c) mit einer Ribonuklease, einer Desoxyribonuklease oder einer Kombination aus diesen behandelt wird.

5. Verfahren nach einem der vorhergehenden Ansprüche, wobei Schritt c) vor dem Extrahieren der RNA das Hinzufügen eines RNase-Hemmers umfasst.

6. Verfahren nach Anspruch 5, wobei der RNase-Hemmer eine Konzentration von über [1X]; alternativ dazu von wenigstens [5X]; alternativ dazu von wenigstens [10X]; alternativ dazu von wenigstens [25X]; und alternativ dazu von wenigstens [50X] aufweist.

7. Verfahren nach Anspruch 5 oder 6, wobei der RNase-Hemmer eine Protease ist.

8. Verfahren nach einem der vorhergehenden Ansprüche, ferner umfassend das Bestimmen eines Mengenverhältnisses zwischen 18S-rRNA und 28S-rRNA in der Extraktion.

9. Verfahren nach Anspruch 8, wobei das Mengenverhältnis zwischen 18S-rRNA und 28S-rRNA zwischen etwa 1:1 und etwa 1:2 liegt.

10. Verfahren nach Anspruch 8 oder 9, wobei das Mengenverhältnis zwischen 18S-rRNA und 28S-rRNA etwa 1:2 beträgt.

11. Verfahren nach Anspruch 4, Folgendes umfassend:
Behandeln der Mikrovesikelfraktion mit DNase, um die gesamte oder im Wesentlichen die gesamte DNA, die sich außerhalb oder auf der Oberfläche der Mikrovesikel in der Fraktion befindet, zu eliminieren; Extrahieren der RNA aus der Probe; und Analysieren der extrahierten RNA.

## Revendications

1. Procédé d'obtention et d'analyse d'ARN d'un échantillon d'urine, comprenant les étapes consistant à :
(a) obtenir un échantillon d'urine et prétraiter l'échantillon d'urine par filtration à travers un filtre de 0,8 µm ;
(b) obtenir une fraction microvésiculaire de l'échantillon d'urine prétraité par ultracentrifugation ou par concentration sous filtration ;
(c) laver la fraction microvésiculaire ;
(d) extraire l'ARN de la fraction microvésiculaire ;
(e) mesurer la qualité de l'ARN de la fraction microvésiculaire en déterminant la quantité d'ARNr 18S et 28S lors de l'extraction afin d'évaluer la qualité de l'extraction d'ARN, où la détection d'ARNr 18S et 28S lors de l'extraction d'ARN indique que l'extraction d'ARN est à haut rendement et/ou haute intégrité ; et
(f) analyser un ou plusieurs ARN issus de l'extraction d'ARN de l'étape e) qui ont été évalués comme étant à haut rendement et/ou haute intégrité.

2. Procédé selon la revendication 1, dans lequel la fraction microvésiculaire est obtenue par concentration sous filtration.

3. Procédé selon la revendication 2, dans lequel la concentration sous filtration comprend l'étape consistant à faire passer l'échantillon d'urine à travers un filtre d'une porosité inférieure ou égale à 0,8 µm.

4. Procédé selon l'une quelconque des revendications précédentes, dans lequel l'échantillon d'urine ou la fraction microvésiculaire est traité(e) avec une ribonucléase, une désoxyribonucléase ou une combinaison d'entre elles, avant d'effectuer le lavage de l'étape c).

5. Procédé selon l'une quelconque des revendications précédentes, dans lequel l'étape c) comprend l'ajout d'un inhibiteur de RNase, avant l'extraction d'ARN.

6. Procédé selon la revendication 5, dans lequel l'inhibiteur de RNase a une concentration supérieure à une concentration de [1X] ; en variante, supérieure ou égale à une concentration de [5X] ; en variante, supérieure ou égale à une concentration de [10X] ; en variante, supérieure ou égale à une concentration de [25X] ; et en variante, supérieure ou égale à une concentration de [50X].

7. Procédé selon la revendication 5 ou 6, dans lequel l'inhibiteur de RNase est une protéase.

8. Procédé selon l'une quelconque des revendications précédentes, comprenant en outre la détermination d'un rapport quantitatif de l'ARNr 18S à l'ARNr 28S lors de l'extraction.

9. Procédé selon la revendication 8, dans lequel le rapport quantitatif de l'ARNr 18S à l'ARNr 28S est compris entre environ 1:1 et environ 1:2.

10. Procédé selon la revendication 8 ou 9, dans lequel le rapport quantitatif de l'ARNr 18S à l'ARNr 28S est d'environ 1:2.

11. Procédé selon la revendication 4, comprenant les étapes consistant à :
traiter la fraction microvésiculaire avec de la DNase pour éliminer tout ou sensiblement tout l'ADN situé en dehors ou sur la surface des microvésicules dans la fraction ; extraire l'ARN de l'échantillon ; et analyser l'ARN extrait.
